# EUROPEAN PATENT SPECIFICATION

(11) **EP 4 174 060 B1**
(45) Date of publication and mention of the grant of the patent: **20.08.2025**
(21) Application number: 21829372.8
(22) Date of filing: 18.06.2021
(51) Int. Cl.: C07D 405/10, C07D 409/10, C07D 403/10, C07D 405/14, C07D 409/14, C07D 403/14, H10K 50/11, H10K 85/40, H10K 85/60, C07D 213/16, C07D 251/24, C07D 405/04, C07D 409/04, C07D 491/048, C07F 7/08, C07D 495/04

(54) **COMPOUND AND ORGANIC LIGHT-EMITTING DEVICE COMPRISING SAME**
VERBINDUNG UND ORGANISCHE LICHTEMITTIERENDE VORRICHTUNG DAMIT
COMPOSÉ ET DISPOSITIF ÉLECTROLUMINESCENT ORGANIQUE LE COMPRENANT

(30) Priority: 26.06.2020 KR 20200078447
(43) Date of publication of application: 03.05.2023
(73) Proprietor: LT Materials Co., Ltd., Yongin-si, Gyeonggi-do 17118 (KR)
(72) Inventor: PARK, Geon-Yu, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/007680
(87) International publication number: WO 2021/261849

(56) References cited:
- WO-A1-2018/021854
- WO-A1-2019/245264
- JP-A- 2006 143 845
- KR-A- 20150 126 756
- KR-A- 20170 070 359
- KR-A- 20180 027 457

## Description

### [Technical Field]

The present specification relates to a compound, and an organic light emitting device including the same.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

WO2019/245264 A1 discloses a heterocyclic compound, an organic light emitting diode comprising the same, a manufacturing method therefore, and a composition for an organic layer.

### [Disclosure]

### [Technical Problem]

The present specification is directed to providing a compound, and an organic light emitting device including the same.

### [Technical Solution]

One embodiment of the present specification provides a compound of the following Chemical Formula 1.

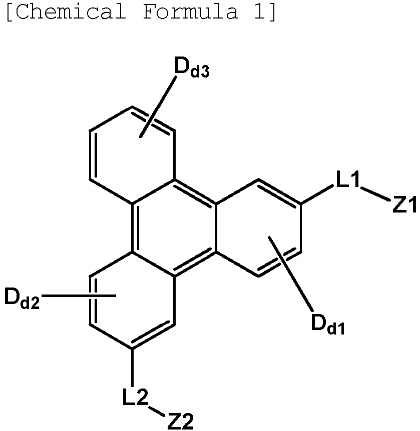

In Chemical Formula 1,
d1 and d2 are each an integer of 0 to 3,
d3 is an integer of 0 to 4,
L1 and L2 are the same as or different from each other, and each independently a direct bond; or a substituted or unsubstituted C6 to C60 arylene group,
Z1 and Z2 are the same as or different from each other, and each independently a substituted or unsubstituted silyl group; a substituted or unsubstituted fluorenyl group; or a heteroaryl group represented by any one of the following Chemical Formulae 1-1 to 1-4, and
at least one of Z1 and Z2 is a heteroaryl group represented by any one of the following Chemical Formulae 1-1 to 1-3, 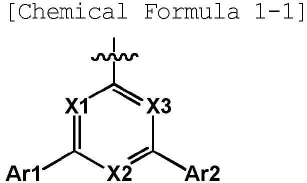 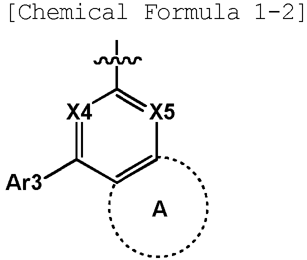 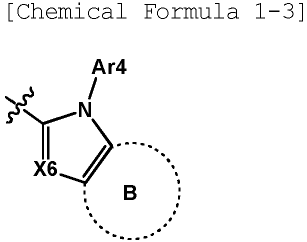 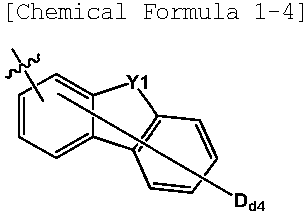
in Chemical Formulae 1-1 to 1-4,
d4 is an integer of 0 to 7,
X1 to X6 are each N or CR,
at least one of X1 to X3 is N,
at least one of X4 and X5 is N,
Y1 is O; S or NR',
R, R', Ar1 to Ar4 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
A and B are each a substituted or unsubstituted monocyclic or polycyclic aryl ring; or a monocyclic or polycyclic heteroring substituted or unsubstituted and including O or S, and
when L1 is a direct bond and Z1 is the heteroaryl group represented by Chemical Formula 1-1, Z2 is a substituted or unsubstituted silyl group; a substituted or unsubstituted fluorenyl group; or the tricyclic or higher heteroaryl group represented by any one of Chemical Formulae 1-2 to 1-4.

Another embodiment provides an organic light emitting device including a first electrode; a second electrode provided opposite to the first electrode; and an organic material layer provided between the first electrode and the second electrode, wherein the organic material layer includes one or more types of the compound of Chemical Formula 1.

Another embodiment provides a composition for forming an organic material layer, the composition including the compound of Chemical Formula 1; and a compound of the following Chemical Formula 2 or 3.

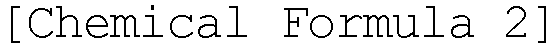

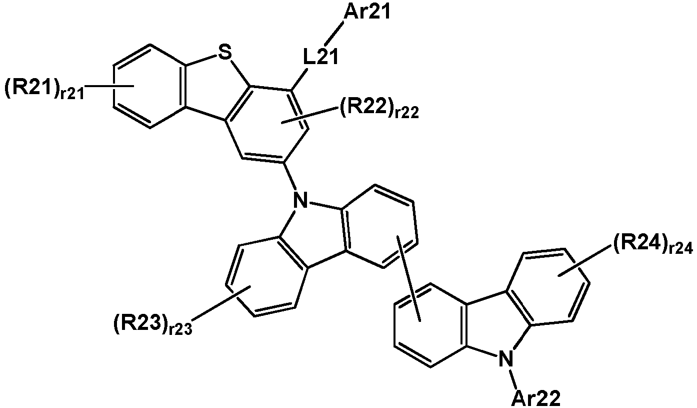

In Chemical Formula 2,
R21 to R24 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
L21 is a direct bond; a substituted or unsubstituted C6 to C60 arylene group; or a substituted or unsubstituted C2 to C60 heteroarylene group,
Ar21 and Ar22 are each independently a substituted or unsubstituted C6 to C60 aryl group; or a substituted or unsubstituted C2 to C60 heteroaryl group,
r21 is an integer of 1 to 4, and when 2 or greater, R21s are the same as or different from each other,
r22 is 1 or 2, and when 2, R22s are the same as or different from each other,
r23 is an integer of 1 to 4, and when 2 or greater, R23s are the same as or different from each other, and
r24 is an integer of 1 to 4, and when 2 or greater, R24s are the same as or different from each other, 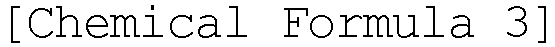 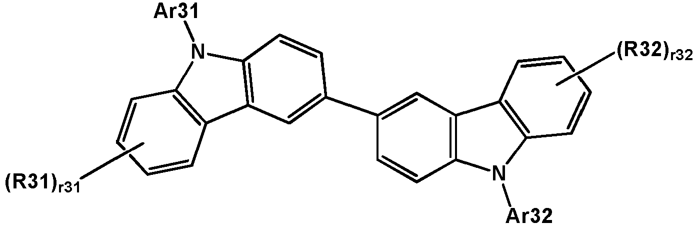
in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a C2 to C60 heteroaryl group substituted or unsubstituted and including O or N,
r31 is an integer of 1 to 4, and when 2 or greater, R31s are the same as or different from each other, and
r32 is an integer of 1 to 4, and when 2 or greater, R32s are the same as or different from each other.

### [Advantageous Effects]

A compound described in the present specification can be used as a material of an organic material layer of an organic light emitting device. The compound is capable of performing a role of a hole injection material, a hole transfer material, a light emitting material, an electron transfer material, an electron injection material, a charge generation material and the like in an organic light emitting device. Particularly, the compound can be used as a material of a light emitting layer of an organic light emitting device.

Chemical Formula 1 has two substituents at specific positions of triphenylene, and by necessarily including a heteroaryl group including N, superior light emission efficiency and lifetime can be provided when used in a device by enhancing electron stability and mobility.

In addition, the triphenylene and all substituents substituting the triphenylene may be substituted with deuterium in Chemical Formula **1,** and by the compound of Chemical Formula 1 being substituted with one or more deuterium, BDE (bond-dissociation energy) of the C-D bond of the molecule substituted with deuterium is enhanced, and a superior lifetime can be provided when used in a device.

When using the compound of Chemical Formula 1 and a compound of Chemical Formula 2 or 3 together as a material of a light emitting layer of an organic light emitting device, a driving voltage can be lowered, light emission efficiency can be enhanced, and lifetime properties can be enhanced in the device.

### [Description of Drawings]

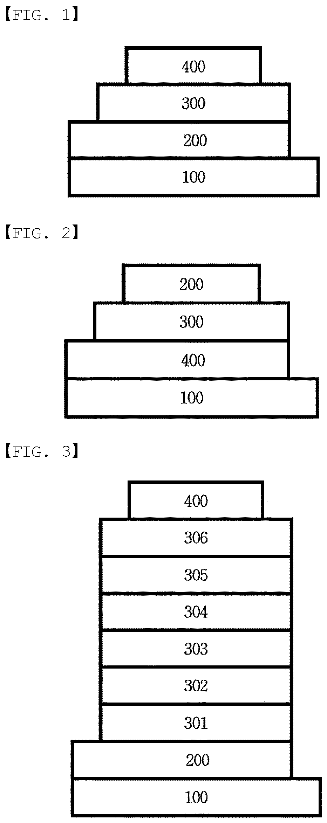

FIG. 1 to FIG. 3 are diagrams each illustrating a lamination structure of an organic light emitting device according to one embodiment of the present specification.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

A term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of deuterium; a halogen group; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or being unsubstituted, or being substituted with a substituent linking two or more substituents selected from among the substituents illustrated above, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 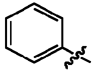 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

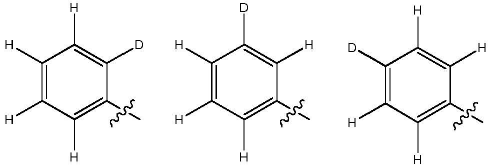

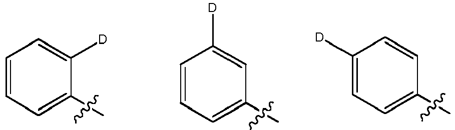

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched, and may be further substituted with other substituents. The number of carbon atoms of the alkynyl group may be from 2 to 60, specifically from 2 to 40 and more specifically from 2 to 20.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon atoms of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. When the aryl group is dicyclic or higher, the number of carbon atoms may be from 8 to 60, 8 to 40, or 8 to 30. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the fluorenyl group may be substituted, and adjacent substituents may bond to each other to form a ring.

When the fluorenyl group is substituted, 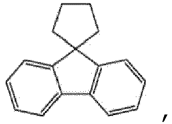 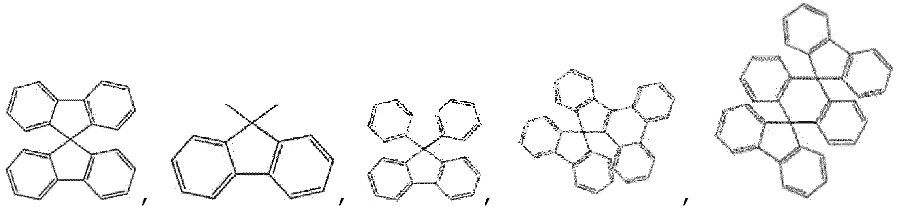 and the like may be included, however, the structure is not limited thereto.

In the present specification, the heteroaryl group includes O, S, SO₂, Se, N or Si as a heteroatom, includes monocyclic or polycyclic, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. When the heteroaryl group is dicyclic or higher, the number of carbon atoms may be from 4 to 60, 4 to 40, or 4 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo [2, 3-a] carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a benzofuro[2,3-d]pyrimidyl group; a benzothieno[2,3-d]pyrimidyl group; a benzofuro[2,3-a]carbazolyl group, a benzothieno[2,3-a]carbazolyl group, a 1,3-dihydroindolo[2,3-a]carbazolyl group, a benzofuro[3,2-a]carbazolyl group, a benzothieno[3,2-a]carbazolyl group, a 1,3-dihydroindolo[3,2-a]carbazolyl group, a benzofuro[2,3-b]carbazolyl group, a benzothieno[2,3-b]carbazolyl group, a 1,3-dihydroindolo[2,3-b]carbazolyl group, a benzofuro[3,2-b]carbazolyl group, a benzothieno[3,2-b]carbazolyl group, a 1,3-dihydroindolo[3,2-b]carbazolyl group, a benzofuro[2,3-c]carbazolyl group, a benzothieno[2,3-c]carbazolyl group, a 1,3-dihydroindolo[2,3-c]carbazolyl group, a benzofuro[3,2-c]carbazolyl group, a benzothieno[3,2-c]carbazolyl group, a 1,3-dihydroindolo[3,2-c]carbazolyl group, a 1,3-dihydroindeno[2,1-b]carbazolyl group, a 5,11-dihydroindeno[1,2-b]carbazolyl group, a 5,12-dihydroindeno[1,2-c]carbazolyl group, a 5,8-dihydroindeno[2,1-c]carbazolyl group, a 7,12-dihydroindeno[1,2-a]carbazolyl group, a 11,12-dihydroindeno[2,1-a]carbazolyl group and the like, but are not limited thereto.

In the present specification, the silyl group is a substituent including Si, having the Si atom directly linked as a radical, and is represented by -Si(R101) (R102) (R103). R101 to R103 are the same as or different from each other, and may be each independently a substituent formed with at least one of hydrogen; deuterium; a halogen group; an alkyl group; an alkenyl group; an alkoxy group; a cycloalkyl group; an aryl group; and a heteroaryl group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the phosphine oxide group is represented by -P(=O) (R104) (R105), and R104 and R105 are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. Specifically, the phosphine oxide group may be substituted with an alkyl group or an aryl group, and as the alkyl group or the aryl group, the examples described above may be applied. Examples of the phosphine oxide group may include a dimethylphosphine oxide group, a diphenylphosphine oxide group, a dinaphthylphosphine oxide group and the like, but are not limited thereto.

In the present specification, the amine group is represented by -N(R106) (R107), and R106 and R107 are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the examples of the aryl group described above may be applied to the arylene group except that the arylene group is a divalent group.

In the present specification, the examples of the heteroaryl group described above may be applied to the heteroarylene group except that the heteroarylene group is a divalent group.

One embodiment of the present specification provides a compound represented by Chemical Formula 1.

In one embodiment of the present specification, d1 and d2 are each an integer of 0 to 3, and d3 is an integer of 0 to 4.

In one embodiment of the present specification, d1, d2 and d3 are 0.

In one embodiment of the present specification, d1 and d2 are 3, and d3 is 4.

In one embodiment of the present specification, L1 and L2 are the same as or different from each other, and each independently a direct bond; or a substituted or unsubstituted C6 to C30 arylene group.

In one embodiment of the present specification, L1 and L2 are the same as or different from each other, and each independently a direct bond; or a substituted or unsubstituted C6 to C20 arylene group.

In one embodiment of the present specification, L1 and L2 are the same as or different from each other, and each independently a direct bond; or a substituted or unsubstituted C6 to C30 arylene group.

In one embodiment of the present specification, L1 and L2 are the same as or different from each other, and each independently a direct bond; or a substituted or unsubstituted C6 to C20 arylene group.

In one embodiment of the present specification, L1 and L2 are the same as or different from each other, and each independently a direct bond; or a substituted or unsubstituted phenylene group.

In one embodiment of the present specification, L1 and L2 are the same as or different from each other, and each independently a direct bond; or any one selected from among the following structures.

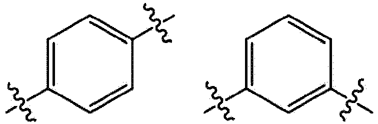

In one embodiment of the present specification, L1 and L2 are the same as or different from each other, and each independently a direct bond; or a phenylene group. When L1 and L2 are a phenylene group, triphenylene and Z1, or triphenylene and Z2 may bond at a para or meta position, which is effective in increasing molecular stability by reducing steric hindrance between the molecules.

In one embodiment of the present specification, L1 and L2 may be a direct bond.

In one embodiment of the present specification, any one of L1 and L2 is a direct bond, and the other one may be a substituted or unsubstituted C6 to C20 arylene group.

In one embodiment of the present specification, Z1 and Z2 are the same as or different from each other, and each independently a substituted or unsubstituted silyl group; a substituted or unsubstituted fluorenyl group; or a heteroaryl group represented by any one of the following Chemical Formulae 1-1 to 1-4, and at least one of Z1 and Z2 is a heteroaryl group represented by any one of the following Chemical Formulae 1-1 to 1-3.

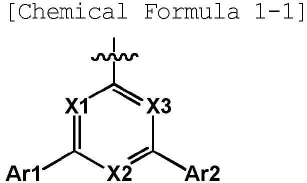

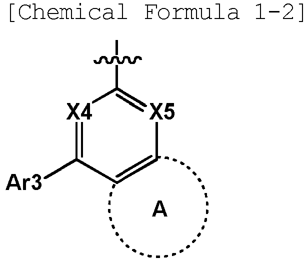

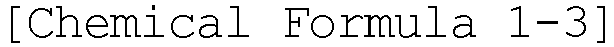

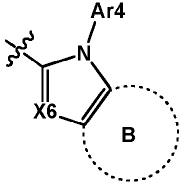

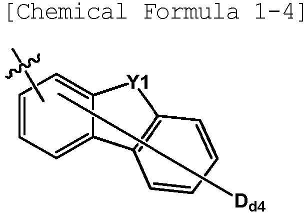

In Chemical Formulae 1-1 to 1-4,
d4 is an integer of 0 to 7,
X1 to X6 are each N or CR,
at least one of X1 to X3 is N,
at least one of X4 and X5 is N,
Y1 is O; S or NR',
R, R', Ar1 to Ar4 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group, and
A and B are each a substituted or unsubstituted monocyclic or polycyclic aryl ring; or a monocyclic or polycyclic heteroring substituted or unsubstituted and including O or S.

In one embodiment of the present specification, X1 to X3 of Chemical Formula 1-1 are each N or CR, and at least one of X1 to X3 is N.

In one embodiment of the present specification, X1 to X3 of Chemical Formula 1-1 may be N.

In one embodiment of the present specification, X1 and X2 of Chemical Formula 1-1 are N, X3 is CR, and R may be hydrogen.

In one embodiment of the present specification, X1 and X3 of Chemical Formula 1-1 are N, X2 is CR, and R may be hydrogen.

In one embodiment of the present specification, X1 of Chemical Formula 1-1 is N, X2 and X3 are CR, and R may be hydrogen.

In one embodiment of the present specification, X2 of Chemical Formula 1-1 is N, X1 and X3 are CR, and R may be hydrogen.

In one embodiment of the present specification, X3 of Chemical Formula 1-1 is N, X1 and X2 are CR, and R may be hydrogen.

In one embodiment of the present specification, Ar1 and Ar2 of Chemical Formula 1-1 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group.

In one embodiment of the present specification, Ar1 and Ar2 of Chemical Formula 1-1 are each independently a substituted or unsubstituted C6 to C60 aryl group; or a substituted or unsubstituted C2 to C60 heteroaryl group.

In one embodiment of the present specification, Ar1 and Ar2 of Chemical Formula 1-1 are each independently a substituted or unsubstituted C6 to C40 aryl group; or a substituted or unsubstituted C2 to C40 heteroaryl group.

In one embodiment of the present specification, Ar1 and Ar2 of Chemical Formula 1-1 are each independently a substituted or unsubstituted C6 to C30 aryl group; or a substituted or unsubstituted C2 to C30 heteroaryl group.

In one embodiment of the present specification, Ar1 and Ar2 of Chemical Formula 1-1 are each independently a substituted or unsubstituted phenyl group; a substituted or unsubstituted biphenyl group; a substituted or unsubstituted fluorenyl group; a substituted or unsubstituted dibenzofuran group; a substituted or unsubstituted dibenzothiophene group; or a substituted or unsubstituted carbazole group.

In one embodiment of the present specification, Ar1 and Ar2 of Chemical Formula 1-1 are each independently a phenyl group unsubstituted or substituted with deuterium; a biphenyl group; a fluorenyl group unsubstituted or substituted with an alkyl group or an aryl group; 9,9'-spirobi[fluorene]; a substituted or unsubstituted dibenzofuran group; a substituted or unsubstituted dibenzothiophene group; or a substituted or unsubstituted carbazole group.

When Ar1 or Ar2 of Chemical Formula 1-1 is a substituted or unsubstituted carbazole group, the bonding position is either nitrogen of the pyridine ring or carbon of the benzene ring.

In one embodiment of the present specification, X4 and X5 of Chemical Formula 1-2 are each N or CR, and at least one of X4 and X5 is N.

In one embodiment of the present specification, X4 and X5 of Chemical Formula 1-2 are N.

In one embodiment of the present specification, A of Chemical Formula 1-2 is a substituted or unsubstituted monocyclic or polycyclic aryl ring; or a monocyclic or polycyclic heteroring substituted or unsubstituted and including O or S.

In one embodiment of the present specification, A of Chemical Formula 1-2 is a substituted or unsubstituted monocyclic aryl ring; or a polycyclic heteroring substituted or unsubstituted and including O or S.

In one embodiment of the present specification, A of Chemical Formula 1-2 is a substituted or unsubstituted benzene ring; a substituted or unsubstituted benzofuran ring; or a substituted or unsubstituted benzothiophene ring.

In one embodiment of the present specification, A of Chemical Formula 1-2 is a benzene ring; a benzofuran ring; or a benzothiophene ring.

In one embodiment of the present specification, Ar3 of Chemical Formula 1-2 is a substituted or unsubstituted C6 to C60 aryl group.

In one embodiment of the present specification, Ar3 of Chemical Formula 1-2 is a substituted or unsubstituted C6 to C30 aryl group.

In one embodiment of the present specification, Ar3 of Chemical Formula 1-2 is a substituted or unsubstituted phenyl group.

In one embodiment of the present specification, Ar3 of Chemical Formula 1-2 is a phenyl group.

In one embodiment of the present specification, X6 of Chemical Formula 1-3 is N.

In one embodiment of the present specification, B of Chemical Formula 1-3 is a substituted or unsubstituted monocyclic or polycyclic aryl ring.

In one embodiment of the present specification, B of Chemical Formula 1-3 is a substituted or unsubstituted monocyclic aryl ring.

In one embodiment of the present specification, B of Chemical Formula 1-3 is a substituted or unsubstituted benzene ring.

In one embodiment of the present specification, B of Chemical Formula 1-3 is a benzene ring.

In one embodiment of the present specification, Ar4 of Chemical Formula 1-3 is a substituted or unsubstituted C6 to C60 aryl group.

In one embodiment of the present specification, Ar4 of Chemical Formula 1-3 is a substituted or unsubstituted C6 to C30 aryl group.

In one embodiment of the present specification, Ar4 of Chemical Formula 1-3 is a substituted or unsubstituted phenyl group.

In one embodiment of the present specification, Ar4 of Chemical Formula 1-3 is a phenyl group.

In one embodiment of the present specification, Y1 of Chemical Formula 1-4 is O; S or NR'.

In one embodiment of the present specification, Y1 of Chemical Formula 1-4 may be O.

In one embodiment of the present specification, Y1 of Chemical Formula 1-4 may be S.

In one embodiment of the present specification, Y1 of Chemical Formula 1-4 is NR', and R' may be a substituted or unsubstituted C6 to C60 aryl group.

In one embodiment of the present specification, Y1 of Chemical Formula 1-4 is NR', and R' may be a substituted or unsubstituted C6 to C30 aryl group.

In one embodiment of the present specification, Y1 of Chemical Formula 1-4 is NR', and R' may be a substituted or unsubstituted phenyl group.

In one embodiment of the present specification, d4 of Chemical Formula 1-4 is an integer of 0 to 7.

In one embodiment of the present specification, d4 of Chemical Formula 1-4 is 0 or 7.

In one embodiment of the present specification, at least one of Z1 and Z2 may be the heteroaryl group represented by Chemical Formula 1-1.

In one embodiment of the present specification, at least one of Z1 and Z2 may be the heteroaryl group represented by Chemical Formula 1-2.

In one embodiment of the present specification, at least one of Z1 and Z2 may be the heteroaryl group represented by Chemical Formula 1-3.

In one embodiment of the present specification, when L1 is a direct bond and Z1 is the heteroaryl group represented by Chemical Formula 1-1, Z2 is a substituted or unsubstituted silyl group; a substituted or unsubstituted fluorenyl group; or the tricyclic or higher heteroaryl group represented by any one of Chemical Formulae 1-2 to 1-4.

In one embodiment of the present specification, when L1 is a direct bond and Z1 is the heteroaryl group represented by Chemical Formula 1-1, Z2 is a substituted or unsubstituted silyl group; a substituted or unsubstituted fluorenyl group; or the heteroaryl group represented by Chemical Formula 1-4.

In one embodiment of the present specification, when L1 is a direct bond and Z1 is the heteroaryl group represented by Chemical Formula 1-1, Z2 may be a substituted or unsubstituted silyl group; or a substituted or unsubstituted fluorenyl group.

In one embodiment of the present specification, when L1 is a direct bond and Z1 is the heteroaryl group represented by Chemical Formula 1-1, Z2 may be a silyl group unsubstituted or substituted with an aryl group; a fluorenyl group unsubstituted or substituted with an alkyl group or an aryl group; or 9,9'-spirobi[fluorene].

In one embodiment of the present specification, when L1 is a direct bond and Z1 is the heteroaryl group represented by Chemical Formula 1-1, Z2 is the heteroaryl group represented by Chemical Formula 1-2, and herein, A of Chemical Formula 1-2 may be a substituted or unsubstituted polycyclic aryl ring; or a polycyclic heteroring substituted or unsubstituted and including O or S.

In one embodiment of the present specification, when L1 is a direct bond and Z1 is the heteroaryl group represented by Chemical Formula 1-1, Z2 is the heteroaryl group represented by Chemical Formula 1-2, and herein, A of Chemical Formula 1-2 may be a substituted or unsubstituted dicyclic aryl ring; or a dicyclic heteroring substituted or unsubstituted and including O or S.

In one embodiment of the present specification, when L1 is a direct bond and Z1 is the heteroaryl group represented by Chemical Formula 1-1, Z2 is the heteroaryl group represented by Chemical Formula 1-2, and herein, A of Chemical Formula 1-2 may be a dicyclic heteroring substituted or unsubstituted and including O or S.

In one embodiment of the present specification, when L1 is a direct bond and Z1 is the heteroaryl group represented by Chemical Formula 1-1, Z2 is the heteroaryl group represented by Chemical Formula 1-2, and herein, A of Chemical Formula 1-2 may be a benzofuran ring; or a benzothiophene ring.

In one embodiment of the present specification, when L1 is a direct bond and Z1 is the heteroaryl group represented by Chemical Formula 1-1, Z2 may be the heteroaryl group represented by Chemical Formula 1-4.

In one embodiment of the present specification, when L1 is a direct bond and Z1 is the heteroaryl group represented by Chemical Formula 1-1, Z2 is a substituted or unsubstituted silyl group; a substituted or unsubstituted fluorenyl group; or a heteroaryl group represented by any one of the following Chemical Formulae 1-1-1 to 1-1-3.

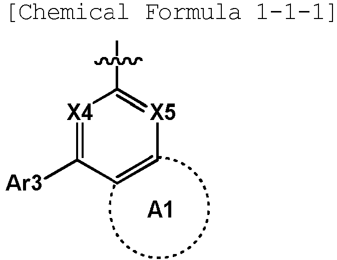

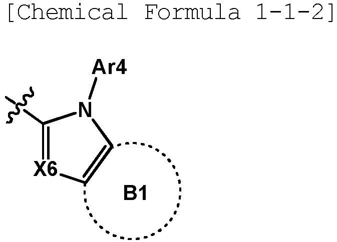

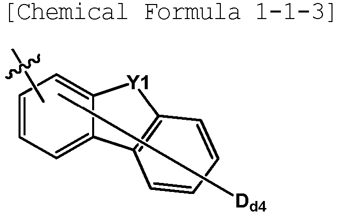

In Chemical Formulae 1-1-1 to 1-1-3,
d4 is an integer of 0 to 7,
X4 and X6 are each N or CR,
at least one of X4 and X5 is N,
Y1 is O; S or NR',
R, R', Ar3 and Ar4 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group, and
A1 and B1 are each independently a substituted or unsubstituted polycyclic aryl ring; or a polycyclic heteroring substituted or unsubstituted and including O or S.

In one embodiment of the present specification, Chemical Formula 1 may be represented by any one of the following compounds, but is not limited thereto.

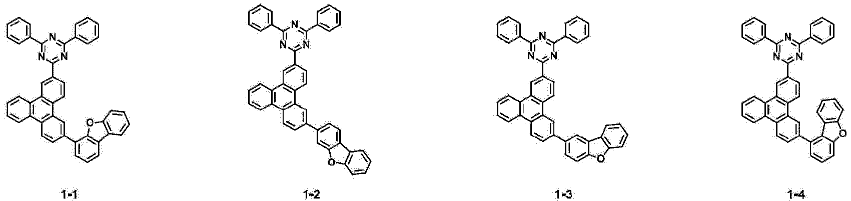

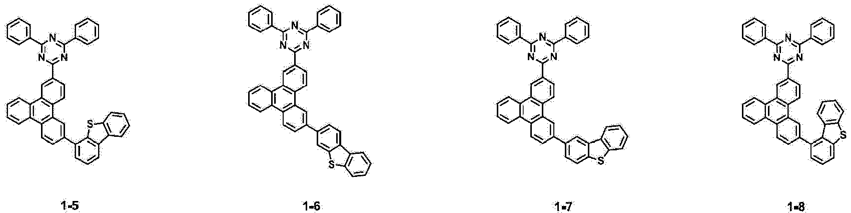

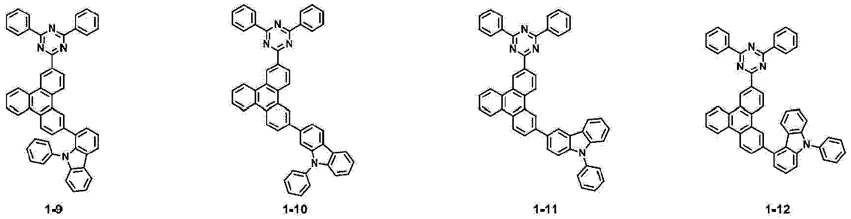

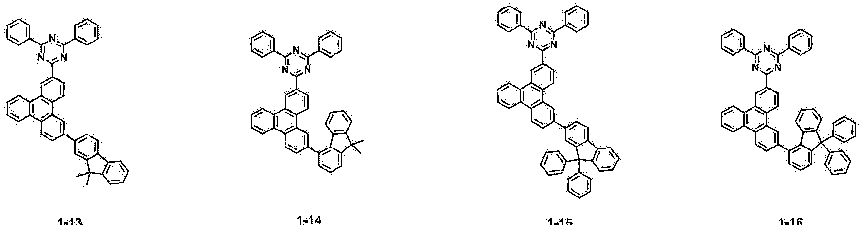

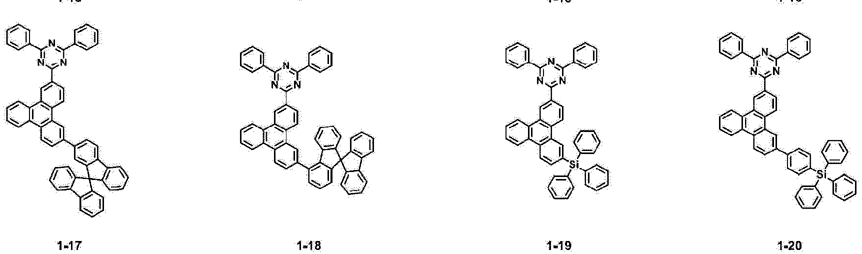

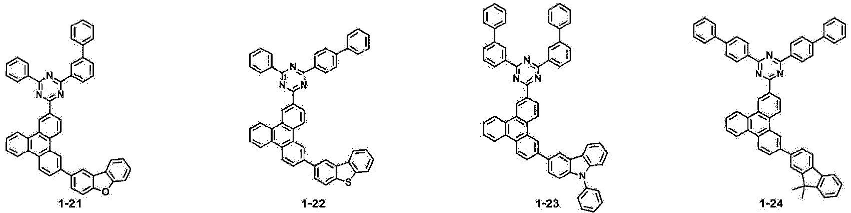

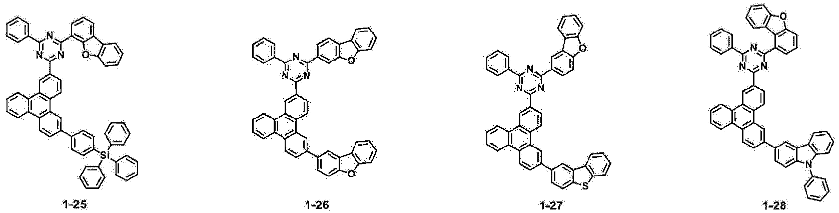

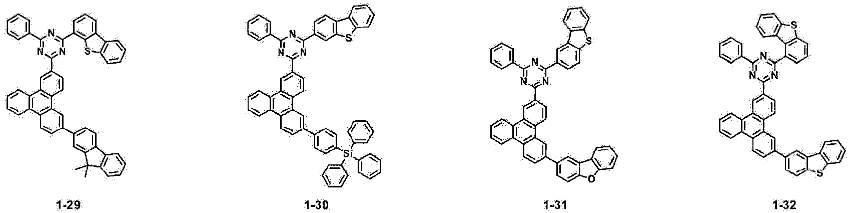

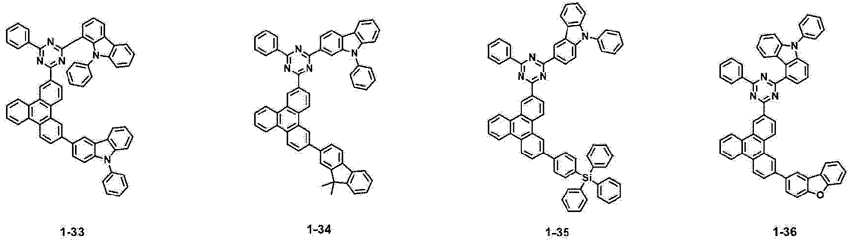

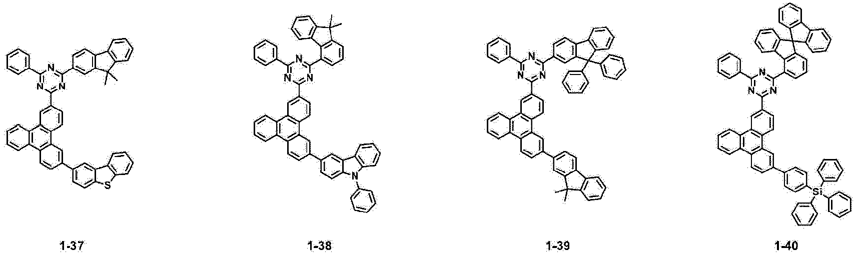

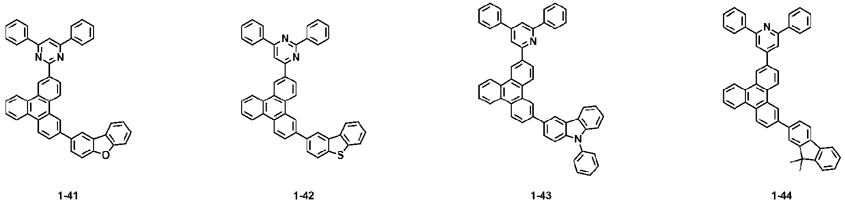

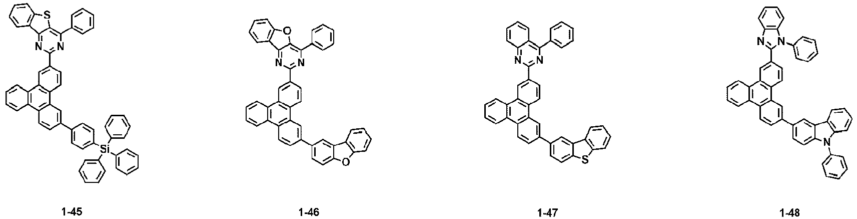

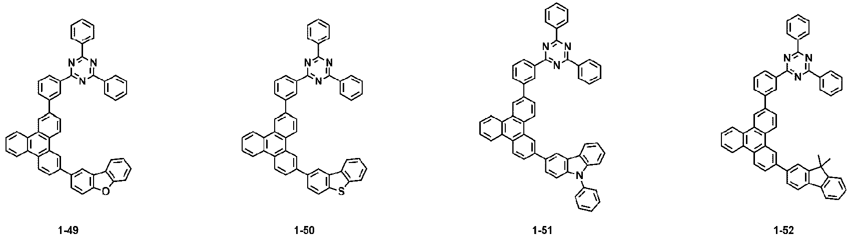

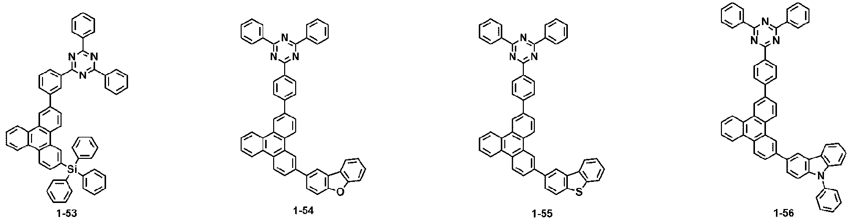

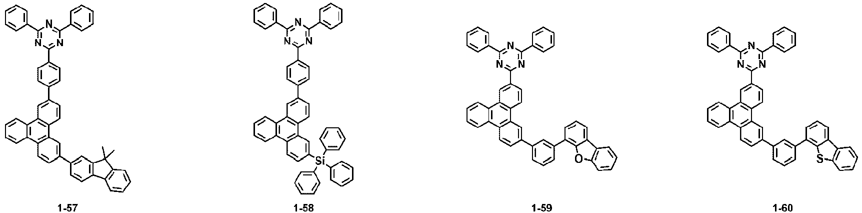

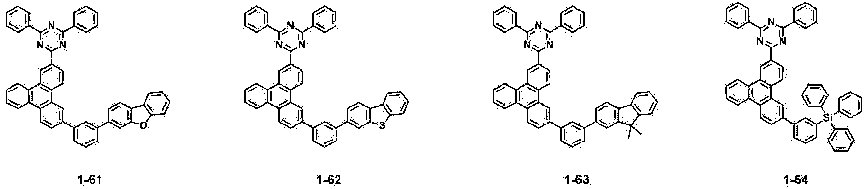

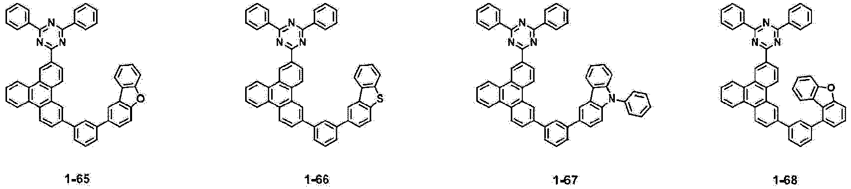

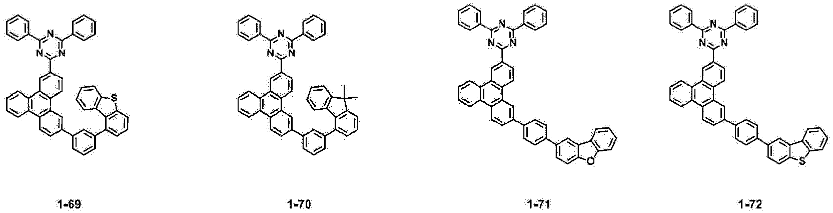

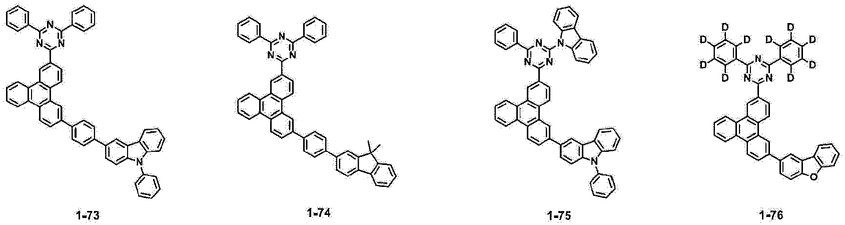

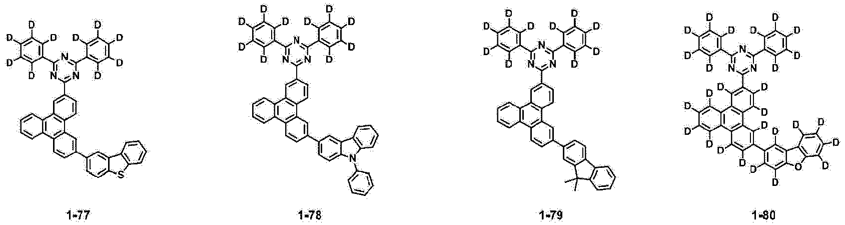

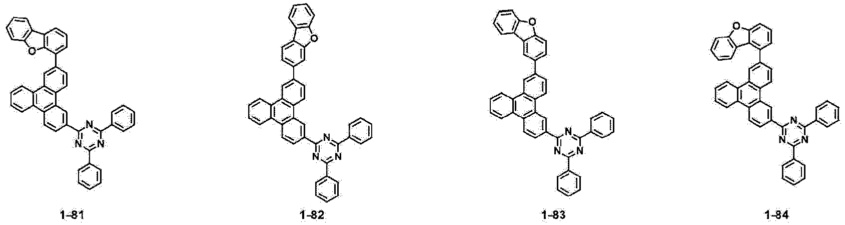

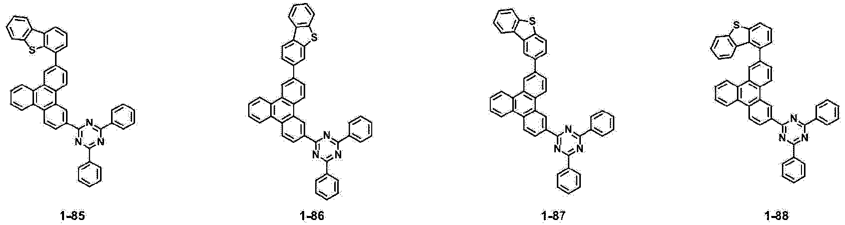

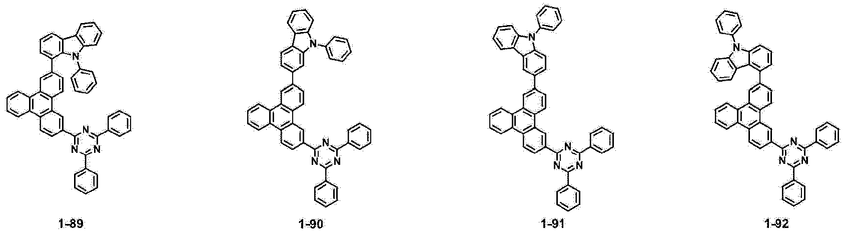

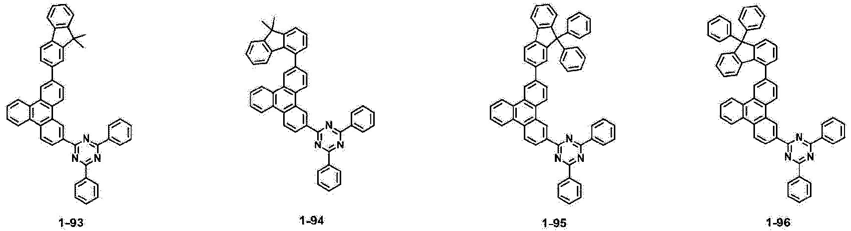

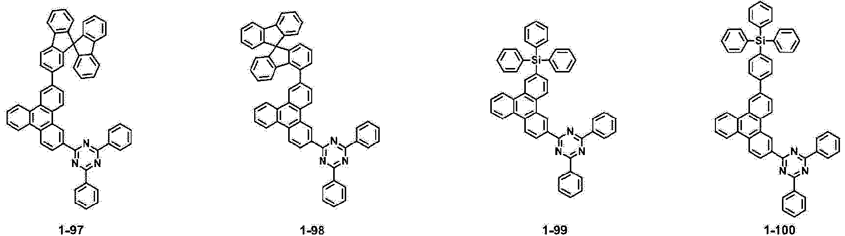

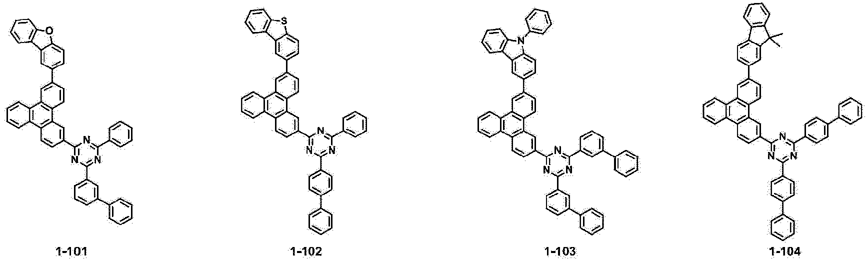

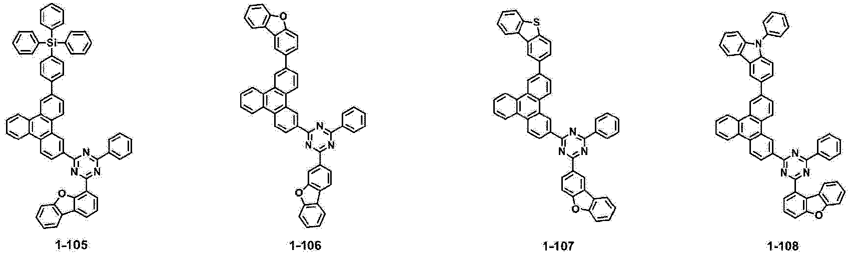

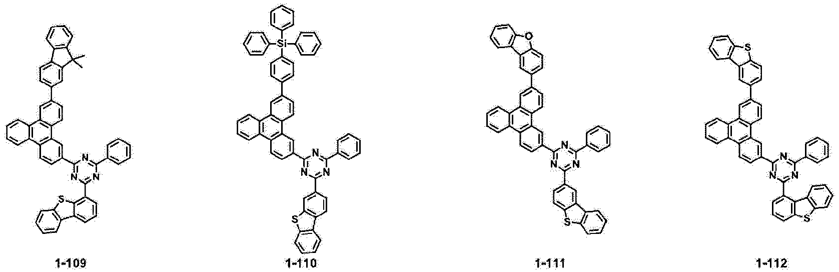

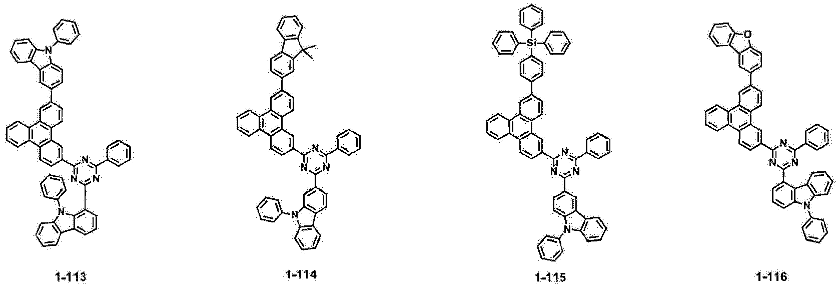

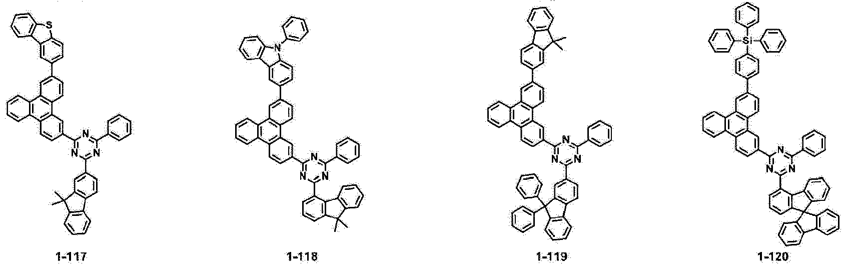

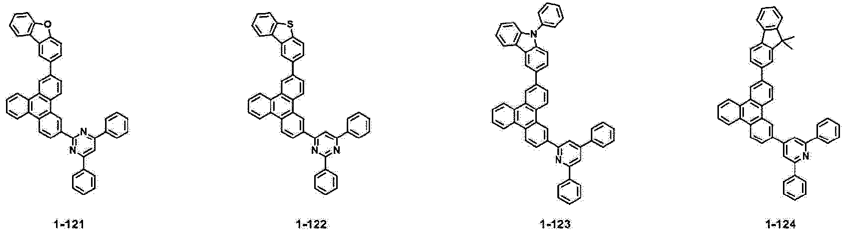

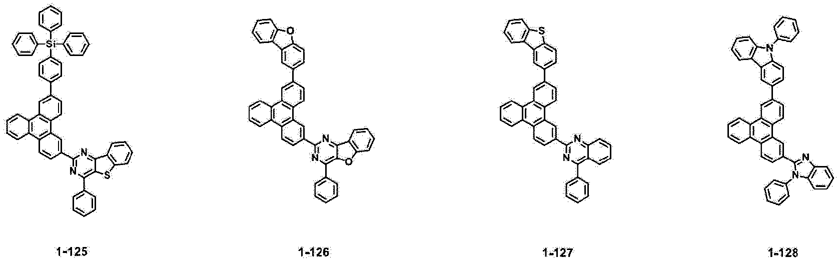

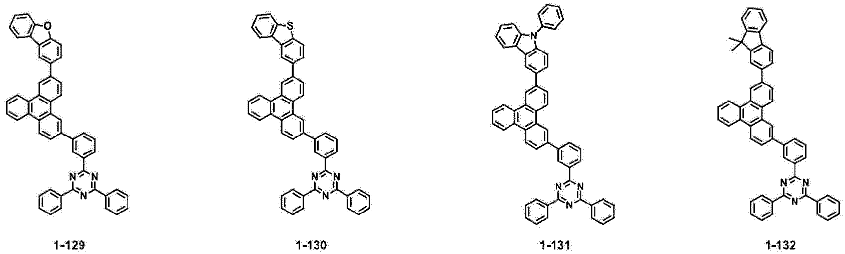

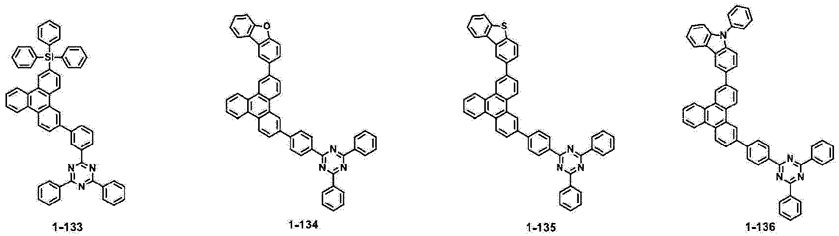

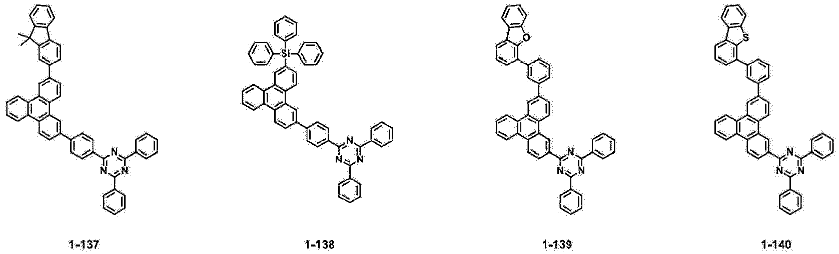

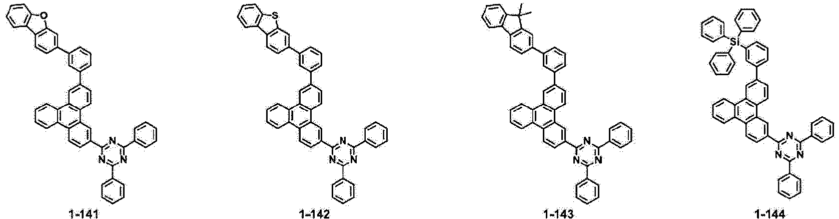

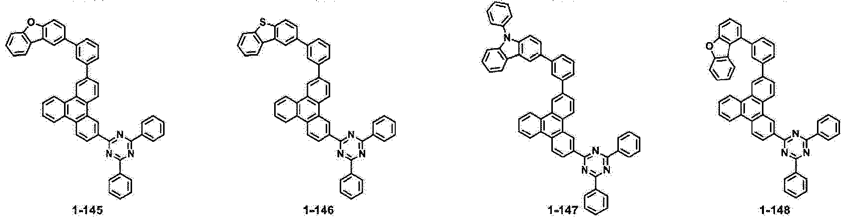

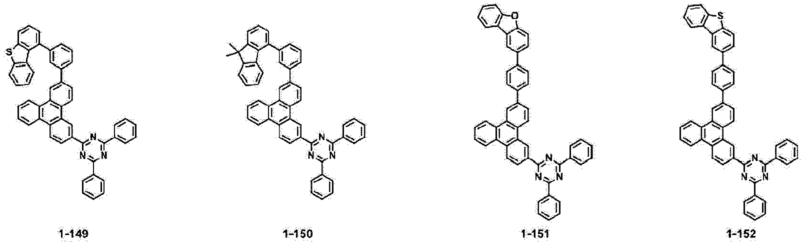

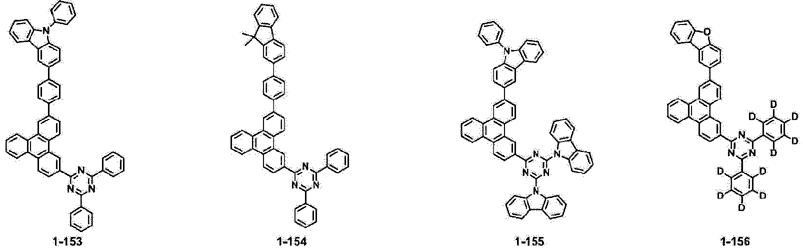

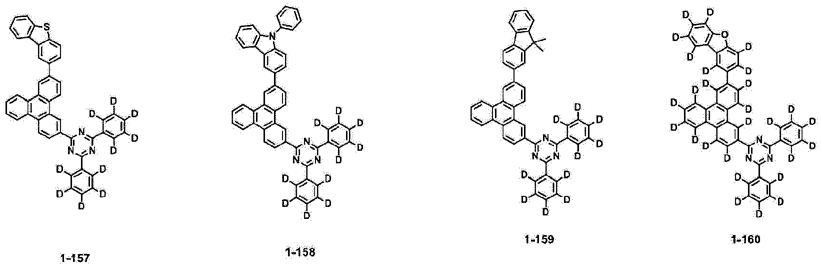

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

One embodiment of the present specification provides an organic light emitting device including a first electrode; a second electrode; and one or more organic material layers provided between the first electrode and the second electrode, wherein one or more layers of the organic material layers include one or more types of the compound of Chemical Formula 1.

In one embodiment of the present specification, one or more layers of the organic material layers include one type of the compound of Chemical Formula 1.

In one embodiment of the present specification, the first electrode may be an anode, and the second electrode may be a cathode.

In another embodiment of the present specification, the first electrode may be a cathode, and the second electrode may be an anode.

In one embodiment of the present specification, the organic light emitting device may be a blue organic light emitting device, and the compound of Chemical Formula 1 may be used as a material of the blue organic light emitting device. For example, the compound of Chemical Formula 1 may be included in a light emitting layer of the blue organic light emitting device.

In one embodiment of the present specification, the organic light emitting device may be a green organic light emitting device, and the compound of Chemical Formula 1 may be used as a material of the green organic light emitting device. For example, the compound of Chemical Formula 1 may be included in a light emitting layer of the green organic light emitting device.

In one embodiment of the present specification, the organic light emitting device may be a red organic light emitting device, and the compound of Chemical Formula 1 may be used as a material of the red organic light emitting device. For example, the compound of Chemical Formula 1 may be included in a light emitting layer of the red organic light emitting device.

The organic light emitting device of the present specification may be manufactured using common organic light emitting device manufacturing methods and materials except that one or more organic material layers are formed using the compound described above.

The compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present specification may be formed in a single layer structure, but may be formed in a multilayer structure in which two or more organic material layers are laminated. For example, the organic light emitting device of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present specification, the organic material layer includes a light emitting layer, and the light emitting layer may include the compound of Chemical Formula 1.

In the organic light emitting device of the present specification, the organic material layer includes a light emitting layer, the light emitting layer includes a host, and the host may include the compound of Chemical Formula 1.

In the organic light emitting device of the present specification, the organic material layer includes a light emitting layer, and the light emitting layer may further include, in addition to the compound of Chemical Formula 1, a compound of the following Chemical Formula 2 or 3.

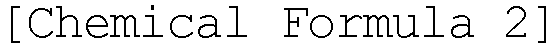

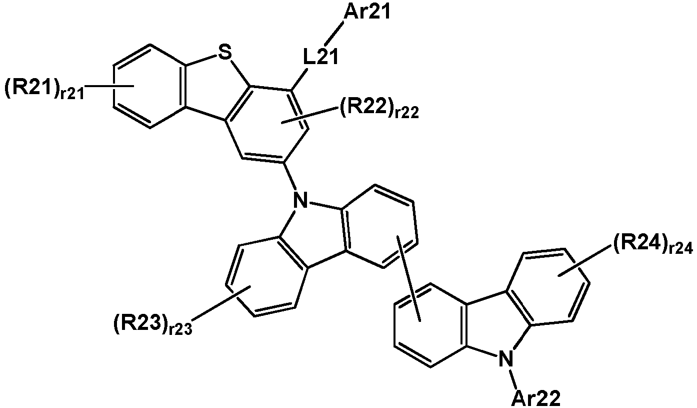

In Chemical Formula 2,
R21 to R24 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
L21 is a direct bond; a substituted or unsubstituted C6 to C60 arylene group; or a substituted or unsubstituted C2 to C60 heteroarylene group,
Ar21 and Ar22 are each independently a substituted or unsubstituted C6 to C60 aryl group; or a substituted or unsubstituted C2 to C60 heteroaryl group,
r21 is an integer of 1 to 4, and when 2 or greater, R21s are the same as or different from each other,
r22 is 1 or 2, and when 2, R22s are the same as or different from each other,
r23 is an integer of 1 to 4, and when 2 or greater, R23s are the same as or different from each other, and
r24 is an integer of 1 to 4, and when 2 or greater, R24s are the same as or different from each other, 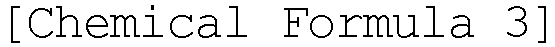 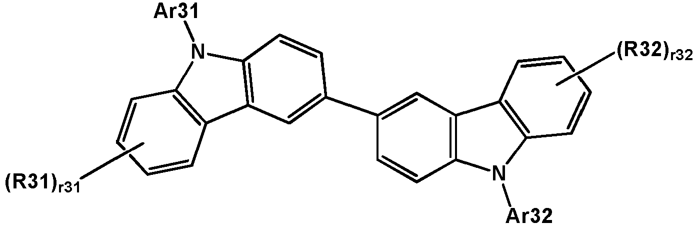
in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a C2 to C60 heteroaryl group substituted or unsubstituted and including O or N,
r31 is an integer of 1 to 4, and when 2 or greater, R31s are the same as or different from each other, and
r32 is an integer of 1 to 4, and when 2 or greater, R32s are the same as or different from each other.

In one embodiment of the present specification, R21 to R24 of Chemical Formula 2 are each independently hydrogen; deuterium; a substituted or unsubstituted C6 to C60 aryl group; or a substituted or unsubstituted C2 to C60 heteroaryl group.

In one embodiment of the present specification, R21 to R24 of Chemical Formula 2 are each independently hydrogen; deuterium; a substituted or unsubstituted C6 to C30 aryl group; or a substituted or unsubstituted C2 to C30 heteroaryl group.

In one embodiment of the present specification, R21 to R24 of Chemical Formula 2 are each independently hydrogen; deuterium; or a substituted or unsubstituted C6 to C30 aryl group.

In one embodiment of the present specification, R21 to R24 of Chemical Formula 2 are hydrogen; or deuterium.

In one embodiment of the present specification, R21 to R24 of Chemical Formula 2 are hydrogen.

In one embodiment of the present specification, L21 of Chemical Formula 2 is a direct bond; a substituted or unsubstituted C6 to C30 arylene group; or a substituted or unsubstituted C2 to C30 heteroarylene group.

In one embodiment of the present specification, L21 of Chemical Formula 2 is a direct bond; or a substituted or unsubstituted C6 to C30 arylene group.

In one embodiment of the present specification, L21 of Chemical Formula 2 is a direct bond; or a substituted or unsubstituted C6 to C20 arylene group.

In one embodiment of the present specification, L21 of Chemical Formula 2 is a direct bond; or a substituted or unsubstituted phenylene group.

In one embodiment of the present specification, L21 of Chemical Formula 2 is a direct bond; or a phenylene group.

In one embodiment of the present specification, Ar21 and Ar22 of Chemical Formula 2 are each independently a substituted or unsubstituted C6 to C40 aryl group; or a substituted or unsubstituted C2 to C40 heteroaryl group.

In one embodiment of the present specification, Ar21 and Ar22 of Chemical Formula 2 are each independently a substituted or unsubstituted C6 to C30 aryl group; or a substituted or unsubstituted C2 to C30 heteroaryl group.

In one embodiment of the present specification, Ar21 and Ar22 of Chemical Formula 2 are each independently a substituted or unsubstituted C6 to C30 aryl group; or a C2 to C30 heteroaryl group substituted or unsubstituted and including O or S.

In one embodiment of the present specification, Ar21 and Ar22 of Chemical Formula 2 are each independently a substituted or unsubstituted phenyl group; a substituted or unsubstituted biphenyl group; a substituted or unsubstituted naphthyl group; a substituted or unsubstituted fluorenyl group; a substituted or unsubstituted dibenzofuran group; or a substituted or unsubstituted dibenzothiophene group.

In one embodiment of the present specification, Ar21 and Ar22 of Chemical Formula 2 are each independently a phenyl group; a biphenyl group; a naphthyl group; a fluorenyl group unsubstituted or substituted with an alkyl group or an aryl group; a dibenzofuran group; or a dibenzothiophene group.

In one embodiment of the present specification, Ar21 and Ar22 of Chemical Formula 2 are each independently a phenyl group; a biphenyl group; a naphthyl group; a fluorenyl group unsubstituted or substituted with an alkyl group; a dibenzofuran group; or a dibenzothiophene group.

In one embodiment of the present specification, Ar21 of Chemical Formula 2 is a phenyl group; a biphenyl group; a naphthyl group; a fluorenyl group unsubstituted or substituted with an alkyl group; a dibenzofuran group; or a dibenzothiophene group.

In one embodiment of the present specification, Ar22 of Chemical Formula 2 is a phenyl group.

In one embodiment of the present specification, Chemical Formula 2 may be represented by any one of the following Chemical Formulae 2-1 to 2-3.

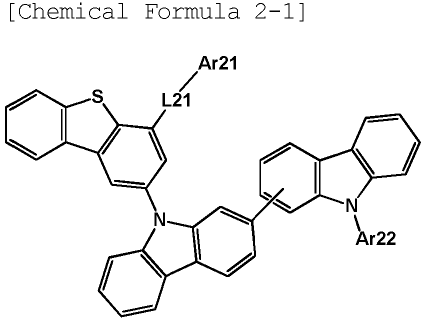

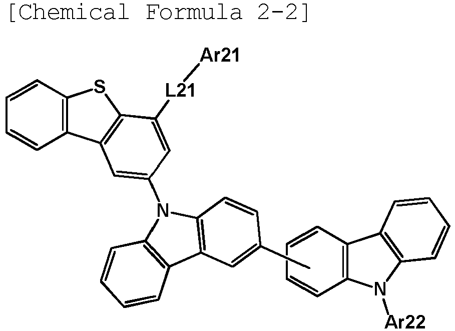

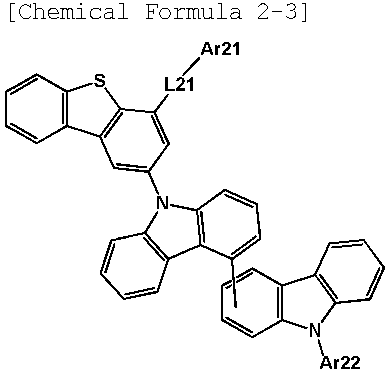

In Chemical Formulae 2-1 to 2-3,
each substituent has the same definition as in Chemical Formula 2.

In one embodiment of the present specification, Chemical Formula 2 may be represented by any one of the following compounds, but is not limited thereto.

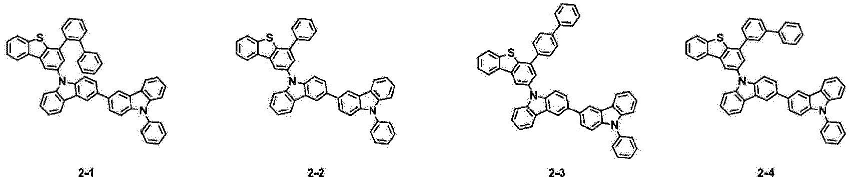

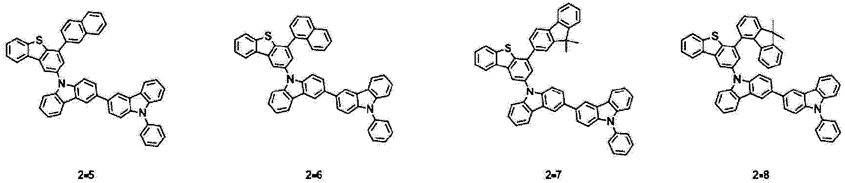

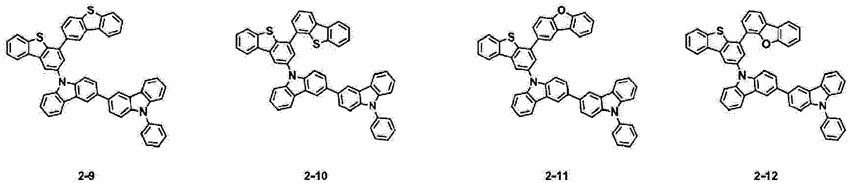

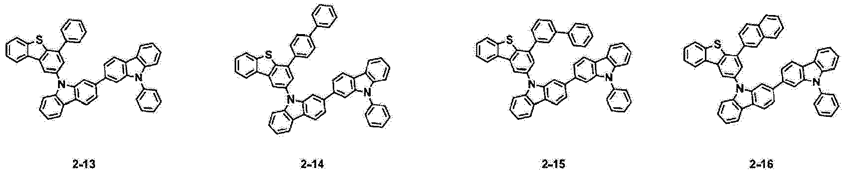

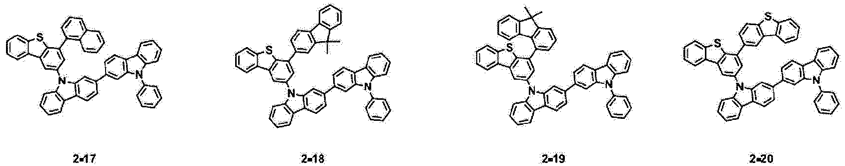

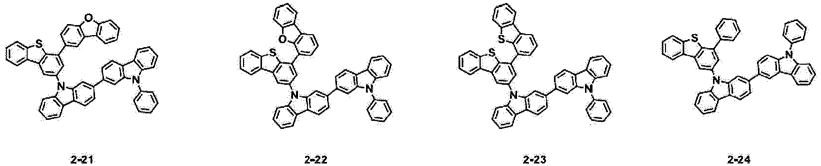

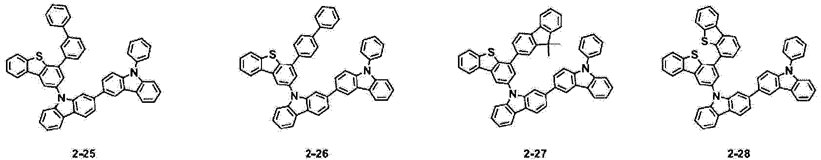

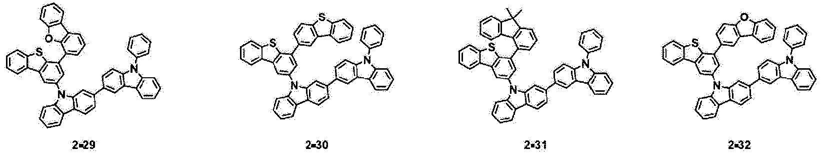

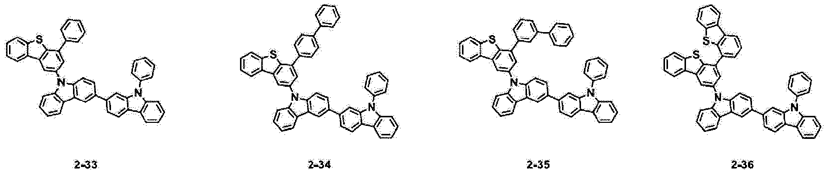

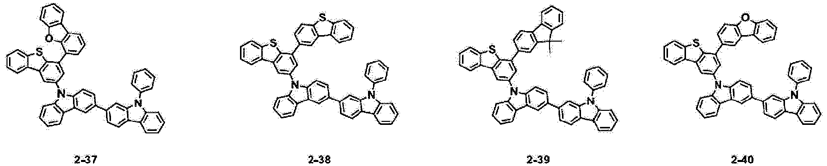

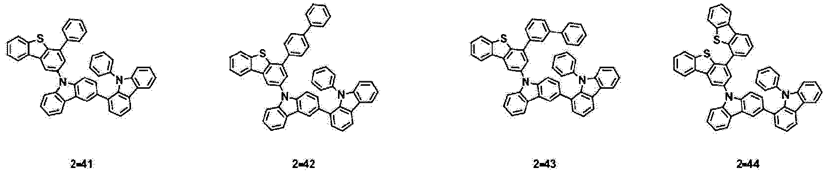

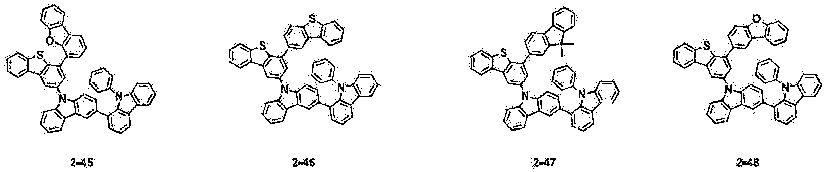

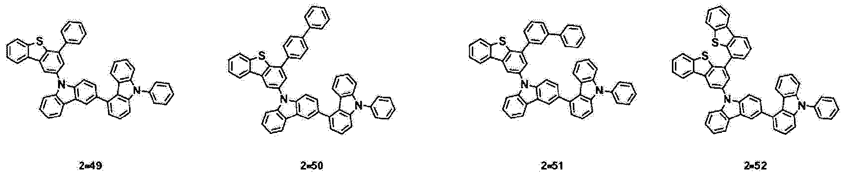

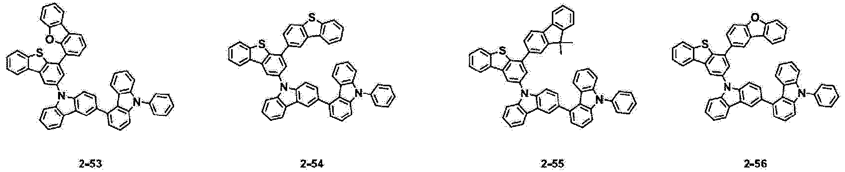

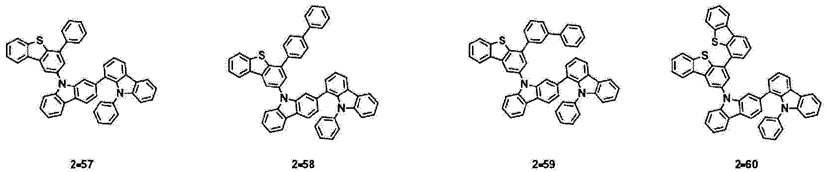

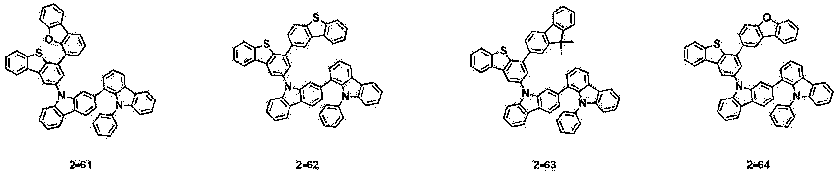

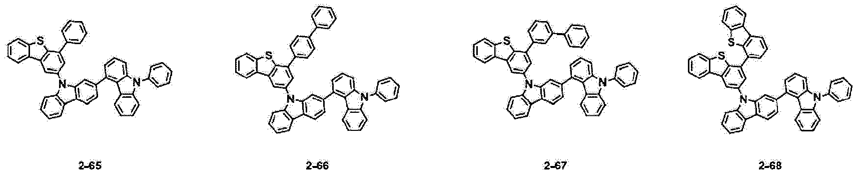

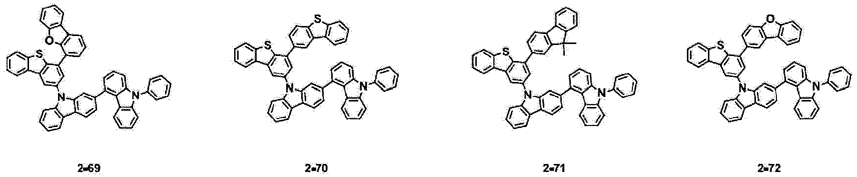

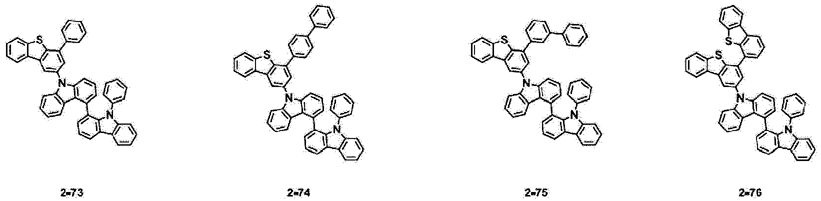

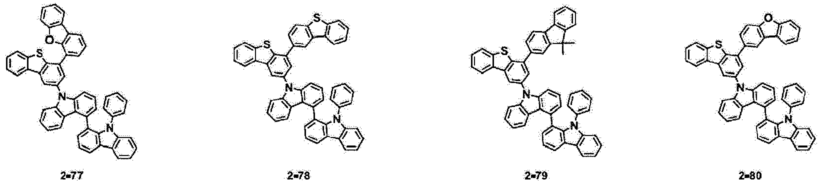

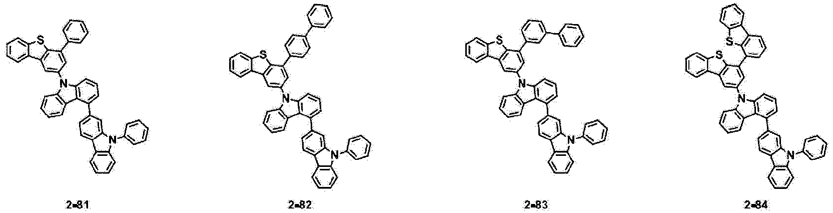

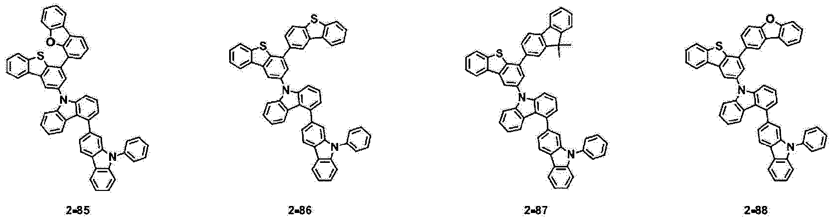

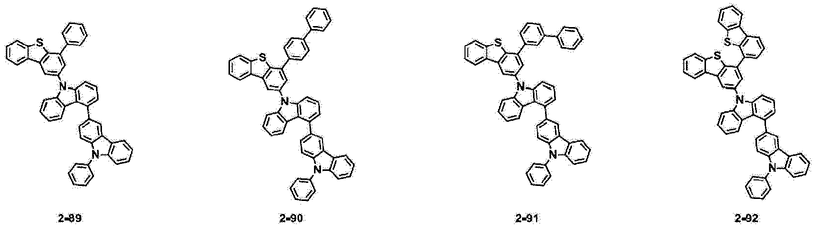

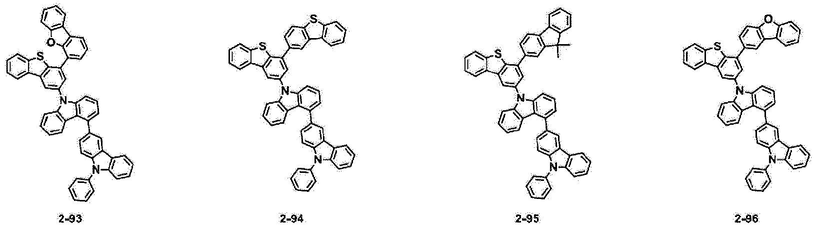

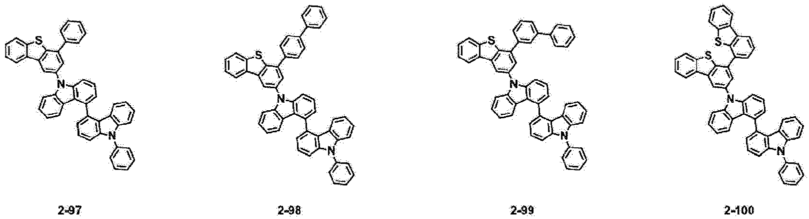

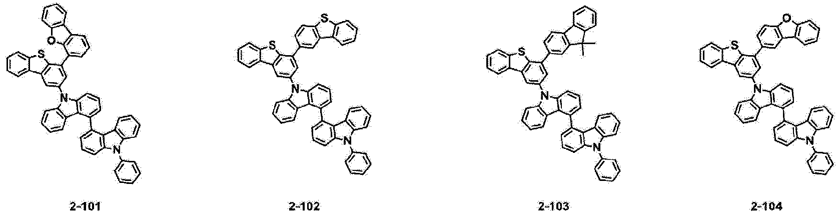

In one embodiment of the present specification, R31 and R32 of Chemical Formula 3 are each independently hydrogen; deuterium; a substituted or unsubstituted C6 to C60 aryl group; or a substituted or unsubstituted C2 to C60 heteroaryl group.

In one embodiment of the present specification, R31 and R32 of Chemical Formula 3 are each independently hydrogen; deuterium; a substituted or unsubstituted C6 to C30 aryl group; or a substituted or unsubstituted C2 to C30 heteroaryl group.

In one embodiment of the present specification, R31 and R32 of Chemical Formula 3 are each independently hydrogen; deuterium; or a substituted or unsubstituted C6 to C30 aryl group.

In one embodiment of the present specification, R31 and R32 of Chemical Formula 3 are hydrogen; or deuterium.

In one embodiment of the present specification, R31 and R32 of Chemical Formula 3 are hydrogen.

In one embodiment of the present specification, Ar31 and Ar32 of Chemical Formula 3 are each independently a substituted or unsubstituted C6 to C40 aryl group; or a C2 to C40 heteroaryl group substituted or unsubstituted and including O **or N.**

In one embodiment of the present specification, Ar31 and Ar32 of Chemical Formula 3 are each independently a substituted or unsubstituted C6 to C40 aryl group.

In one embodiment of the present specification, Ar31 and Ar32 of Chemical Formula 3 are each independently a substituted or unsubstituted C6 to C30 aryl group.

In one embodiment of the present specification, Ar31 and Ar32 of Chemical Formula 3 are each independently a substituted or unsubstituted phenyl group; a substituted or unsubstituted biphenyl group; a substituted or unsubstituted terphenyl group; a substituted or unsubstituted naphthyl group; a substituted or unsubstituted fluorenyl group; or a substituted or unsubstituted triphenylene group.

In one embodiment of the present specification, Ar31 and Ar32 of Chemical Formula 3 are each independently a phenyl group unsubstituted or substituted with a cyano group, a silyl group or an aryl group; a biphenyl group; a terphenyl group; a naphthyl group; a fluorenyl group unsubstituted or substituted with an alkyl group or an aryl group; 9,9'-spirobi[fluorene]; or a triphenylene group.

In one embodiment of the present specification, Ar31 and Ar32 of Chemical Formula 3 are each independently a phenyl group unsubstituted or substituted with a cyano group, a triphenylsilyl group or an aryl group; a biphenyl group; a terphenyl group; a naphthyl group; a fluorenyl group unsubstituted or substituted with an alkyl group or an aryl group; 9,9'-spirobi[fluorene]; or a triphenylene group.

In one embodiment of the present specification, Chemical Formula 3 may be represented by any one of the following compounds, but is not limited thereto.

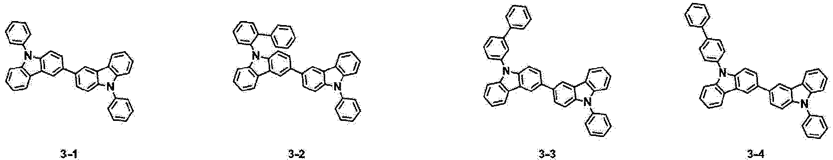

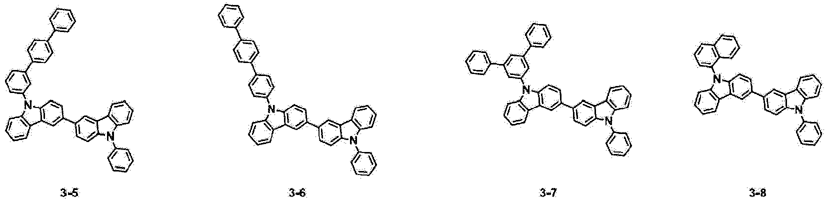

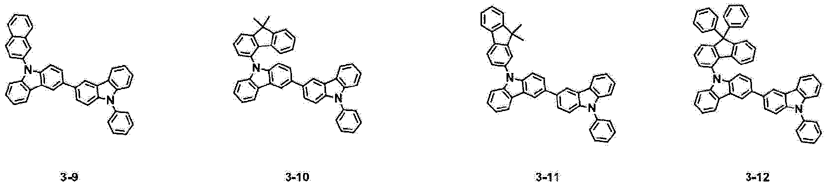

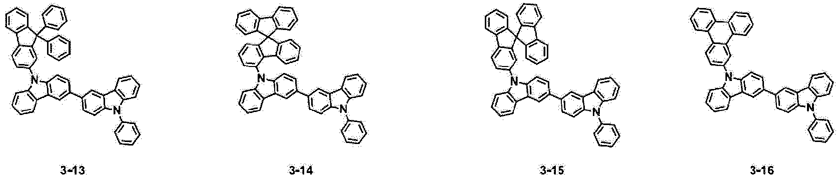

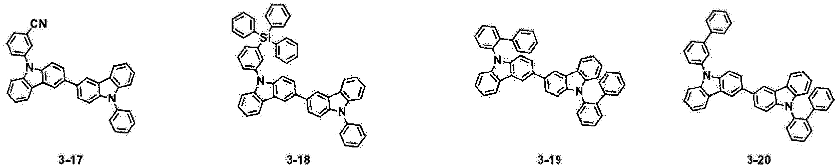

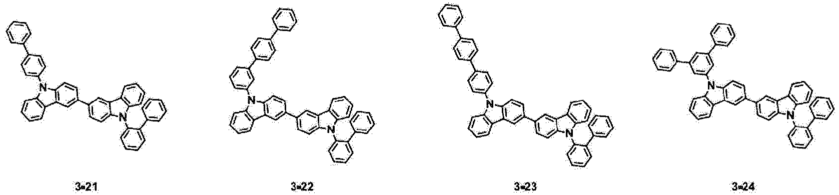

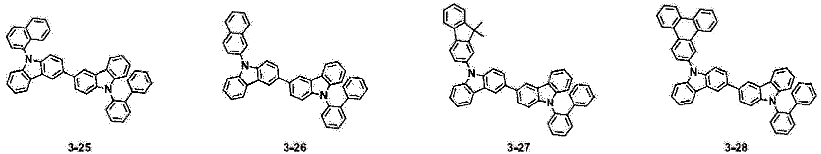

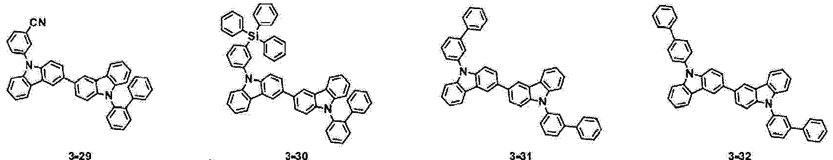

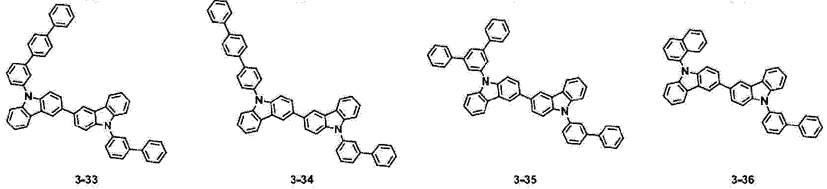

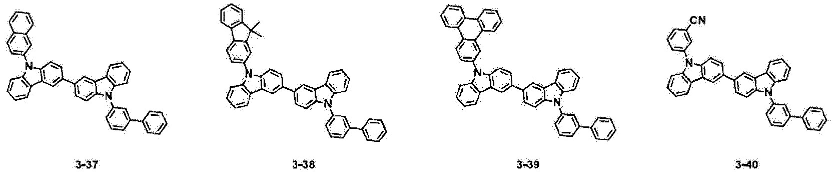

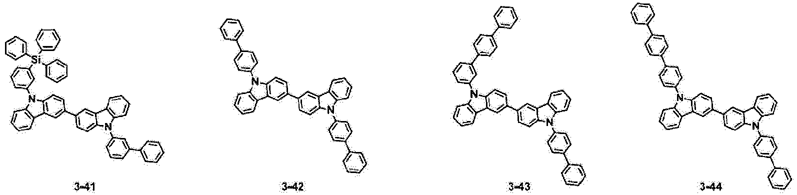

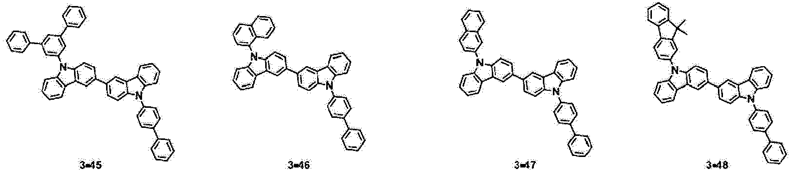

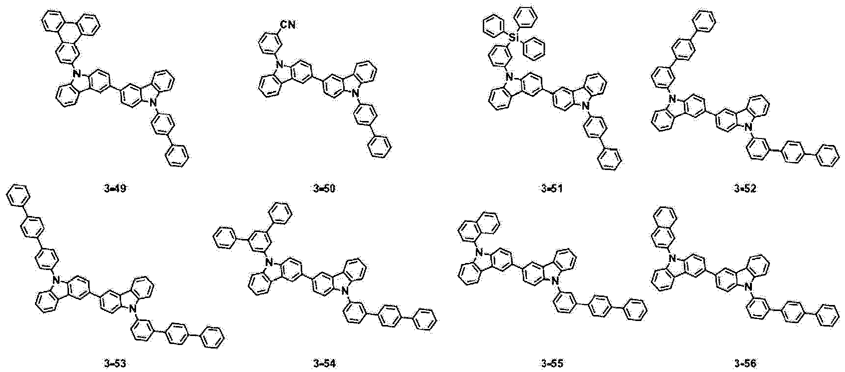

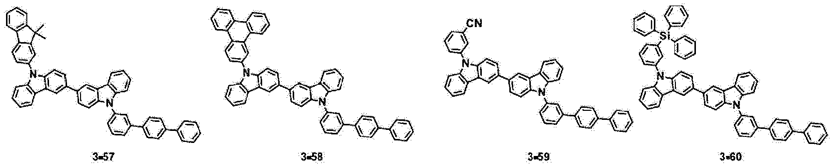

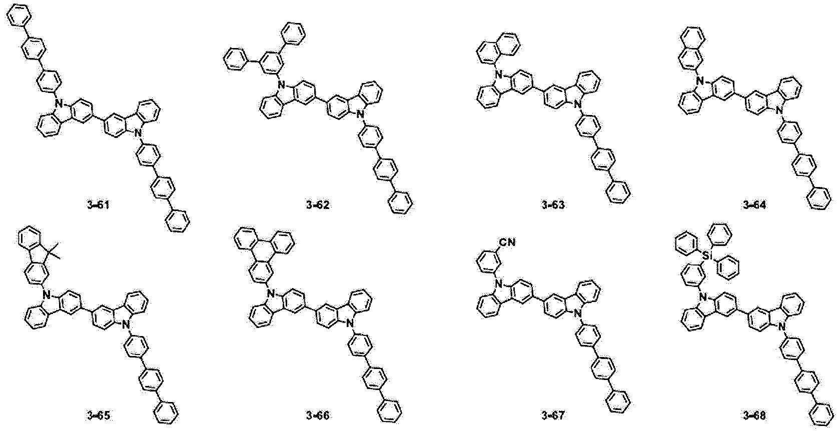

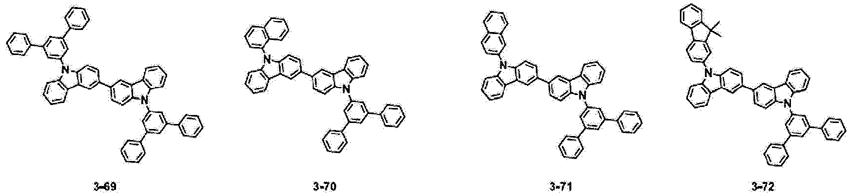

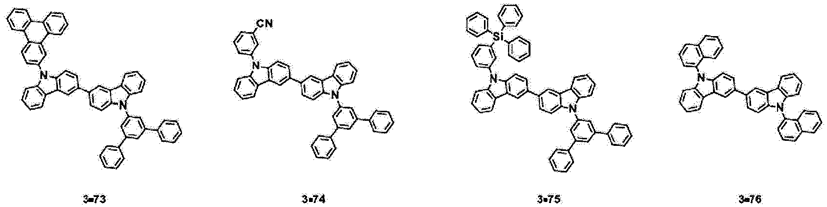

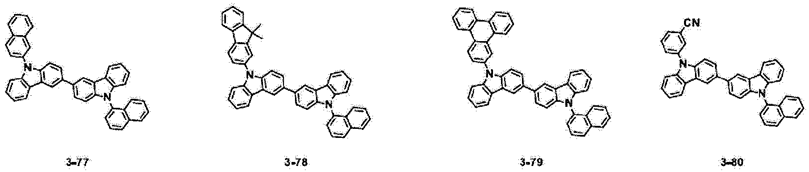

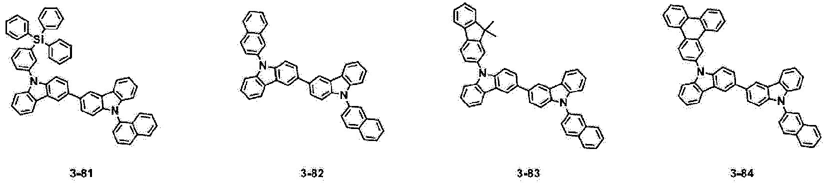

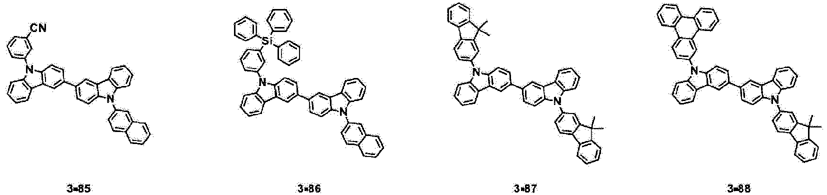

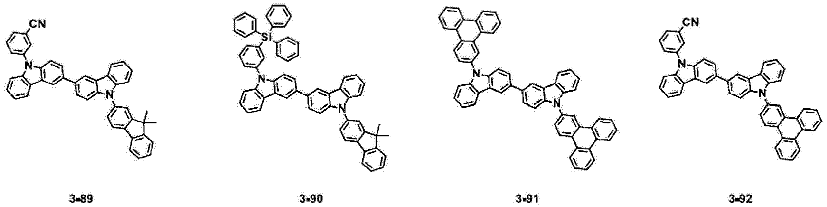

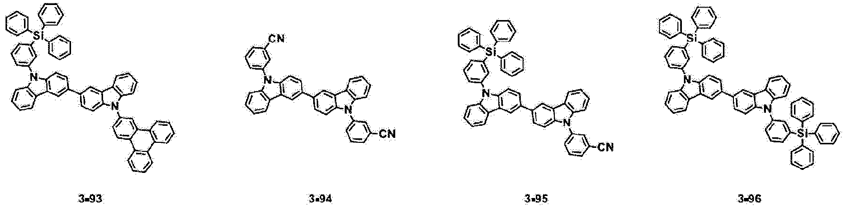

In one embodiment of the present specification, the organic material layer includes the compound of Chemical Formula 1 and the compound of Chemical Formula 2 or 3 in a weight ratio of 1:10 to 10:1, a weight ratio of 1:8 to 8:1, a weight ratio of 1:5 to 5:1, and a weight ratio of 1:2 to 2:1.

When the compound of Chemical Formula 1 and the compound of Chemical Formula 2 or 3 are included in the above-described weight ratio ranges, an organic light emitting device having a low driving voltage, and superior light emission efficiency and lifetime may be provided. Particularly, when these compounds are included in a weight ratio of 1:2 to 2:1, an organic light emitting device having high light emission efficiency and lifetime is obtained.

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 3 illustrate a lamination order of electrodes and organic material layers of the organic light emitting device according to one embodiment of the present specification. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer including the compound of Chemical Formula 1 may further include other materials as necessary.

In the organic light emitting device according to one embodiment of the present specification, materials other than the compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris (4-carbazoyl-9-ylphenyl)amine (TCTA), 4,4',4"-tri[phenyl (m-tolyl)amino]triphenylamine (m-MTDATA) or 1,3,5-tris[4-(3-methylphenylphenylamino) phenyl] benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present specification may be a top-emission type, a bottom-emission type or a dual-emission type depending on the materials used.

The compound according to one embodiment of the present specification may also be used in an organic electronic device including an organic solar cell, an organic photo conductor, an organic transistor and the like under a similar principle used in the organic light emitting device.

One embodiment of the present specification provides a composition for forming an organic material layer, the composition including the compound of Chemical Formula 1; and the compound of Chemical Formula 2 or 3.

The composition for forming an organic material layer according to one embodiment of the present specification includes the compound of Chemical Formula 1 and the compound of Chemical Formula 2 or 3 in a weight ratio of 1:10 to 10:1, a weight ratio of 1:8 to 8:1, a weight ratio of 1:5 to 5:1, and a weight ratio of 1:2 to 2:1.

When the compound of Chemical Formula 1 and the compound of Chemical Formula 2 or 3 are included in the above-described weight ratio ranges, an organic light emitting device having a low driving voltage, and superior light emission efficiency and lifetime may be provided. Particularly, when these compounds are included in a weight ratio of 1:2 to 2:1, an organic light emitting device having high light emission efficiency and lifetime is obtained.

The composition for forming an organic material layer according to one embodiment of the present specification may be used as a material of a light emitting layer of an organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 1-3

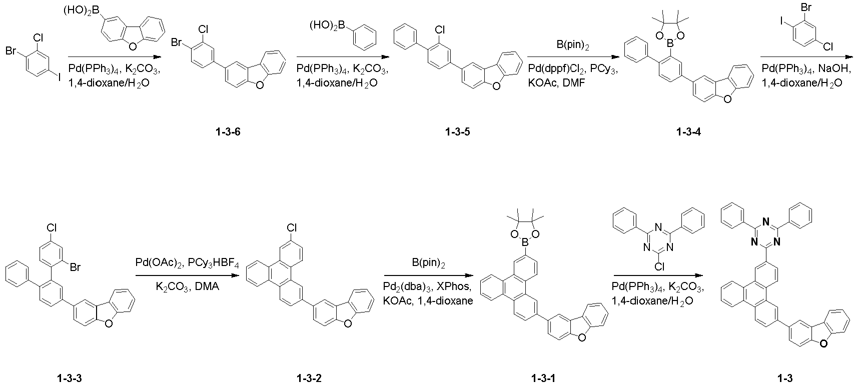

### 1) Preparation of Compound 1-3-6

1-Bromo-2-chloro-4-iodobenzene (20.0 g, 63.0 mM), dibenzo[b,d]furan-2-ylboronic acid (11.1 g, 52.5 mM), Pd(PPh₃)₄ (tetrakis(triphenylphosphine)palladium(0)) (3.0 g, 2.6 mM) and K₂CO₃ (14.5 g, 105.0 mM) were dissolved in 1,4-dioxane/H₂O (400 mL/80 mL), and then refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:hexane=1:10) to obtain Compound 1-3-6 (14.4 g, 80%).

### 2) Preparation of Compound 1-3-5

Compound 1-3-6 (13.4 g, 37.4 mM), phenylboronic acid (5.5 g, 44.9 mM), Pd(PPh₃)₄ (2.2 g, 1.9 mM) and K₂CO₃ (10.3 g, 74.8 mM) were dissolved in 1,4-dioxane/H₂O (200 mL/40 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:10) to obtain Compound 1-3-5 (10.8 g, 85%).

### 3) Preparation of Compound 1-3-4

Compound 1-3-5 (6.8 g, 19.2 mM), bis (pinacolato)diboron (7.3 g, 28.8 mM), Pd(dppf)Cl₂ ([1,1'-bis(diphenylphosphino)ferrocene]dichloropalladium(II)) (702 mg, 1.0 mM), tricyclohexylphosphine (PCy₃) (533 mg, 1.9 mM) and potassium acetate (KOAc) (5.6 g, 57.3 mM) were dissolved in DMF (100 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain Compound 1-3-4 (7.1 g, 85%).

### 4) Preparation of Compound 1-3-3

Compound 1-3-4 (16.7 g, 37.4 mM), 2-bromo-4-chloro-1-iodobenzene (14.2 g, 44.9 mM), Pd(PPh₃)₄ (2.2 g, 1.9 mM) and NaOH (3.0 g, 74.8 mM) were dissolved in 1,4-dioxane/H₂O (200 mL/40 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:10) to obtain Compound 1-3-3 (15.8 g, 85%).

### 5) Preparation of Compound 1-3-2

Compound 1-3-3 (14.1 g, 27.7 mM), Pd(OAc)₂ (palladium(II) acetate) (622 mg, 2.8 mM), PCy₃·HBF₄ (tricyclohexylphosphine tetrafluoroborate) (2.0 g, 5.5 mM) and K₂CO₃ (7.7 g, 55.4 mM) were dissolved in dimethylacetamide (DMA) (100 mL), and then refluxed for 12 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:10) to obtain Compound 1-3-2 (8.0 g, 70%).

### 6) Preparation of Compound 1-3-1

Compound 1-3-2 (8.2 g, 19.2 mM), bis(pinacolato)diboron (7.3 g, 28.8 mM), Pd₂(dba)₃ (879 mg, 1.0 mM), Xphos (2-dicyclohexylphosphino-2',4',6'-triisopropylbiphenyl) (915 mg, 1.9 mM) and KOAc (5.6 g, 57.3 mM) were dissolved in 1,4-dioxane (100 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain Compound 1-3-1 (8.3 g, 85%).

### 7) Preparation of Compound 1-3

Compound 1-3-1 (8.4 g, 16.1 mM), 2-chloro-4,6-diphenyl-1,3,5-triazine (4.7 g, 17.7 mM), Pd(PPh₃)₄ (0.9 g, 0.8 mM) and K₂CO₃ (4.5 g, 32.3 mM) were dissolved in 1,4-dioxane/H₂O (200 mL/40 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain target Compound 1-3 (8.1 g, 82%).

Target compounds were synthesized in the same manner as in Preparation Example 1 except that Intermediate A of the following Table 1 was used instead of 1-bromo-2-chloro-4-iodobenzene, Intermediate B of the following Table 1 was used instead of dibenzo[b,d]furan-2-ylboronic acid, Intermediate C of the following Table 1 was used instead of 2-bromo-4-chloro-1-iodobenzene, and Intermediate D of the following Table 1 was used instead of 2-chloro-4,6-diphenyl-1,3,5-triazine.

**[Table 1]**

| Compo und No. | Intermed iate A | Intermed iate B | Intermed iate C | Intermedia te D | Target Compound |
|---|---|---|---|---|---|
| 1-7 | 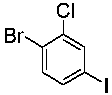 | 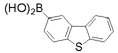 | 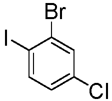 | 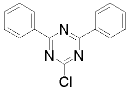 | 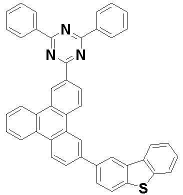 |
| 1-11 | 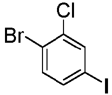 | 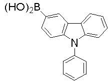 | 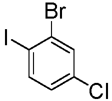 | 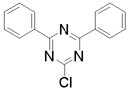 | 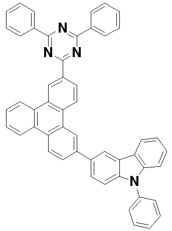 |
| 1-14 | 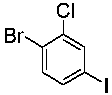 | 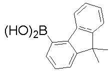 | 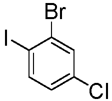 | 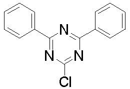 | 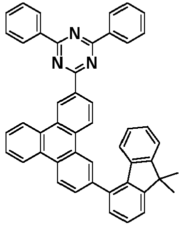 |
| 1-26 | 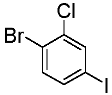 | 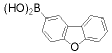 | 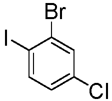 | 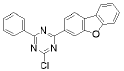 | 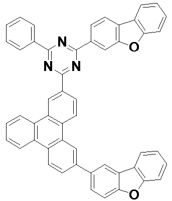 |
| 1-51 | 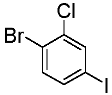 | 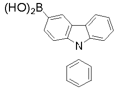 | 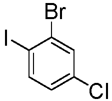 | 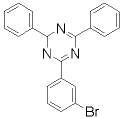 | 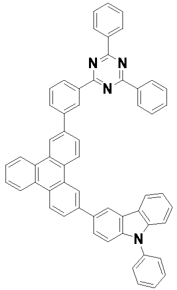 |
| 1-56 | 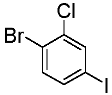 | 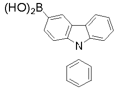 | 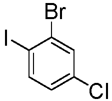 | 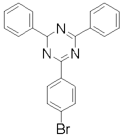 | 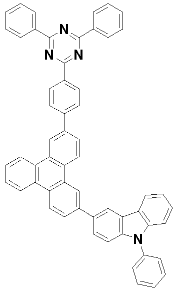 |
| 1-60 | 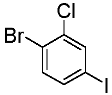 | 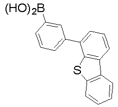 | 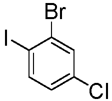 | 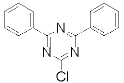 | 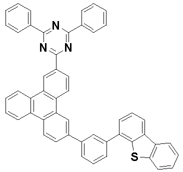 |
| 1-76 | 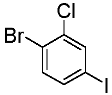 | 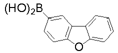 | 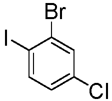 | 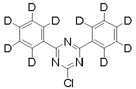 | 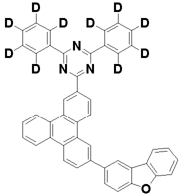 |
| 1-83 | 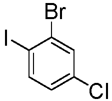 | 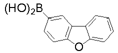 | 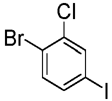 | 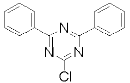 | 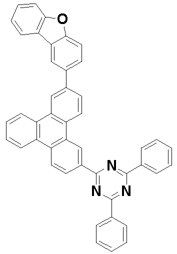 |
| 1-87 | 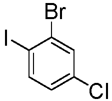 | 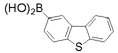 | 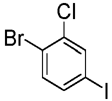 | 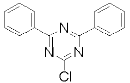 | 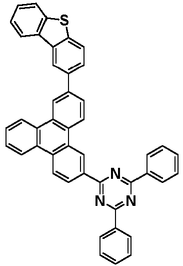 |
| 1-91 | 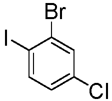 | 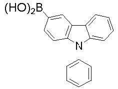 | 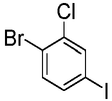 | 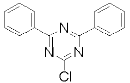 | 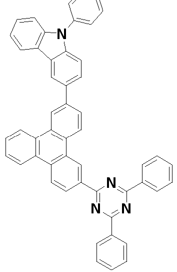 |

### <Preparation Example 2> Synthesis of Compound 2-2

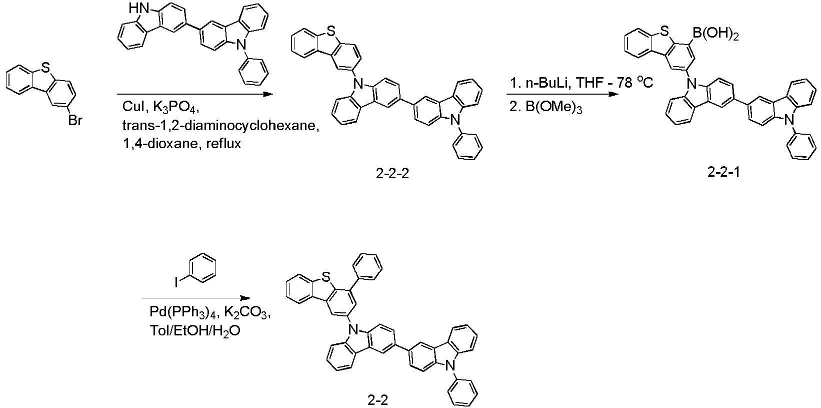

### 1) Preparation of Compound 2-2-2

2-Bromodibenzo[b,d]thiophene (4.2 g, 15.8 mM), 9-phenyl-9H,9'H-3,3'-bicarbazole (6.5 g, 15.8 mM), CuI (3.0 g, 15.8 mM), trans-1, 2-diaminocyclohexane (1.9 mL, 15.8 mM) and K₃PO₄ (3.3 g, 31.6 mM) were dissolved in 1,4-dioxane (100 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain Compound 2-2-2 (7.9 g, 85%).

### 2) Preparation of Compound 2-2-1

To a mixture solution obtained by introducing Compound 2-2-2 (8.4 g, 14.3 mmol) and tetrahydrofuran (THF) (100 mL), 2.5 M n-butyllithium (n-BuLi) (7.4 mL, 18.6 mmol) was added dropwise at -78°C, and the result was stirred for 1 hour at room temperature. Trimethyl borate (4.8 mL, 42.9 mmol) was added dropwise to the reaction mixture, and the result was stirred for 2 hours at room temperature. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:methanol (MeOH)=100:3), and recrystallized with DCM to obtain Compound 2-2-1 (3.9 g, 70%).

### 3) Preparation of Compound 2-2

Compound 2-2-1 (6.7 g, 10.5 mM), iodobenzene (2.1 g, 10.5 mM), Pd(PPh₃)₄ (606 mg, 0.52 mM) and K₂CO₃ (2.9 g, 21.0 mM) were dissolved in toluene/ethanol (EtOH)/H₂O (100 mL/20 mL/20 mL), and then refluxed for 12 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target compound 2-2 (4.9 g, 70%).

### <Preparation Example 2-1> Synthesis of Compound 2-3

Target Compound 2-3 (83%) was obtained in the same manner as in Preparation of Compound 2-2 of Preparation Example 2 except that 4-iodo-1,1'-biphenyl was used instead of iodobenzene.

### <Preparation Example 2-2> Synthesis of Compound 2-12

Target Compound 2-12 (80%) was obtained in the same manner as in Preparation of Compound 2-2 of Preparation Example 2 except that 4-iododibenzo[b,d]furan was used instead of iodobenzene.

### <Preparation Example 3> Synthesis of Compound 3-3

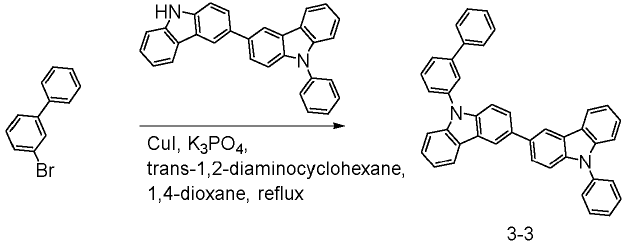

### 1) Preparation of Compound 3-3

3-Bromo-1,1'-biphenyl (3.7 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) were dissolved in 1,4-dioxane (100 mL), and then refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 3-3 (7.5 g, 85%).

Target compounds were synthesized in the same manner as in Preparation Example 3 except that Intermediate A of the following Table 2 was used instead of 3-bromo-1,1'-biphenyl, and Intermediate B of Table 2 was used instead of 9-phenyl-9H,9'H-3,3'-bicarbazole.

**[Table 2]**

| Compound No. | Intermediate A | Intermediate B | Target Compound |
|---|---|---|---|
| 3-4 | 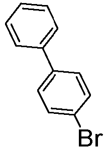 | 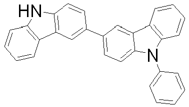 | 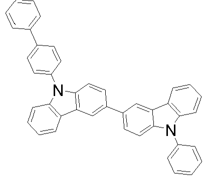 |
| 3-7 | 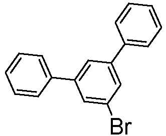 | 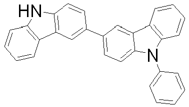 | 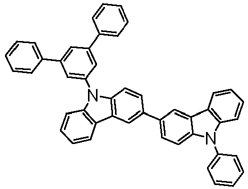 |
| 3-9 | 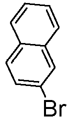 | 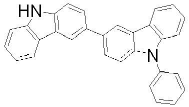 | 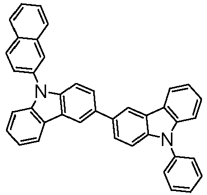 |
| 3-31 | 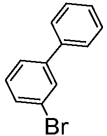 | 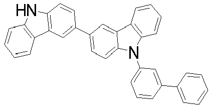 | 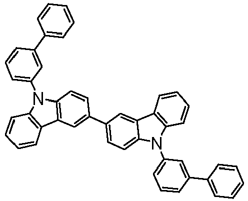 |
| 3-32 | 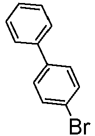 | 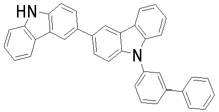 | 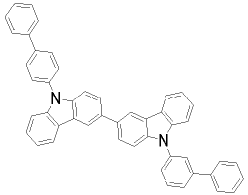 |
| 3-42 | 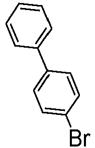 | 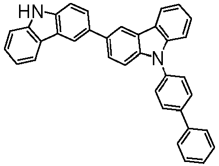 | 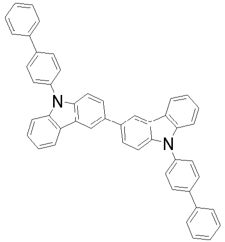 |

Compounds other than the compounds described in Preparation Examples 1 to 3 and Tables 1 and 2 were also prepared in the same manner as in the preparation examples described above, and the synthesis results are shown in the following Table 3 and Table 4.

**[Table 3]**

| Compoun d No. | ¹H NMR (CDCl₃, 200 Mz) |
|---|---|
| 1-3 | δ=9.60 (1H, d), 9.27 (2H, s), 8.37-8.30 (7H, m), 8.15 (1H, d), 7.98 (1H, d), 7.88-7.79 (3H, m), 7.70-7.64 (2H, m), 7.54-7.50 (8H, m), 7.39-7.31 (2H, m) |
| 1-7 | δ=9.60 (1H, d), 9.27 (2H, s), 8.45-8.30 (8H, m), 8.15-8.12 (3H, m), 7.99-7.93 (2H, m), 7.70-7.49 (11H, m) |
| 1-11 | δ=9.60 (1H, d), 9.27 (2H, s), 8.37-8.30 (8H, m), 8.19-8.13 (3H, m), 7.89 (1H, s), 7.70-7.50 (16H, m), 7.20 (1H, t) |
| 1-14 | δ=9.60 (1H, d), 9.27 (2H, s), 8.37-8.30 (7H, m), 8.15 (1H, d), 7.90 (1H, d), 7.78-7.64 (4H, m), 7.55-7.38 (10H, m), 7.28 (1H, t), 1.69 (6H, s) |
| 1-26 | δ=9.60 (1H, d), 9.27 (2H, s), 8.37-8.30 (5H, m), 8.15 (1H, d), 8.03-7.98 (3H, m), 7.86-7.64 (7H, m), 7.54-7.50 (6H, m), 7.39-7.31 (4H, m) |
| 1-51 | δ=9.60 (1H, d), 9.27 (2H, s), 8.55 (1H, d), 8.38-8.30 (9H, m), 7.99-7.89 (4H, m), 7.77-7.50 (17H, m), 7.35 (1H, t), 7.16 (1H, t) |
| 1-56 | δ=9.60 (1H, d), 9.27 (2H, s), 8.55 (1H, d), 8.38-8.30 (8H, m), 7.99-7.89 (5H, m), 7.77-7.50 (15H, m), 7.35 (1H, t), 7.25 (2H, d), 7.16 (1H, t) |
| 1-60 | δ=9.60 (1H, d), 9.27 (2H, s), 8.55 (1H, d), 8.45-8.30 (9H, m), 8.15 (1H, d), 7.94-7.93 (2H, m), 7.73-7.49 (15H, m) |
| 1-76 | δ=9.60 (1H, d), 9.27 (2H, s), 8.37-8.30 (3H, m), 8.15 (1H, d), 7.98 (1H, d), 7.88-7.52 (7H, m), 7.39-7.31 (2H, m) |
| 1-83 | δ=9.60 (1H, d), 9.27 (1H, s), 9.11 (1H, d), 8.46-8.30 (7H, m), 8.15 (1H, d), 7.98 (1H, d), 7.88-7.79 (3H, m), 7.70-7.64 (2H, m), 7.54-7.50 (8H, m), 7.39-7.31 (2H, m) |
| 1-87 | δ=9.60 (1H, d), 9.27 (1H, s), 9.11 (1H, d), 8.46-8.30 (8H, m), 8.15-8.12 (3H, m), 7.99-7.93 (2H, m), 7.70-7.49 (11H, m) |
| 1-91 | δ=9.60 (1H, d), 9.27 (1H, s), 9.11 (1H, d), 8.46-8.30 (8H, m), 8.19-8.13 (3H, m), 7.89 (1H, s), 7.70-7.50 (16H, m), 7.20 (1H, t) |
| 2-2 | δ=8.55(1H, d), 8.45(1H, d), 8.30(1H, d), 8.19(1H, d), 8.13(1H, d), 8.00~7.89(6H, m), 7.77(2H, m), 7.62~7.35(15H, m), 7.20~7.16(2H, m) |
| 2-3 | δ=8.55(1H, d), 8.45(1H, d), 8.30(1H, d), 8.19(1H, d), 8.13(1H, d), 8.00~7.89(6H, m), 7.77-7.75(4H, m), 7.62~7.35(13H, m), 7.25~7.16(6H, m) |
| 2-12 | δ=8.55(1H, d), 8.45(1H, d), 8.30(1H, d), 8.19~7.89(11H, m), 7.77(2H, m), 7.62~7.31(14H, m), 7.20~7.16(2H, m) |
| 3-3 | δ=8.55 (1H, d), 8.30 (1H, d), 8.21-8.13 (3H, m), 7.99-7.89 (4H, m), 7.77-7.35 (17H, m), 7.20-7.16 (2H, m) |
| 3-4 | δ=8.55 (1H, d), 8.30(1H, d), 8.19-8.13(2H, m), 7.99-7.89(8H, m), 7.77-7.75 (3H, m), 7.62-7.35 (11H, m), 7.20-7.16 (2H, m) |
| 3-7 | δ=8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 3-9 | δ=8.55 (1H, d), 8.30 (1H, d), 8.19-8.13 (2H, m), 8.03-7.77 (9H, m), 7.62-7.50 (9H, m), 7.36-7.35 (2H, m), 7.20-7.16 (2H, m) |
| 3-31 | δ=8.55 (1H, d), 8.30 (1H, d), 8.21-8.13 (4H, m), 7.99-7.89 (4H, m), 7.77-7.35 (20H, m), 7.20-7.16 (2H, m) |
| 3-32 | δ=8.55 (1H, d), 8.30 (1H, d), 8.21-8.13 (3H, m), 7.99-7.89 (8H, m), 7.77-7.35 (17H, m), 7.20-7.16 (2H, m) |
| 3-42 | δ=8.55 (1H, d), 8.30 (1H, d), 8.19 (1H, d), 8.13 (1H, d), 7.99-7.89 (12H, m), 7.77-7.75 (5H, m), 7.58 (1H, d), 7.50-7.35 (8H, m), 7.20-7.16 (2H, m) |

**[Table 4]**

| Compound | FD-MS | Compound | FD-MS |
|---|---|---|---|
| 1-3 | m/z=625.22 (C₄₅H₂₇N₃O=625.73) | 1-7 | m/z=641.19 (C₄₅H₂₇N₃O=641.79) |
| 1-11 | m/ z=700.26 (C₅₁H₃₂N₄=700.84) | 1-14 | m/z=651.27 (C₄₈H₃₃N₃=651.81) |
| 1-26 | m/z=715.23 (C₅₁H₂₉N₃O₂=715.81) | 1-51 | m/z=776.29 (C₅₇H₃₆N₄=776.94) |
| 1-56 | m/z=776.29 (C₅₇H₃₆N₄=776.94) | 1-60 | m/z=717.22 (C₅₁H₃₁N₃S=717.89) |
| 1-76 | m/z=635.28 (C₄₅H₁₇D₁₀N₃O=635.79) | 1-83 | m/z=625.22 (C₄₅H₂₇N₃O=625.73) |
| 1-87 | m/z=641.19 (C₄₅H₂₇N₃O=641.79) | 1-91 | m/z=700.26 (C₅₁H₃₂N₄=700.84) |
| 2-2 | m/z=666.21(C₄₈H₃₀N₂S=666.84) | 2-3 | m/z=742.24 (C₅₄H₃₄N₂S=742.94) |
| 2-12 | m/z=756.22 (C₅₄H₃₂N₂OS=756.92) | 3-3 | m/z=560.23 (C₄₂H₂₈N₂=560.70) |
| 3-4 | m/z=560.23 (C₄₂H₂₈N₂=560.70) | 3-7 | m/z=636.26 (C₄₈H₃₂N₂=636.80) |
| 3-9 | m/z=534.21 (C₄₀H₂₆N₂=534.66) | 3-31 | m/z=636.26 (C₄₈H₃₂N₂=636.80) |
| 3-32 | m/z=636.26 (C₄₈H₃₂N₂=636.80) | 3-42 | m/z=636.26 (C₄₈H₃₂N₂=636.80) |

### <Experimental Example 1>

### - Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, a compound of Chemical Formula 1 described in the following Table 5 was deposited to 400 Å as a host, and a green phosphorescent dopant [Ir(ppy)₃] was doped by 7% of the deposited thickness of the light emitting layer and deposited. After that, BCP (bathocuproine) was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color (EL color) and lifetime of the organic light emitting devices manufactured according to the present disclosure are as shown in the following Table 5.

**[Table 5]**

| | Light Emitting Layer Compound | Driving Voltage (V) | Efficie ncy (cd/A) | Color (EL color) | Lifetime (T₉₀) |
|---|---|---|---|---|---|
| Example 1 | 1-3 | 4.66 | 71.1 | Green | 352 |
| Example 2 | 1-7 | 4.32 | 71.5 | Green | 289 |
| Example 3 | 1-11 | 4.33 | 74.2 | Green | 405 |
| Example 4 | 1-14 | 4.36 | 78.9 | Green | 256 |
| Example 5 | 1-26 | 4.66 | 71.2 | Green | 360 |
| Example 6 | 1-51 | 4.69 | 77.2 | Green | 221 |
| Example 7 | 1-56 | 4.41 | 75.8 | Green | 225 |
| Example 8 | 1-60 | 4.41 | 75.8 | Green | 249 |
| Example 9 | 1-76 | 4.67 | 71.2 | Green | 356 |
| Example 10 | 1-83 | 4.66 | 71.1 | Green | 347 |
| Example 11 | 1-87 | 4.67 | 71.2 | Green | 277 |
| Example 12 | 1-91 | 4.67 | 71.2 | Green | 390 |
| Comparative Example 1 | Ref. 1 | 5.42 | 45.7 | Green | 47 |
| Comparative Example 2 | Ref. 2 | 5.37 | 53.5 | Green | 82 |
| Comparative Example 3 | Ref. 3 | 5.45 | 59.8 | Green | 78 |
| Comparative Example 4 | Ref. 4 | 5.82 | 50.8 | Green | 68 |
| Comparative Example 5 | Ref. 5 | 5.47 | 41.2 | Green | 57 |
| Comparative Example 6 | Ref. 6 | 5.03 | 48.7 | Green | 69 |
| Comparative Example 7 | Ref. 7 | 5.66 | 45.8 | Green | 48 |
| Comparative Example 8 | Ref. 8 | 5.17 | 63.2 | Green | 154 |
| Comparative Example 9 | Ref. 9 | 5.35 | 61.2 | Green | 105 |

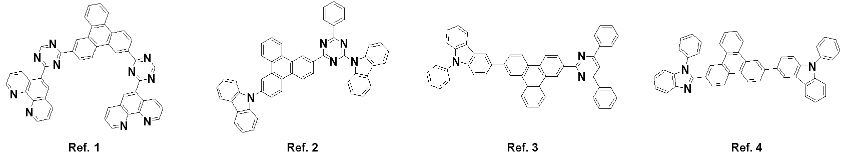

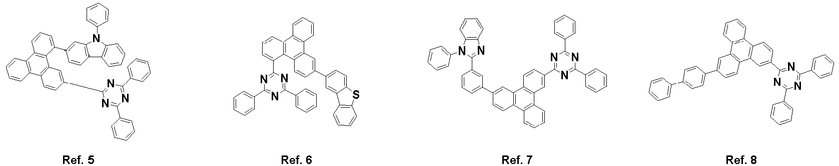

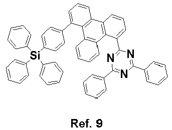

As seen from the results of Table 5, the organic electroluminescent device using the light emitting layer material of the organic electroluminescent device of the present disclosure had lower driving voltage, enhanced light emission efficiency, and significantly improved lifetime compared to Comparative Examples 1 to 9.

Specifically, in the following Table 6, the LUMO orbital of Compound Ref. 1 is widely delocalized across phenanthroline-triazine-triphenylene-triazine-phenanthroline, and the LUMO level is -2.38. It may be identified that such a result inhibits a charge balance by creating excessive electron mobility, and efficiency is measured to be low by the LUMO level lower than the dopant inhibiting energy transfer. In addition, it may be identified that having three substituents based on triazine (Compound 1-11) has superior stability and enhanced lifetime compared to having two substituents (Ref. 1).

**[Table 6]**

| Compou nd | Structural Formula | HOMO Orbital | LUMO Orbital |
|---|---|---|---|
| Ref. 1 | 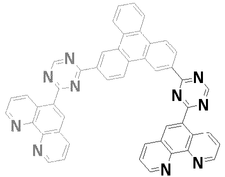 | 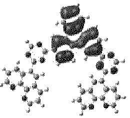 -6.24 | 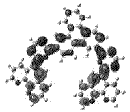 -2.38 |
| 1-11 | 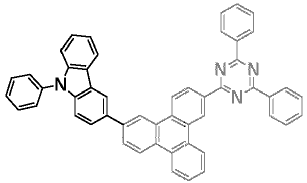 | 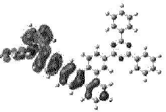 -5.26 | 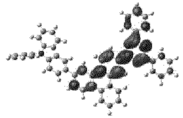 -1.88 |

In addition, in the following Table 7, the HOMO orbital of Compound Ref. 2 is localized to carbazole, whereas the HOMO orbital of Compound 1-11 is widely delocalized to carbazole and triphenylene. It may be identified that this enhances efficiency and lifetime of the device by enhancing hole stability and mobility.

**[Table 7]**

| Compou nd | Structural Formula | HOMO Orbital | LUMO Orbital |
|---|---|---|---|
| Ref. 2 | 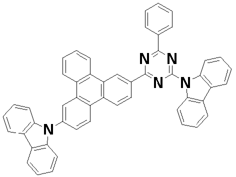 | 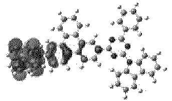 -5.37 | 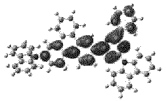 -2.08 |

Compounds Ref. 2, 3 and 4 are different from Chemical Formula 1 of the present disclosure in the position of substitution. In the structures of Compounds Ref. 2, 3 and 4, the HOMO core, the linker and the LUMO core are linearly connected facilitating charge transfer in the molecules and lowering a band gap. It may be identified that the low band gap inhibits hole and electron injections from the auxiliary layer to the host, which reduces efficiency and lifetime of the device.

Likewise, Compounds Ref. 5, 6 and 9 are also different from Chemical Formula 1 of the present disclosure in the position of substitution. Materials having a molecular steric hindrance as in the structures of Compounds Ref. 5, 6 and 9 may have problems in the molecular stability causing a problem of reducing a device lifetime.

In the following Table 8, Compounds Ref. 7 and 8 have the same position of substitution as Chemical Formula 1 of the present disclosure, but have different substituents. Specifically, when a monocyclic heteroaryl group directly substitutes as Z1 (or Z2) on the triphenylene in the present disclosure, the other substituent Z2 (or Z1) is a silyl group, a fluorenyl group or a tricyclic or higher heteroaryl group, whereas Compounds Ref. 7 and 8 are substituted with a dicyclic heteroaryl group and an aryl group (biphenyl group).

**[Table 8]**

| Compou nd | Structural Formula | HOMO Orbital | LUMO Orbital |
|---|---|---|---|
| Ref. 7 | 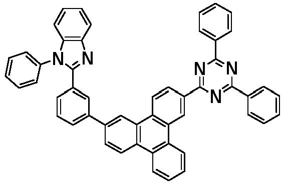 | 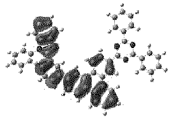 -5.68 | 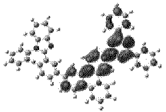 -1.88 |
| Ref. 8 | 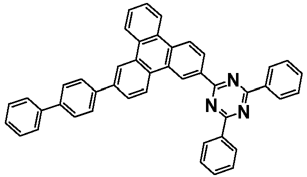 | 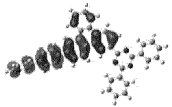 -5.63 | 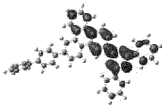 -1.94 |

In Compound Ref. 7, the HOMO orbital is widely delocalized to imidazole and triphenylene. The HOMO core needs to stabilize holes, however, imidazole is not suited for stabilizing holes compared to carbazole of Compound 1-11, and reduces a lifetime of the device.

In addition, the HOMO orbital of Compound Ref. 8 is delocalized to phenyl and triphenylene. The HOMO core of Compound Ref. 8 is capable of stabilizing holes compared to imidazole, however, it may be identified that, compared to carbazole, low hole stability and short lifetime are obtained.

In an organic electroluminescent device, a result of a host in a light emitting layer may change depending on the degree of balance between holes and electrons transferred to the light emitting layer.

### <Experimental Example 2>

### - Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type of a compound of Chemical Formula 1 and one type of a compound of Chemical Formula 2 or Chemical Formula 3 were pre-mixed as described in the following Table 9 and deposited to 400 Å in one source of supply as a host, and a green phosphorescent dopant [Ir(ppy)₃] was doped by an amount of 7% of the deposited thickness of the light emitting layer and deposited. After that, BCP (bathocuproine) was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color (EL color) and lifetime of the organic light emitting devices manufactured according to the present disclosure are as shown in the following Table 9.

**[Table 9]**

| | Light Emitting Layer Compound | Ratio | Driving Voltage (V) | Efficienc y (cd/A) | Color (EL color) | Lifetime (T₉₀) |
|---|---|---|---|---|---|---|
| Example 13 | 1-11:2-2 | 1:8 | 4.73 | 54.2 | Green | 338 |
| Example 14 | 1-11:2-2 | 1:5 | 4.71 | 57.2 | Green | 444 |
| Example 15 | 1-11:2-2 | 1:2 | 4.33 | 75.2 | Green | 679 |
| Example 16 | 1-11:2-2 | 1:1 | 4.48 | 70.2 | Green | 546 |
| Example 17 | 1-11:2-2 | 2:1 | 4.69 | 69.2 | Green | 525 |
| Example 18 | 1-11:2-2 | 5:1 | 4.32 | 68.3 | Green | 433 |
| Example 19 | 1-11:2-2 | 8:1 | 4.21 | 67.0 | Green | 325 |
| Example 20 | 1-26:2-3 | 1:2 | 4.35 | 79.2 | Green | 599 |
| Example 21 | 1-26:2-3 | 1:1 | 4.41 | 75.8 | Green | 591 |
| Example 22 | 1-26:2-3 | 2:1 | 4.67 | 71.2 | Green | 540 |
| Example 23 | 1-7:2-12 | 1:2 | 4.38 | 76.4 | Green | 539 |
| Example 24 | 1-7:2-12 | 1:1 | 4.45 | 72.8 | Green | 522 |
| Example 25 | 1-7:2-12 | 2:1 | 4.66 | 71.1 | Green | 500 |
| Example 26 | 1-91:3-4 | 1:2 | 4.33 | 75.2 | Green | 620 |
| Example 27 | 1-91:3-4 | 1:1 | 4.48 | 70.2 | Green | 582 |
| Example 28 | 1-91:3-4 | 2:1 | 4.69 | 69.2 | Green | 543 |
| Example 29 | 1-3:3-7 | 1:2 | 4.33 | 75.2 | Green | 569 |
| Example 30 | 1-3:3-7 | 1:1 | 4.48 | 70.2 | Green | 543 |
| Example 31 | 1-3:3-7 | 2:1 | 4.69 | 69.2 | Green | 518 |
| Example 32 | 1-76:3-31 | 1:2 | 4.31 | 79.2 | Green | 586 |
| Example 33 | 1-76:3-31 | 1:1 | 4.42 | 75.7 | Green | 558 |
| Example 34 | 1-76:3-31 | 2:1 | 4.66 | 71.1 | Green | 536 |
| Example 35 | 1-83:3-32 | 1:2 | 4.33 | 74.2 | Green | 562 |
| Example 36 | 1-83:3-32 | 1:1 | 4.42 | 72.2 | Green | 553 |
| Example 37 | 1-83:3-32 | 2:1 | 4.66 | 71.2 | Green | 528 |

From the results of Table 9, it may be identified that effects of more superior efficiency and lifetime are obtained when including the compound of Chemical Formula 1 (N-type) and the compound of Chemical Formula 2 or 3 (P-type) at the same time. Such results may lead to a forecast that an exciplex phenomenon occurs when including the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime. In the disclosure of the present application, the compound of Chemical Formula 2 or 3 performed the donor role, and the compound of Chemical Formula 1 performed the acceptor role, and when used together as the light emitting layer host, excellent device properties were obtained.

## Claims

1. A compound of the following Chemical Formula 1: 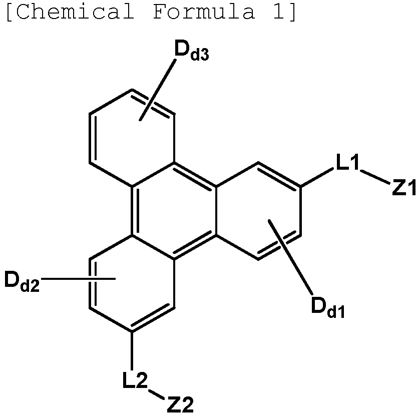 wherein, in Chemical Formula 1,
d1 and d2 are each an integer of 0 to 3,
d3 is an integer of 0 to 4,
L1 and L2 are the same as or different from each other, and each independently a direct bond; or a substituted or
unsubstituted C6 to C60 arylene group,
Z1 and Z2 are the same as or different from each other, and each independently a substituted or unsubstituted silyl group; a substituted or unsubstituted fluorenyl group; or a heteroaryl group represented by any one of the following Chemical Formulae 1-1 to 1-4, and
at least one of Z1 and Z2 is a heteroaryl group represented by any one of the following Chemical Formulae 1-1 to 1-3, 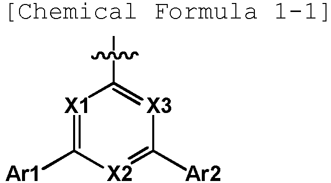 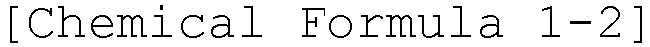 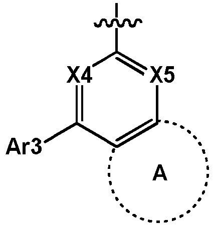 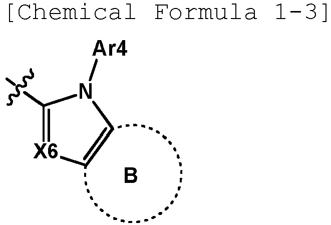 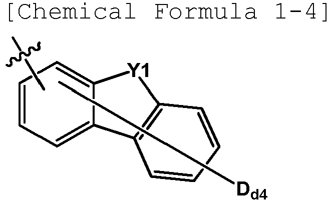
in Chemical Formulae 1-1 to 1-4,
d4 is an integer of 0 to 7,
X1 to X6 are each N or CR,
at least one of X1 to X3 is N,
at least one of X4 and X5 is N,
Y1 is O; S or NR',
R, R', Ar1 to Ar4 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
A and B are each a substituted or unsubstituted monocyclic or polycyclic aryl ring; or a monocyclic or polycyclic heteroring substituted or unsubstituted and including O or S, and
when L1 is a direct bond and Z1 is the heteroaryl group represented by Chemical Formula 1-1, Z2 is a substituted or unsubstituted silyl group; a substituted or unsubstituted fluorenyl group; or the tricyclic or higher heteroaryl group represented by any one of Chemical Formulae 1-2 to 1-4.

2. The compound of Claim 1, wherein Ar1 and Ar2 of Chemical Formula 1-1 are each independently a substituted or unsubstituted C6 to C30 aryl group; or a substituted or unsubstituted C2 to C30 heteroaryl group.

3. The compound of Claim 1, wherein A of Chemical Formula 1-2 is a substituted or unsubstituted monocyclic aryl ring; or a polycyclic heteroring substituted or unsubstituted and including O or S, and B of Chemical Formula 1-3 is a substituted or unsubstituted monocyclic aryl ring.

4. The compound of Claim 1, wherein at least one of Z1 and Z2 is the heteroaryl group represented by Chemical Formula 1-1.

5. The compound of Claim 1, wherein Chemical Formula 1 is represented by any one of the following compounds:

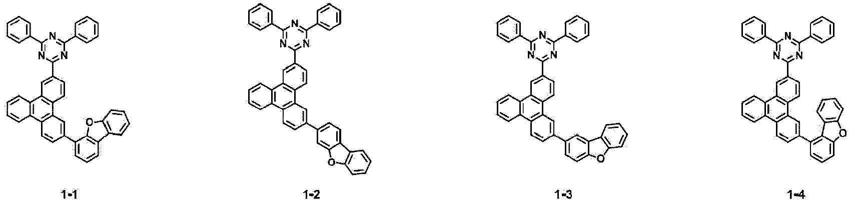

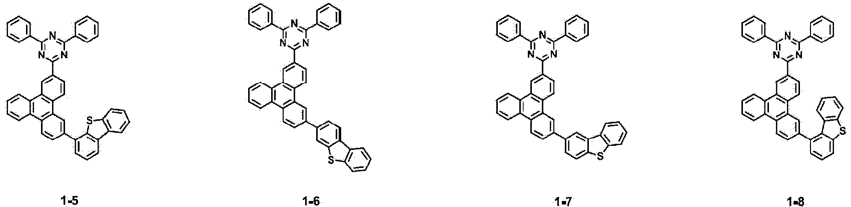

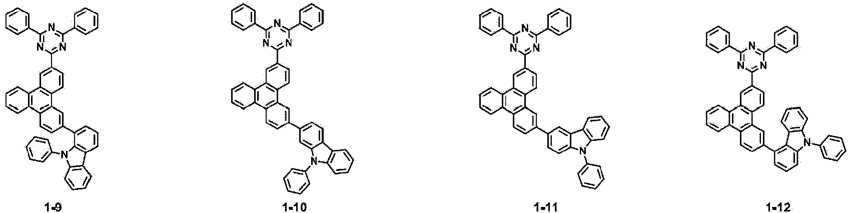

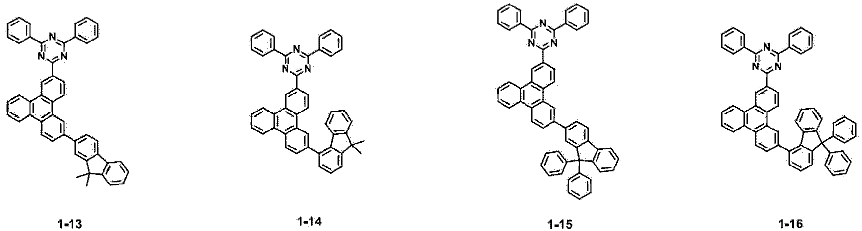

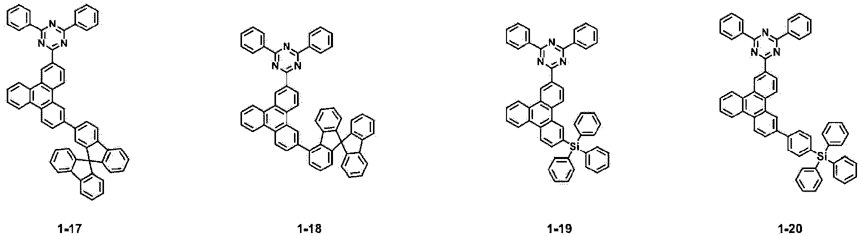

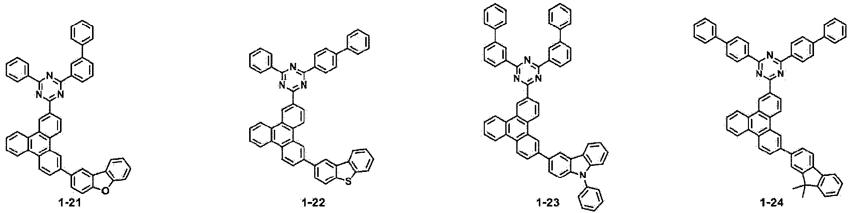

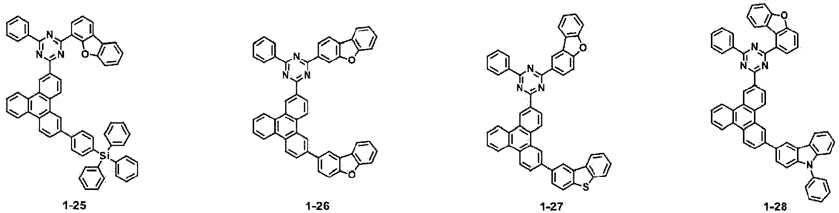

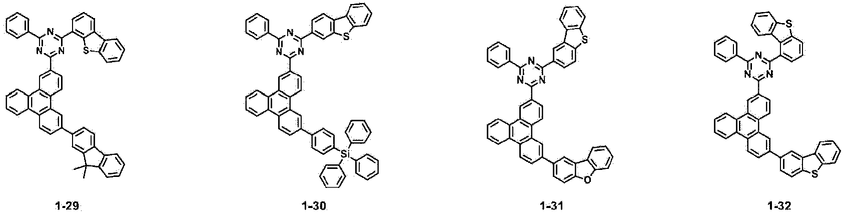

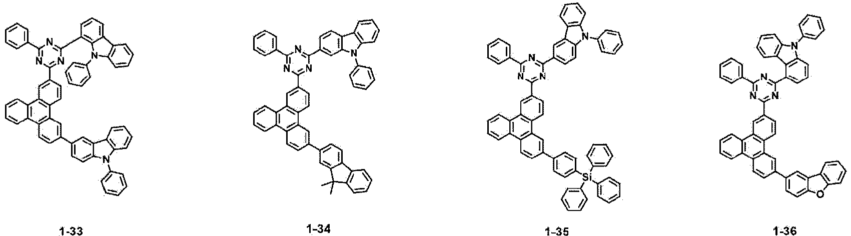

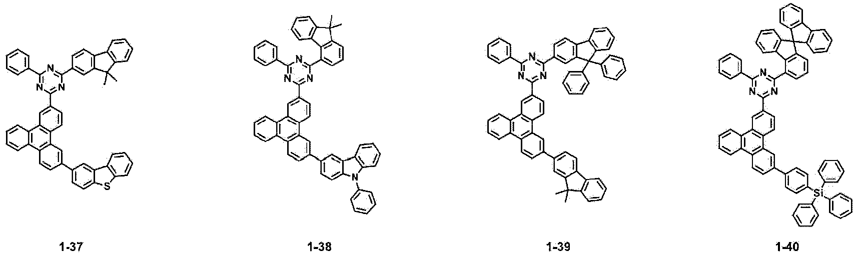

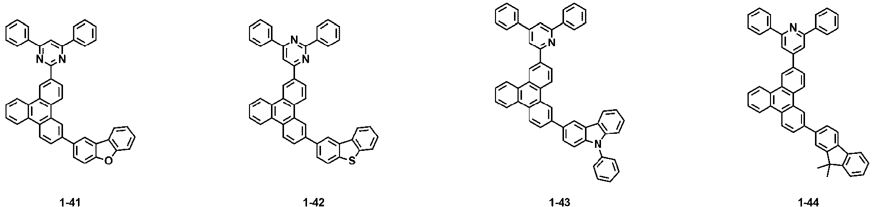

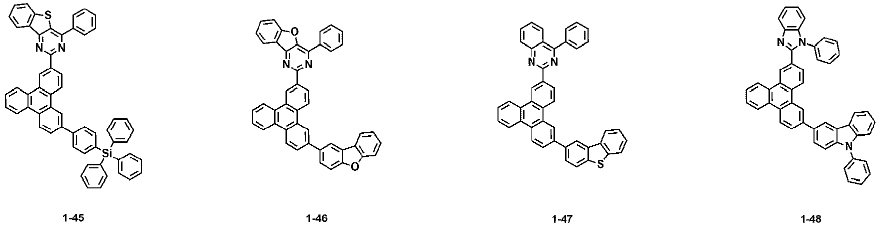

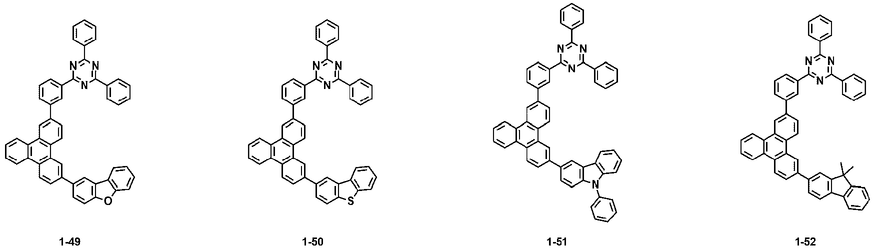

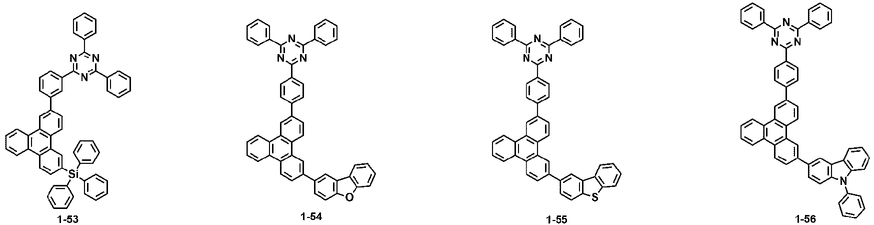

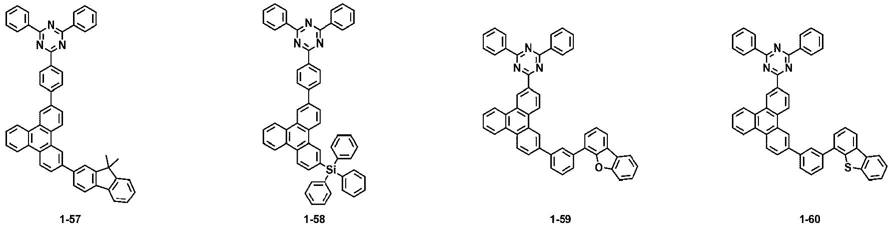

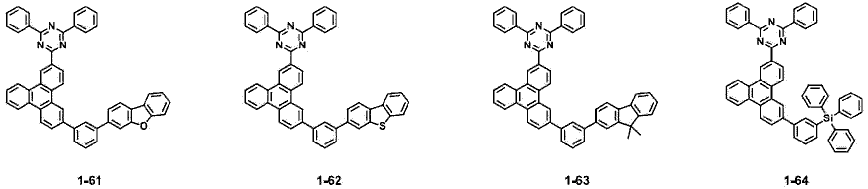

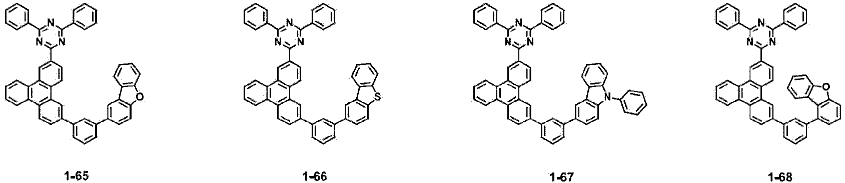

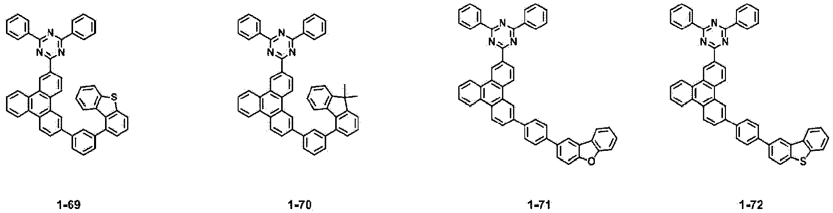

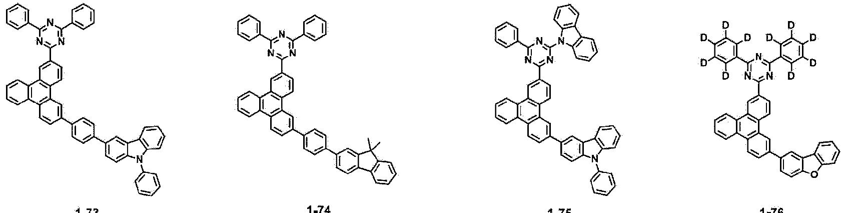

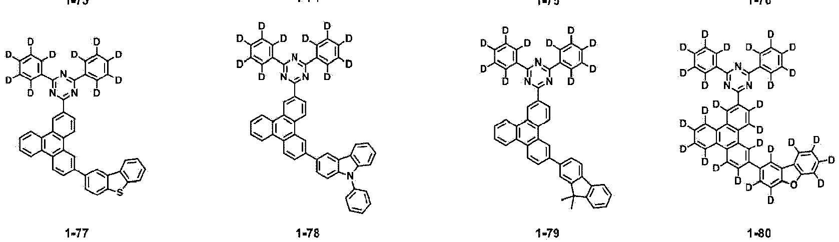

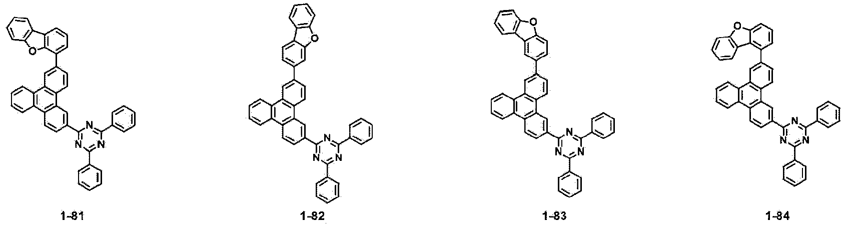

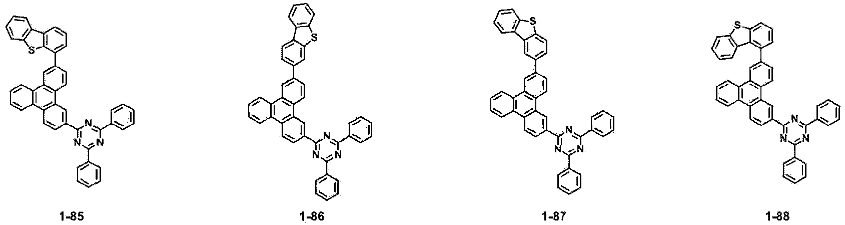

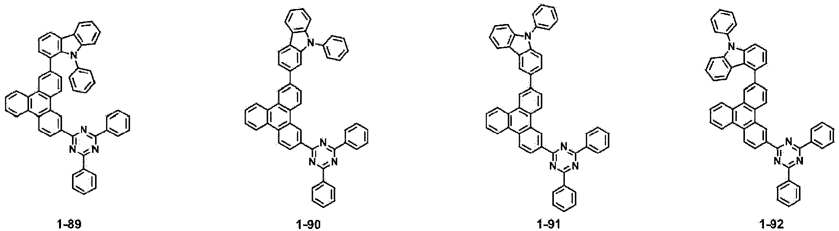

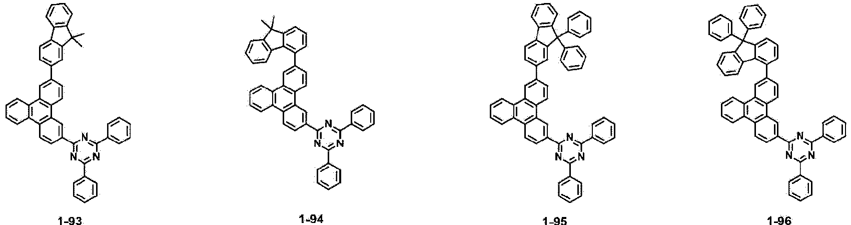

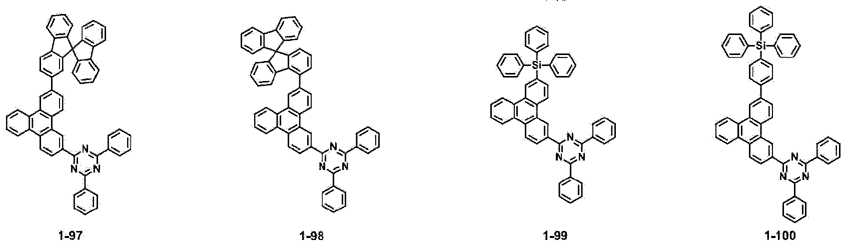

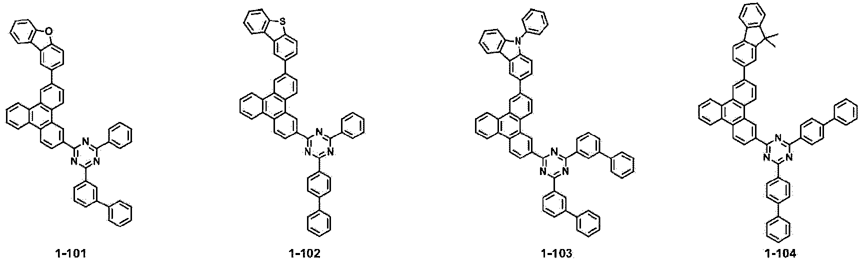

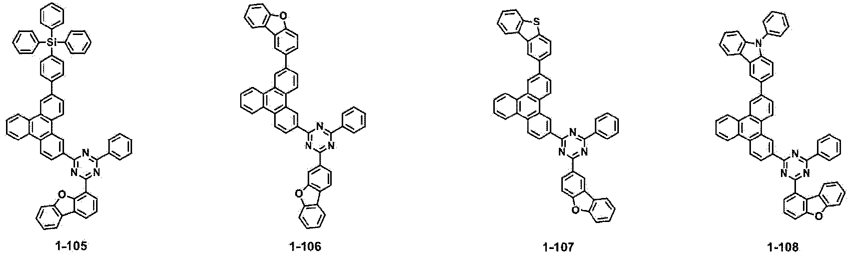

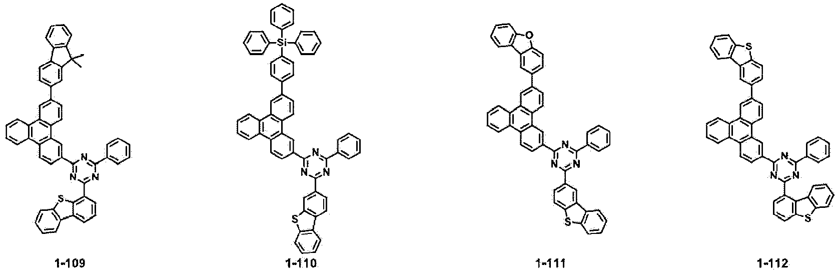

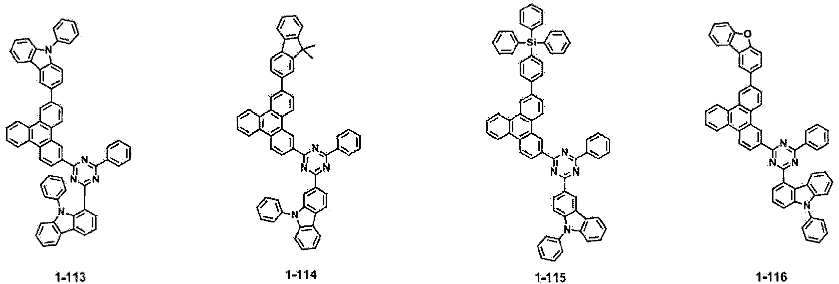

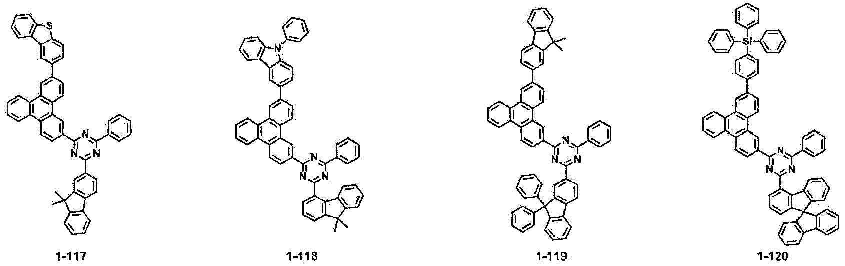

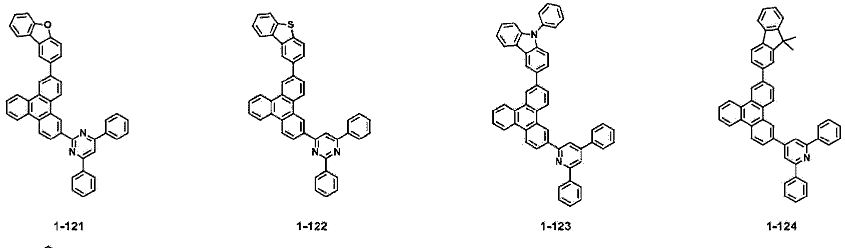

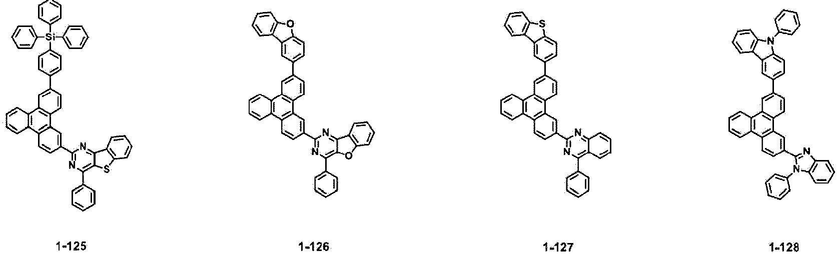

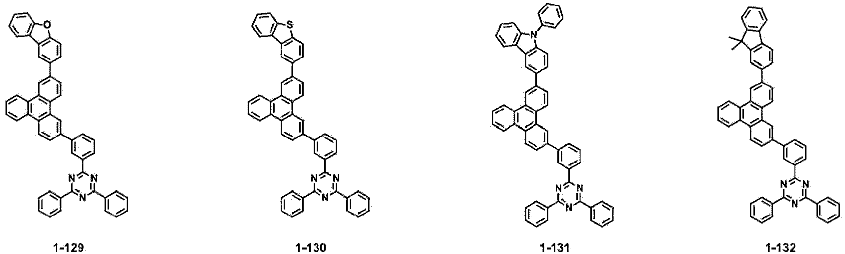

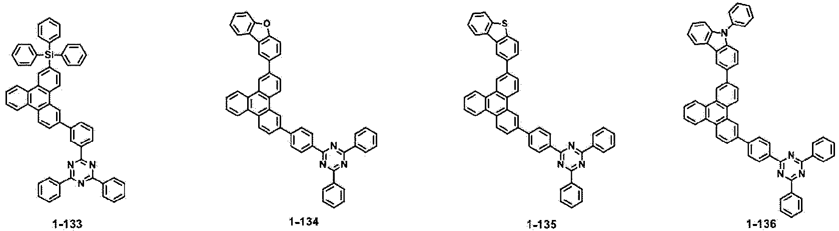

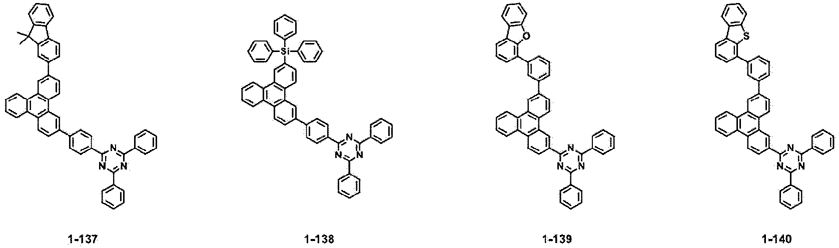

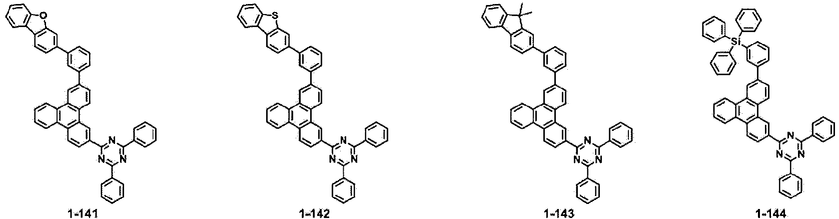

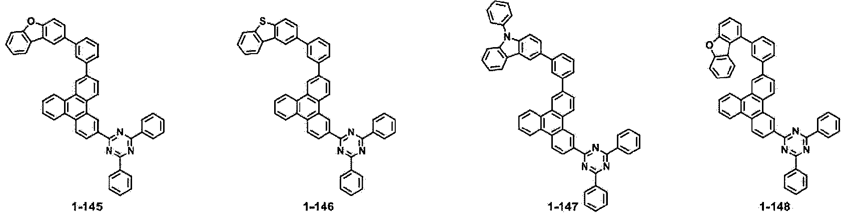

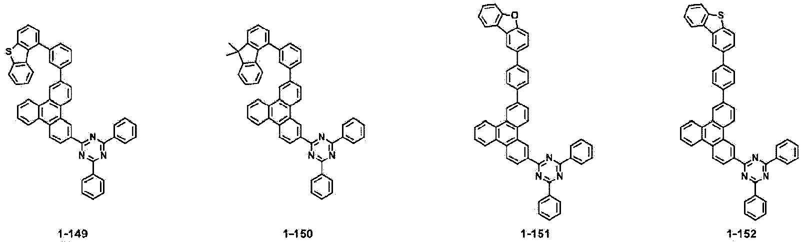

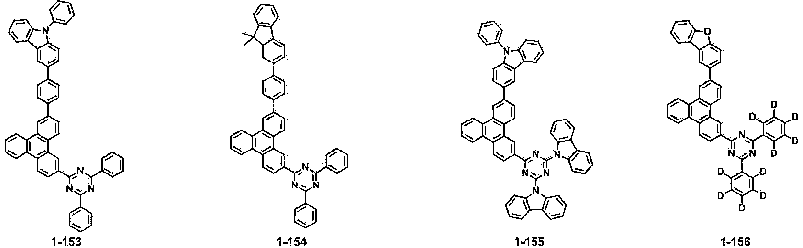

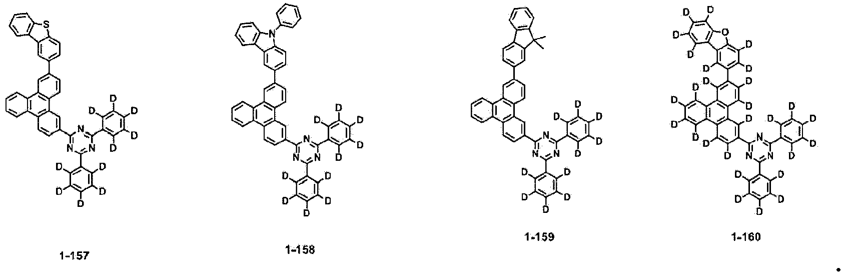

6. An organic light emitting device comprising:
a first electrode;
a second electrode; and
an organic material layer provided between the first electrode and the second electrode,
wherein the organic material layer includes one or more types of the compound of any one of Claims 1 to 5.

7. The organic light emitting device of Claim 6, wherein the organic material layer includes a light emitting layer, and the light emitting layer includes the compound.

8. The organic light emitting device of Claim 6, wherein the organic material layer includes a light emitting layer, the light emitting layer includes a host, and the host includes the compound.

9. The organic light emitting device of Claim 7, wherein the light emitting layer further includes a compound of the following Chemical Formula 2 or 3: 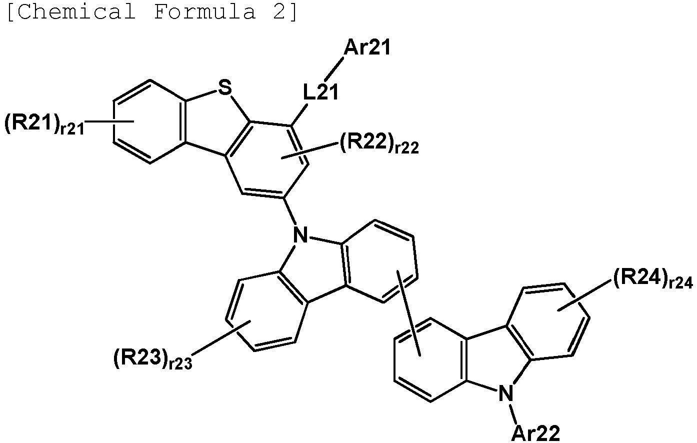 in Chemical Formula 2,
R21 to R24 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
L21 is a direct bond; a substituted or unsubstituted C6 to C60 arylene group; or a substituted or unsubstituted C2 to C60 heteroarylene group,
Ar21 and Ar22 are each independently a substituted or unsubstituted C6 to C60 aryl group; or a substituted or unsubstituted C2 to C60 heteroaryl group,
r21 is an integer of 1 to **4,** and when 2 or greater, R21s are the same as or different from each other,
r22 is 1 or **2,** and when **2,** R22s are the same as or different from each other,
r23 is an integer of 1 to **4,** and when 2 or greater, R23s are the same as or different from each other, and
r24 is an integer of 1 to **4,** and when 2 or greater, R24s are the same as or different from each other, 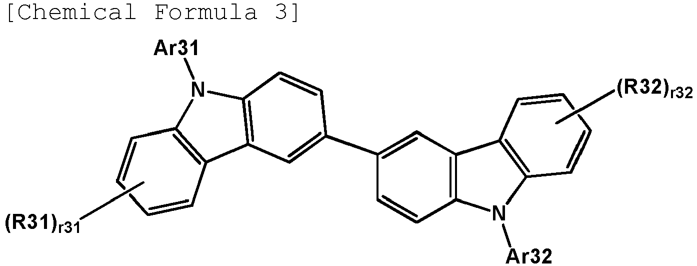
in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a C2 to C60 heteroaryl group substituted or unsubstituted and including O or N,
r31 is an integer of 1 to 4, and when 2 or greater, R31s are the same as or different from each other, and
r32 is an integer of 1 to 4, and when 2 or greater, R32s are the same as or different from each other.

10. The organic light emitting device of Claim 9, wherein Chemical Formula 2 is represented by any one of the following compounds:

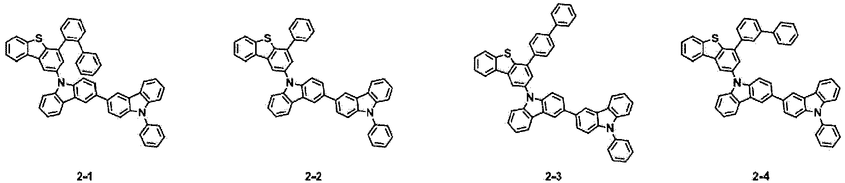

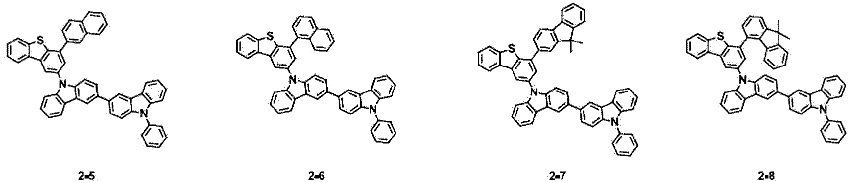

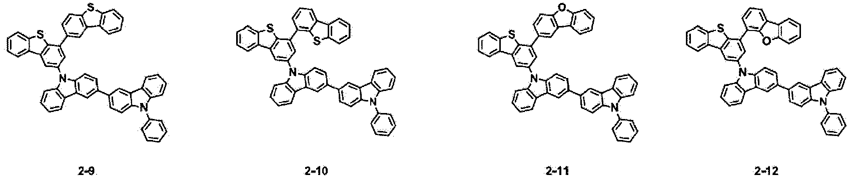

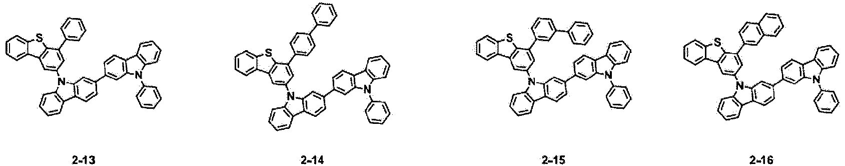

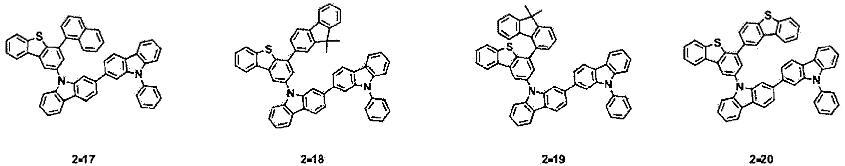

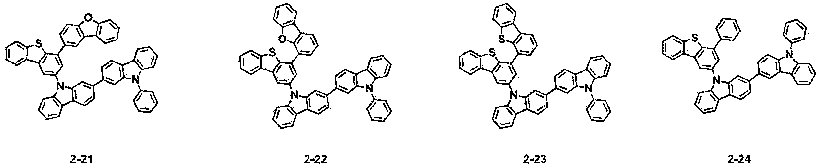

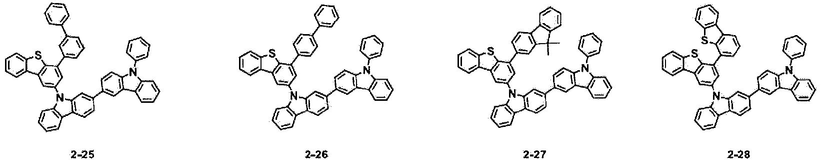

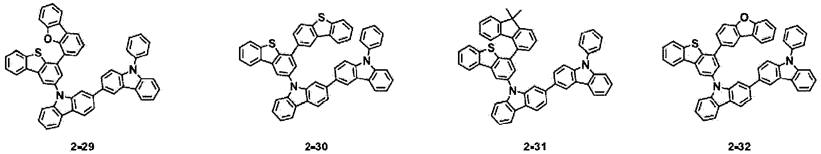

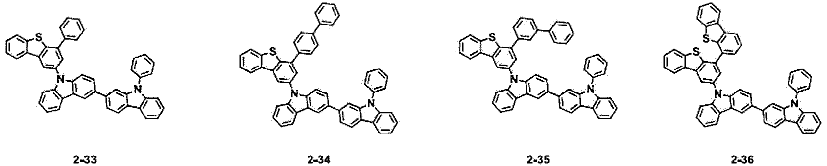

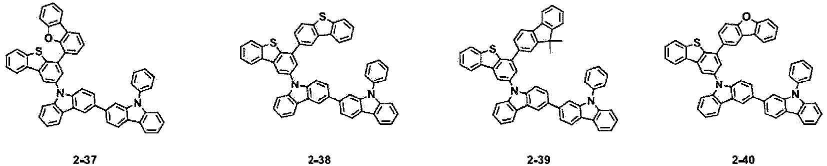

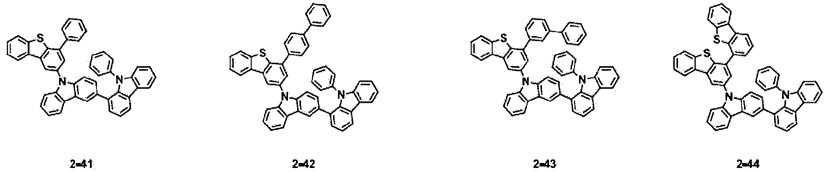

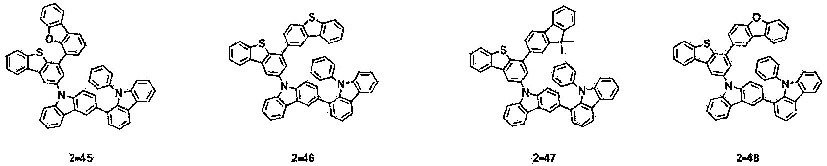

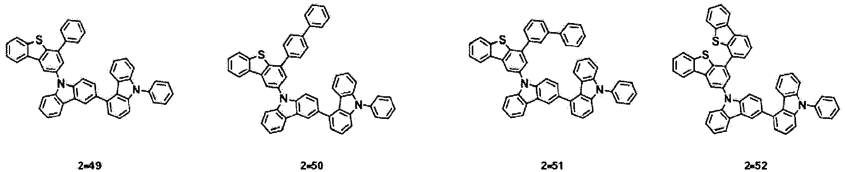

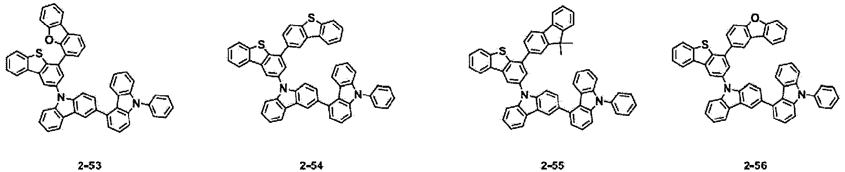

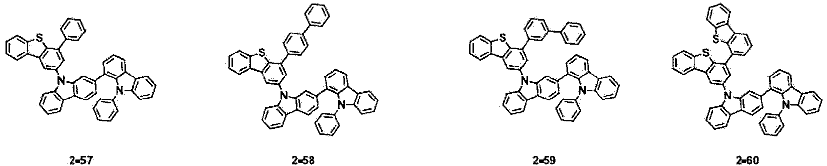

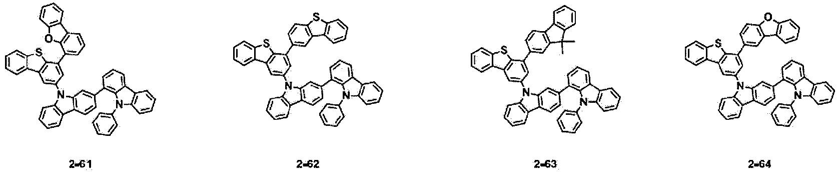

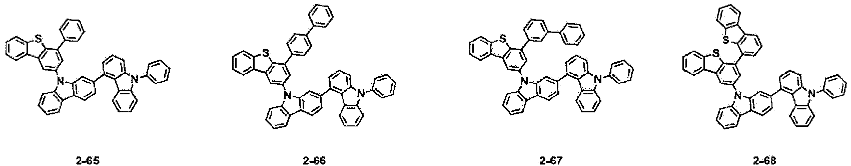

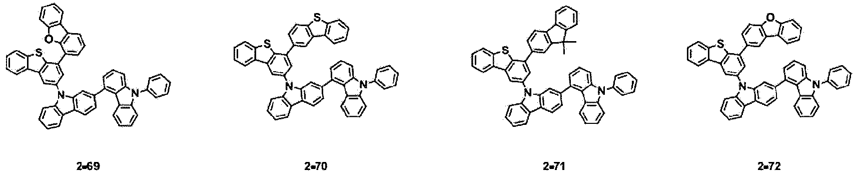

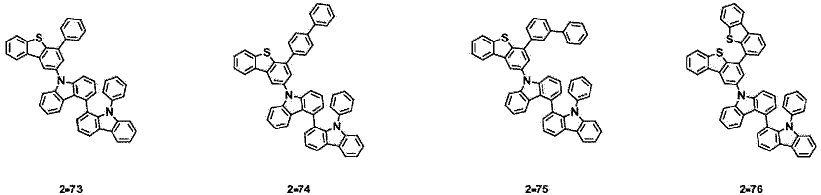

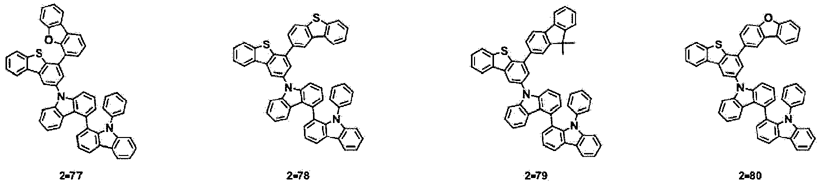

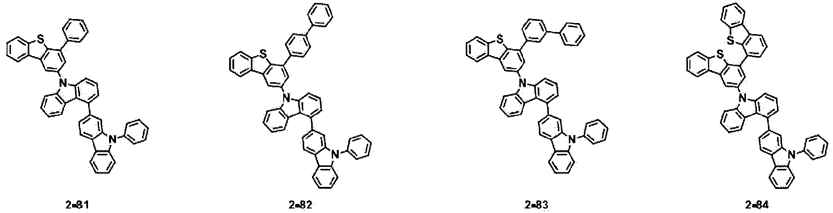

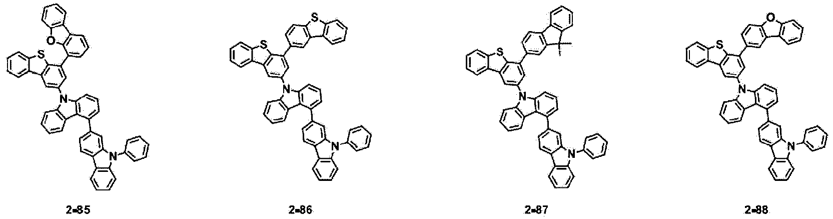

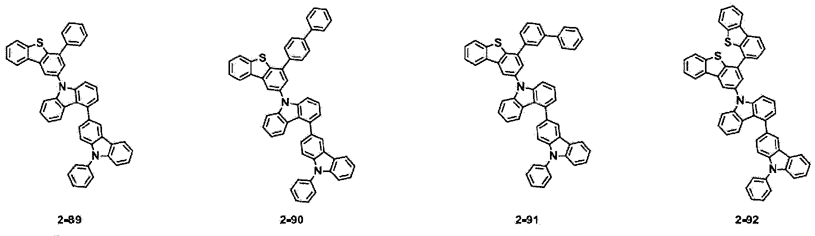

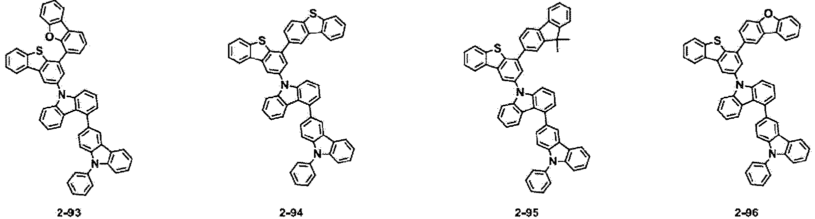

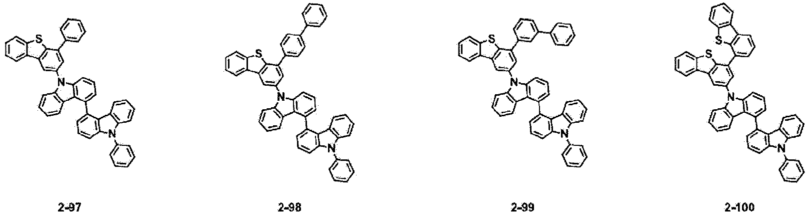

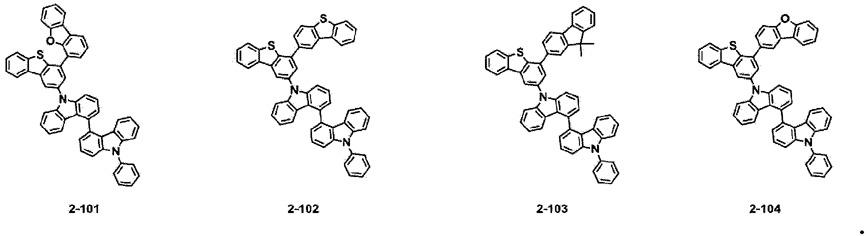

11. The organic light emitting device of Claim 9, wherein Chemical Formula 3 is represented by any one of the following compounds:

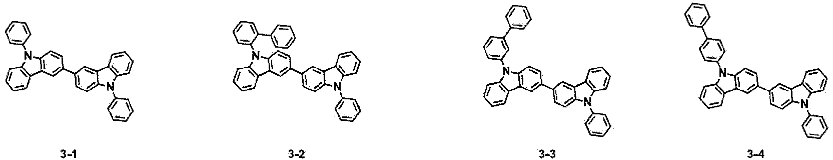

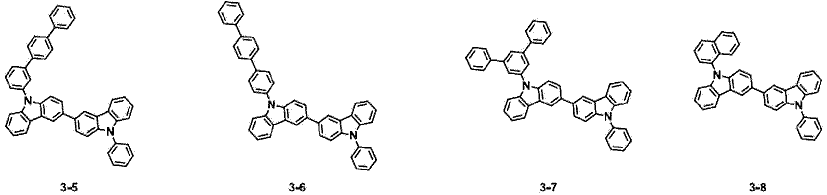

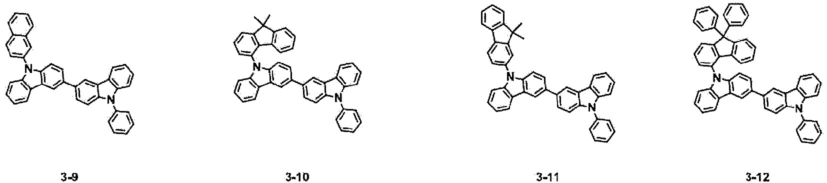

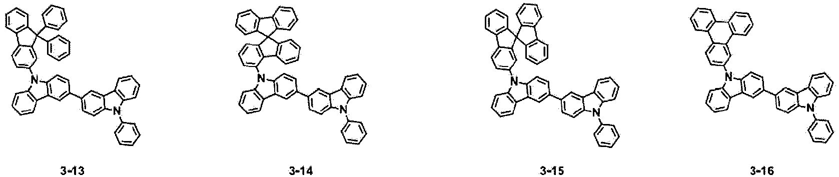

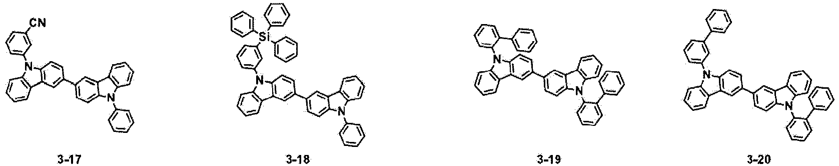

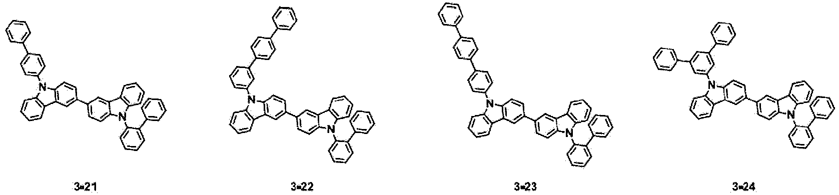

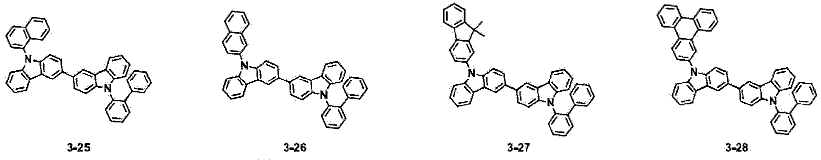

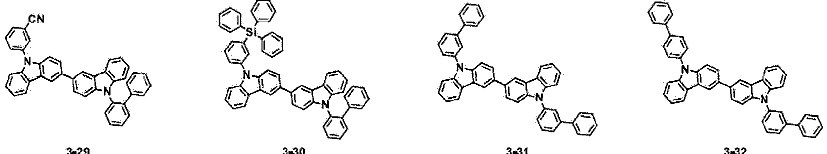

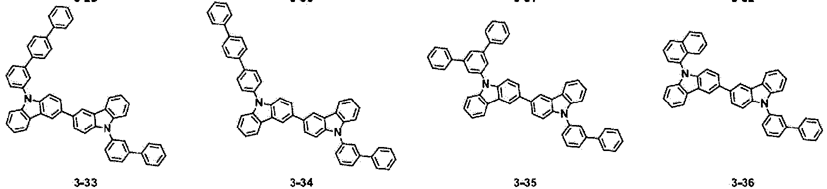

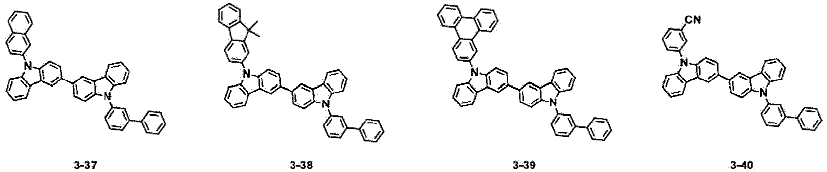

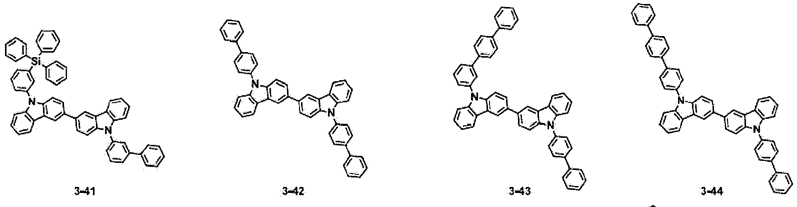

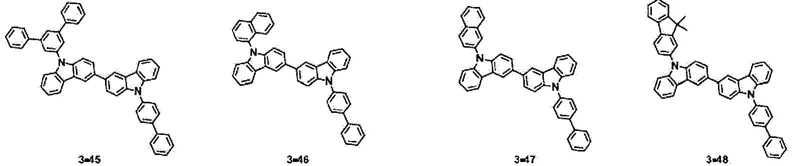

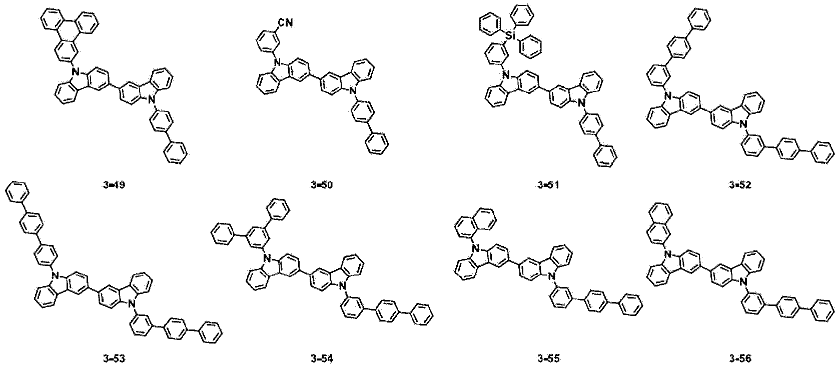

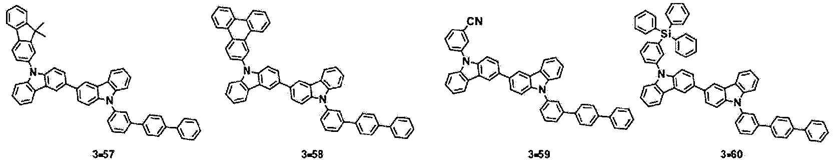

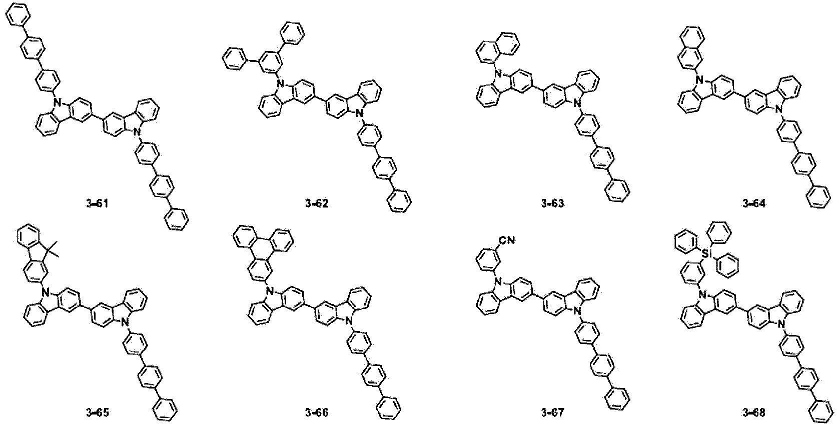

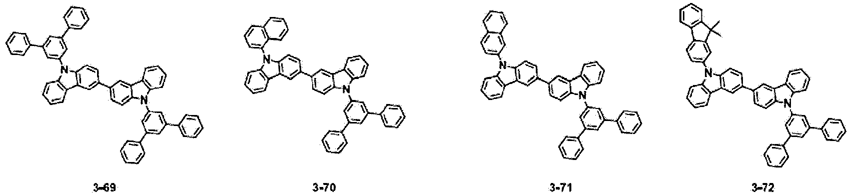

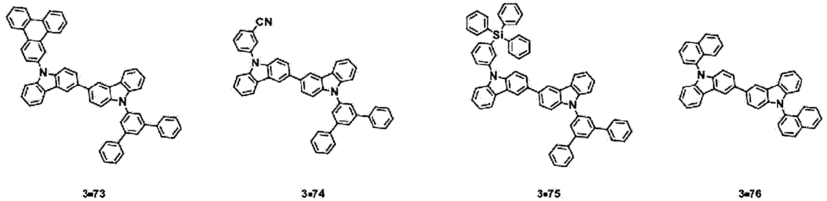

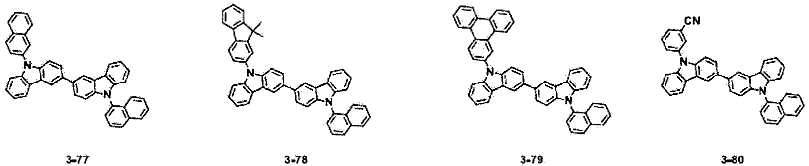

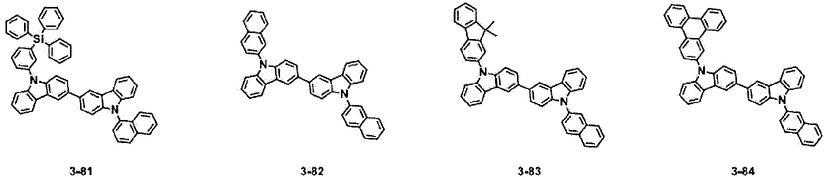

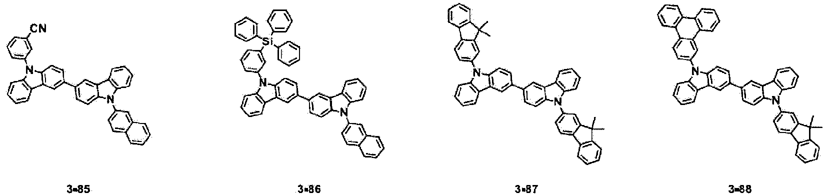

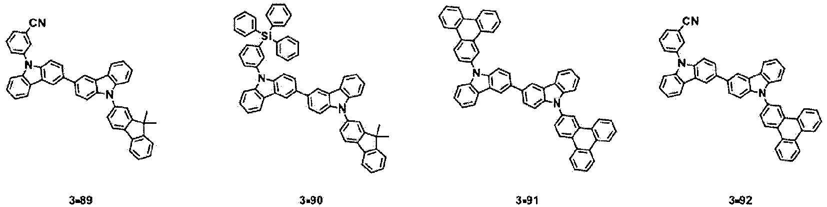

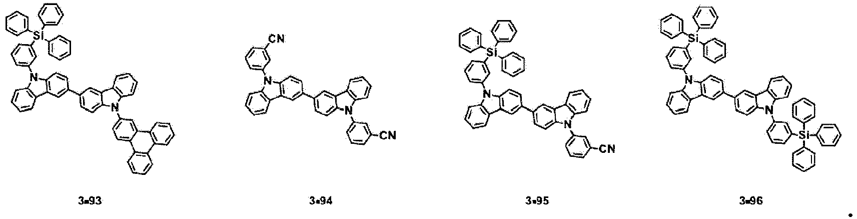

12. A composition for forming an organic material layer, the composition comprising:
a compound of the following Chemical Formula 1; and
a compound of the following Chemical Formula 2 or 3: 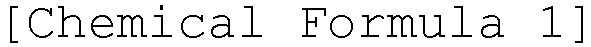 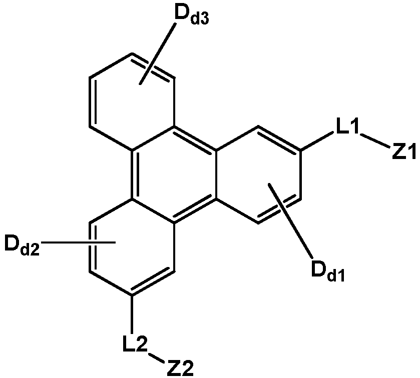
wherein, in Chemical Formula 1,
d1 and d2 are each an integer of 0 to 3,
d3 is an integer of 0 to 4,
L1 and L2 are the same as or different from each other, and each independently a direct bond; or a substituted or unsubstituted C6 to C60 arylene group,
Z1 and Z2 are the same as or different from each other, and each independently a substituted or unsubstituted silyl group; a substituted or unsubstituted fluorenyl group; or a heteroaryl group represented by any one of the following Chemical Formulae 1-1 to 1-4, and
at least one of Z1 and Z2 is a heteroaryl group represented by any one of the following Chemical Formulae 1-1 to 1-3, 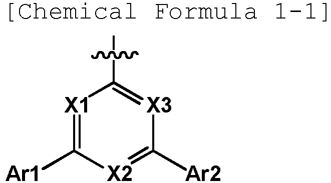 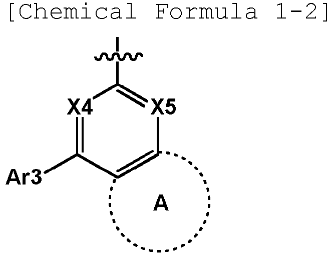 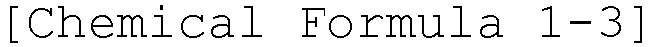 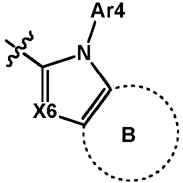 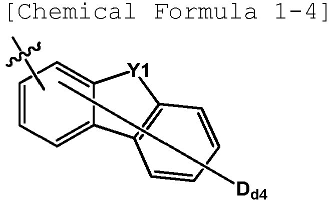
in Chemical Formulae 1-1 to 1-4,
d4 is an integer of 0 to 7,
X1 to X6 are each N or CR,
at least one of X1 to X3 is N,
at least one of X4 and X5 is N,
Y1 is O; S or NR',
R, R', Ar1 to Ar4 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
A and B are each a substituted or unsubstituted monocyclic or polycyclic aryl ring; or a monocyclic or polycyclic heteroring substituted or unsubstituted and including O or S, and
when L1 is a direct bond and Z1 is the heteroaryl group represented by Chemical Formula 1-1, Z2 is a substituted or unsubstituted silyl group; a substituted or unsubstituted fluorenyl group; or the tricyclic or higher heteroaryl group represented by any one of Chemical Formulae 1-2 to 1-4, 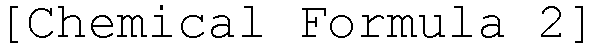 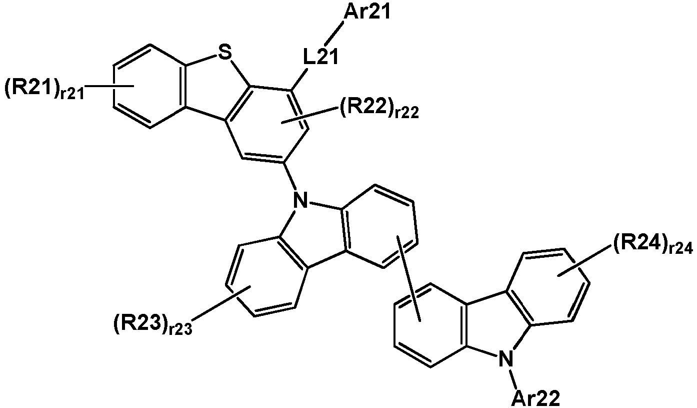
in Chemical Formula 2,
R21 to R24 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
L21 is a direct bond; a substituted or unsubstituted C6 to C60 arylene group; or a substituted or unsubstituted C2 to C60 heteroarylene group,
Ar21 and Ar22 are each independently a substituted or unsubstituted C6 to C60 aryl group; or a substituted or unsubstituted C2 to C60 heteroaryl group,
r21 is an integer of 1 to 4, and when 2 or greater, R21s are the same as or different from each other,
r22 is 1 or 2, and when 2, R22s are the same as or different from each other,
r23 is an integer of 1 to 4, and when 2 or greater, R23s are the same as or different from each other, and
r24 is an integer of 1 to 4, and when 2 or greater, R24s are the same as or different from each other, 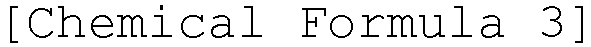 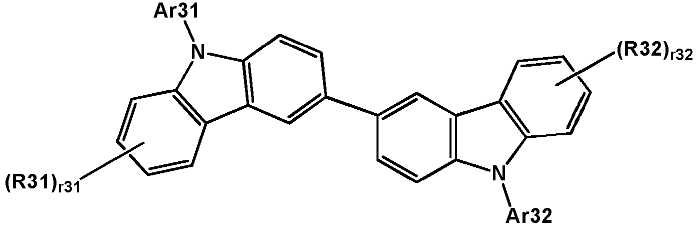
in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a C2 to C60 heteroaryl group substituted or unsubstituted and including O or N,
r31 is an integer of 1 to 4, and when 2 or greater, R31s are the same as or different from each other, and
r32 is an integer of 1 to 4, and when 2 or greater, R32s are the same as or different from each other.

13. The composition for forming an organic material layer of Claim 12, wherein the compound of Chemical Formula 1 and the compound of Chemical Formula 2 or 3 have a weight ratio of 1:10 to 10:1.

## Patentansprüche

1. Verbindung der folgenden chemischen Formel 1: 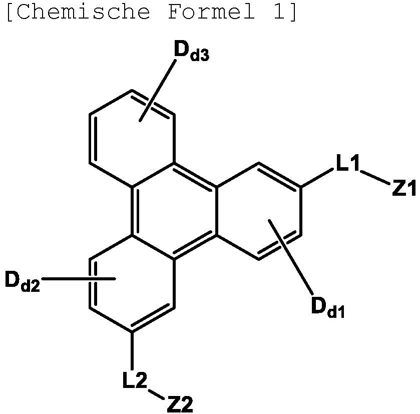 wobei in der chemischen Formel 1
d1 und d2 jeweils eine ganze Zahl von 0 bis 3 sind,
d3 eine ganze Zahl von 0 bis 4 ist,
L1 und L2 gleich oder verschieden voneinander sind und jeweils unabhängig eine direkte Bindung oder eine substituierte oder unsubstituierte
C6 - bis C60 -Arylengruppe sind,
Z1 und Z2 gleich oder verschieden voneinander sind und jeweils unabhängig eine substituierte oder unsubstituierte Silylgruppe, eine substituierte oder unsubstituierte Fluorenylgruppe oder eine Heteroarylgruppe sind, die durch eine der folgenden chemischen Formeln 1-1 bis 1-4 dargestellt ist, und
mindestens eines von Z1 und Z2 eine Heteroarylgruppe ist, die durch eine der folgenden chemischen Formeln 1-1 bis 1-3 dargestellt ist, 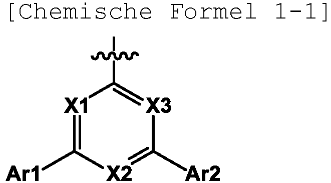 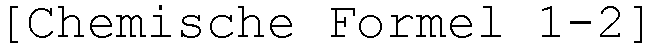 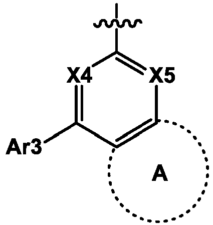 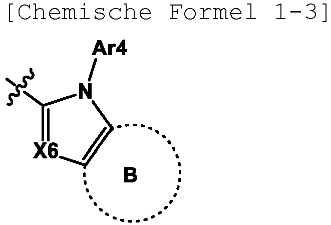 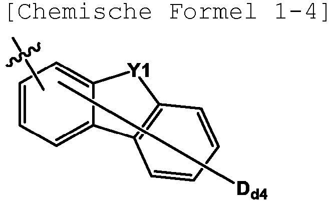
wobei in den chemischen Formeln 1-1 bis 1-4
d4 eine ganze Zahl von 0 bis 7 ist,
X1 bis X6 jeweils N oder CR sind,
mindestens eines von X1 bis X3 N ist,
mindestens eines von X4 und X5 N ist,
Y1 O; S oder NR' ist,
R, R', Ar1 bis Ar4 jeweils unabhängig Wasserstoff; Deuterium; eine Halogengruppe; eine Cyanogruppe; eine substituierte oder unsubstituierte C1 - bis C60 -Alkylgruppe; eine substituierte oder unsubstituierte C6 - bis C60 -Arylgruppe oder eine substituierte oder unsubstituierte C2 - bis C60 - Heteroarylgruppe sind,
A und B jeweils ein substituierter oder unsubstituierter monocyclischer oder polycyclischer Arylring oder ein monocyclischer oder polycyclischer Heteroring sind, der substituiert oder unsubstituiert ist und O oder S enthält, und
wenn L1 eine direkte Bindung ist und Z1 die Heteroarylgruppe ist, die durch die chemische Formel 1-1 dargestellt ist, Z2 eine substituierte oder unsubstituierte Silylgruppe; eine substituierte oder unsubstituierte Fluorenylgruppe oder die tricyclische oder höhere Heteroarylgruppe ist, die durch eine der chemischen Formeln 1-2 bis 1-4 dargestellt ist.

2. Verbindung nach Anspruch 1, wobei Ar1 und Ar2 der chemischen Formel 1-1 jeweils unabhängig eine substituierte oder unsubstituierte C6 - bis C30 -Arylgruppe oder eine substituierte oder unsubstituierte C2 - bis C30 -Heteroarylgruppe sind.

3. Verbindung nach Anspruch 1, wobei A der chemischen Formel 1-2 ein substituierter oder unsubstituierter monocyclischer Arylring oder ein polycyclischer Heteroring ist, der substituiert oder unsubstituiert ist und O oder S enthält, und B der chemischen Formel 1-3 ein substituierter oder unsubstituierter monocyclischer Arylring ist.

4. Verbindung nach Anspruch 1, wobei mindestens eines von Z1 und Z2 die Heteroarylgruppe ist, die durch die chemische Formel 1-1 dargestellt ist.

5. Verbindung nach Anspruch 1, wobei die chemische Formel 1 durch eine der folgenden Verbindungen dargestellt ist:

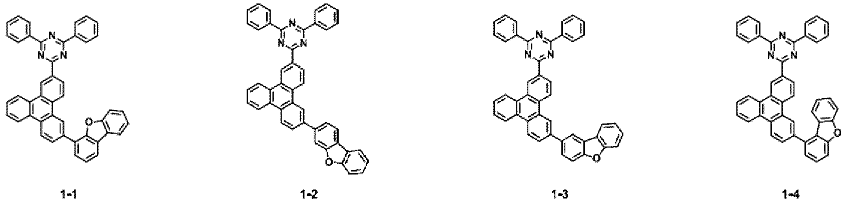

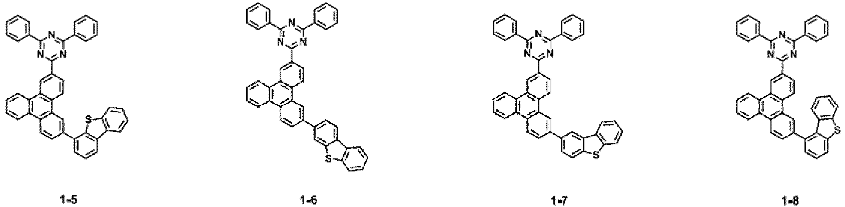

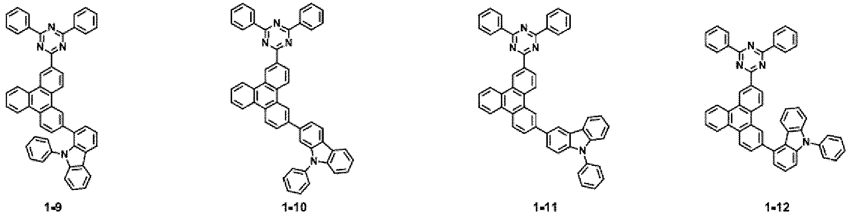

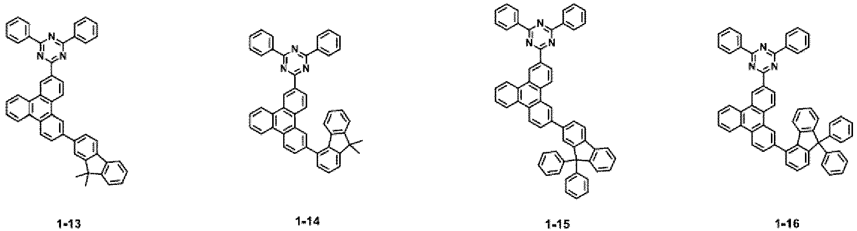

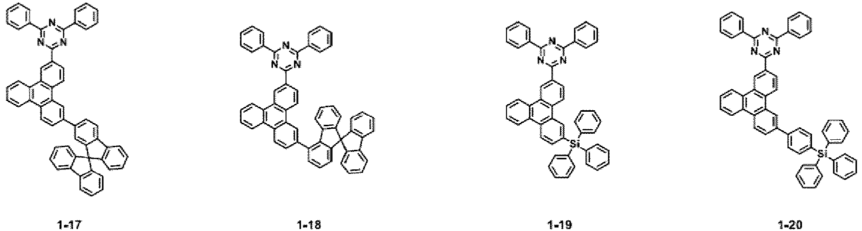

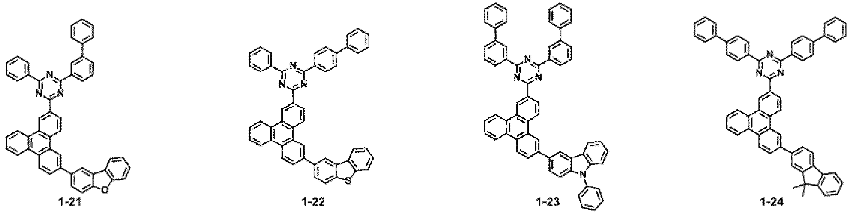

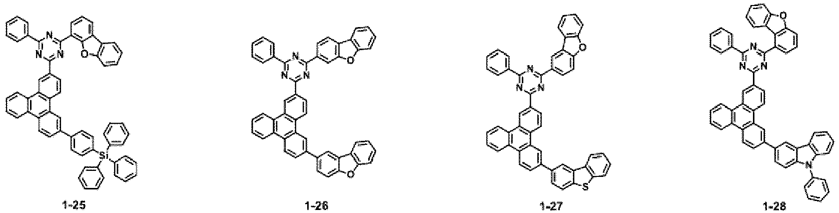

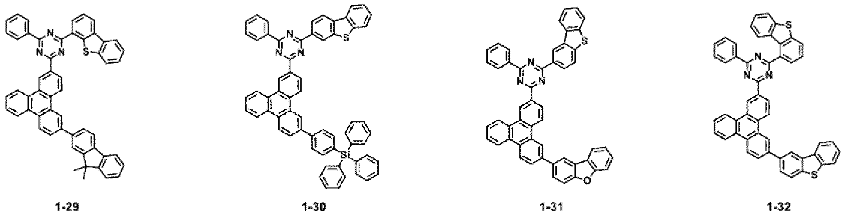

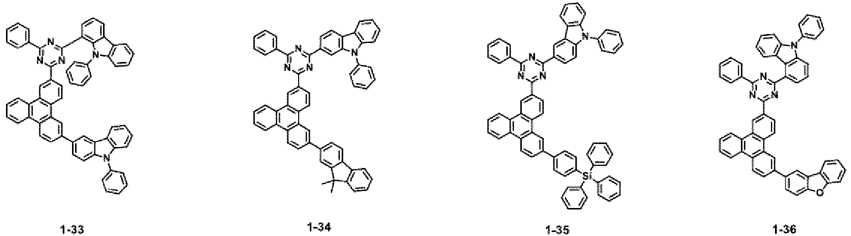

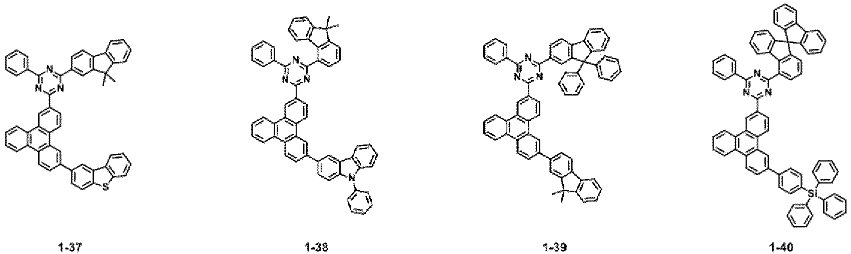

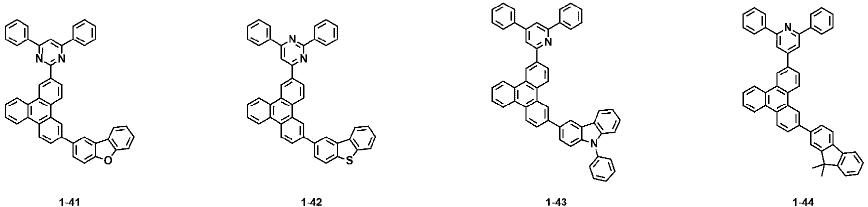

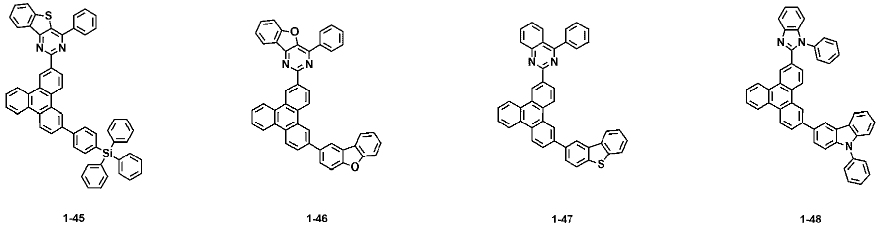

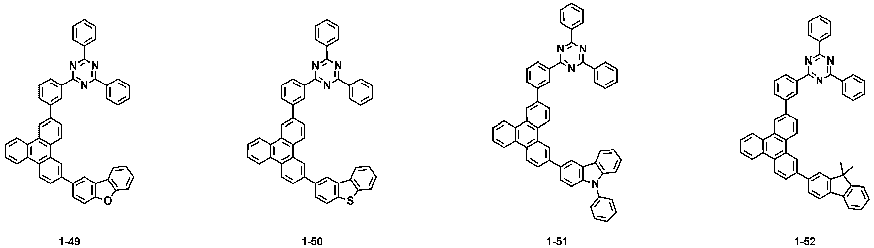

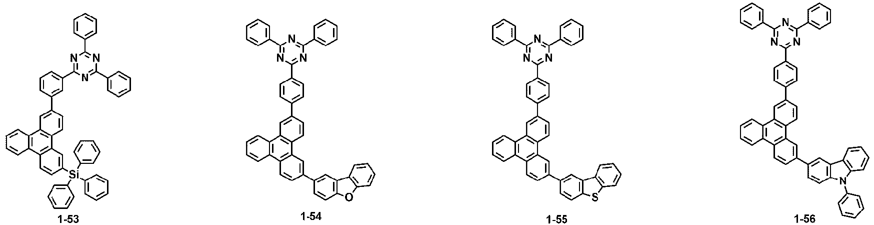

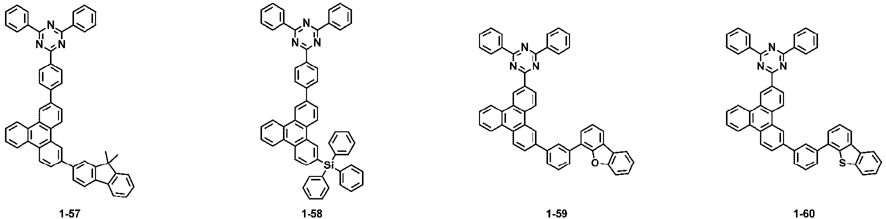

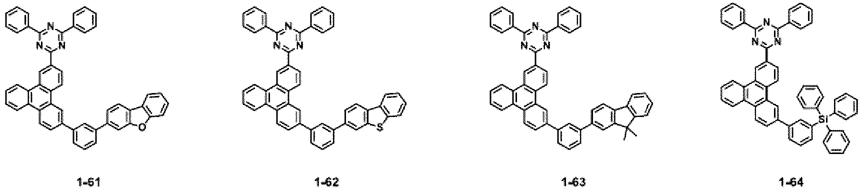

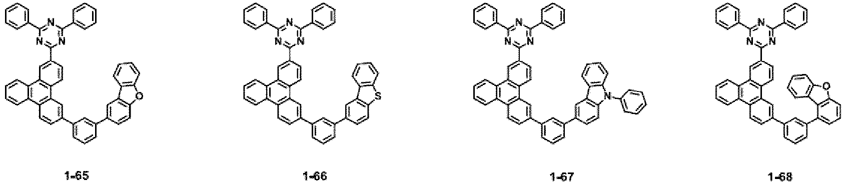

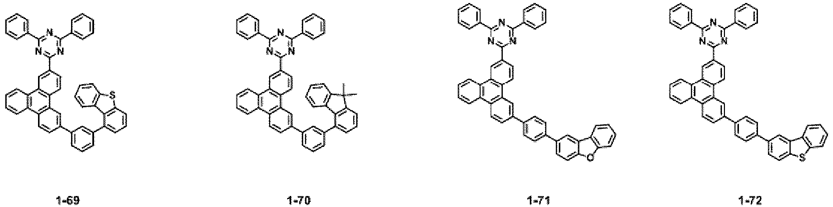

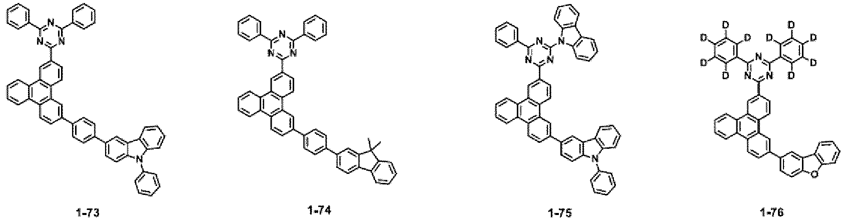

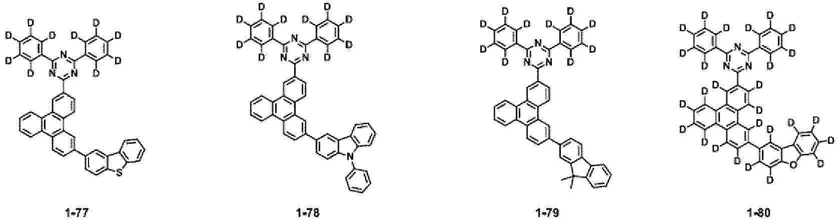

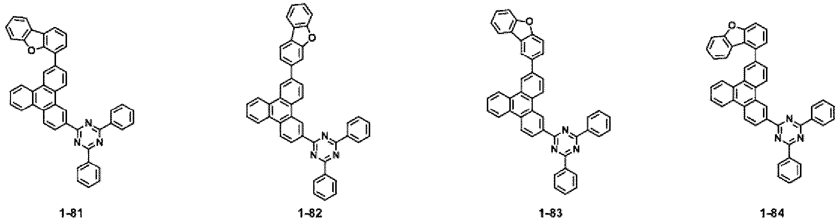

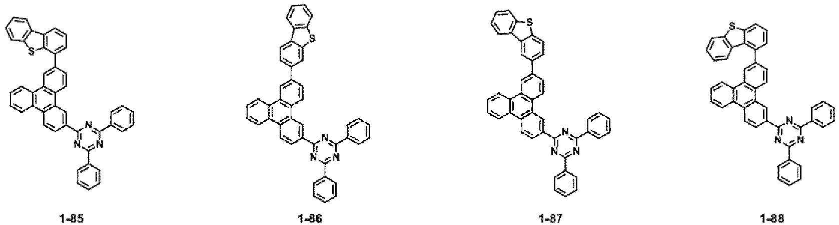

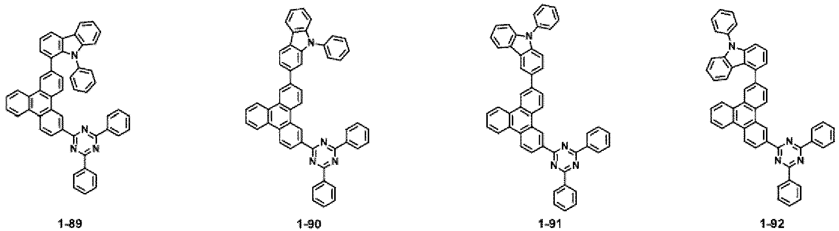

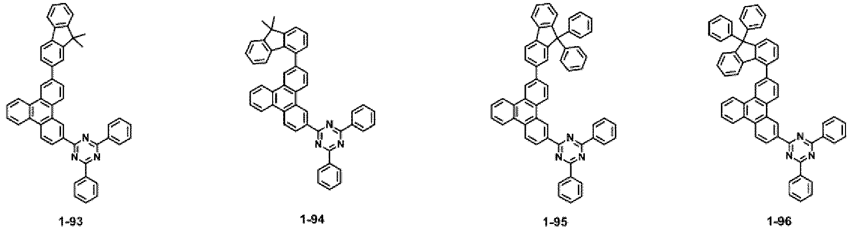

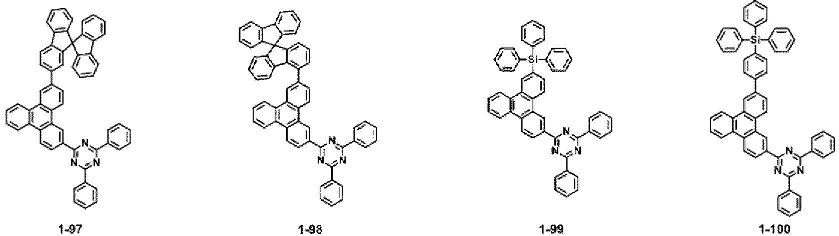

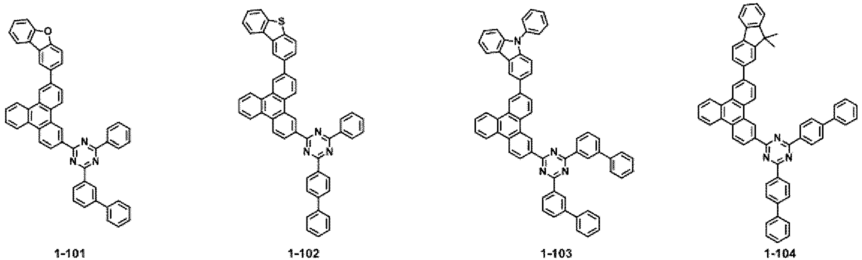

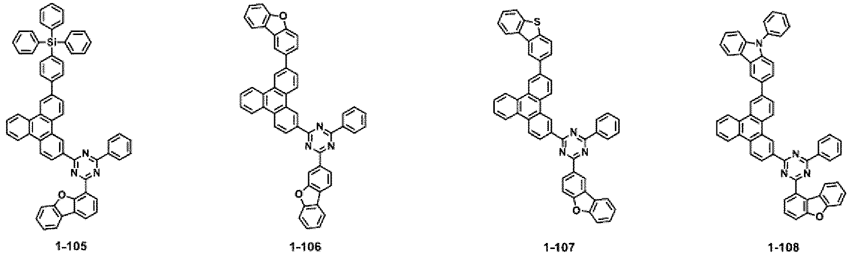

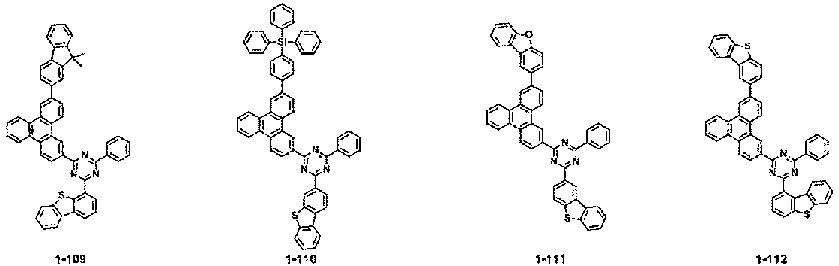

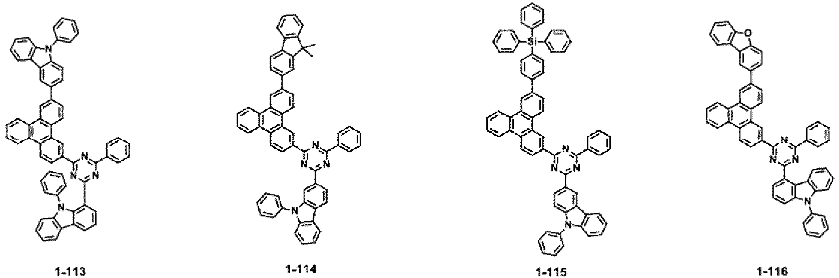

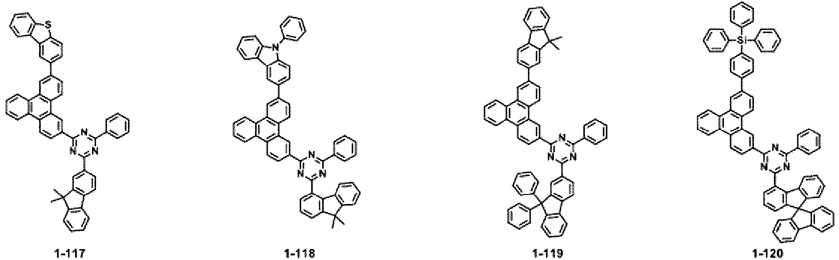

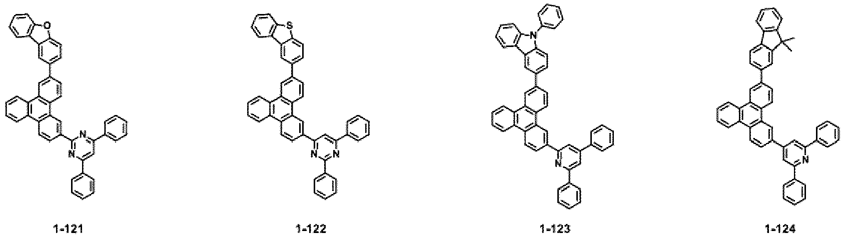

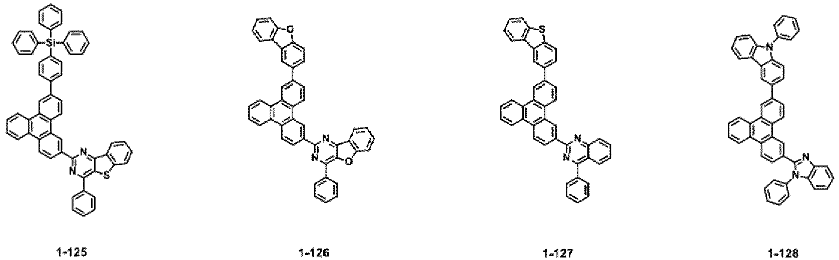

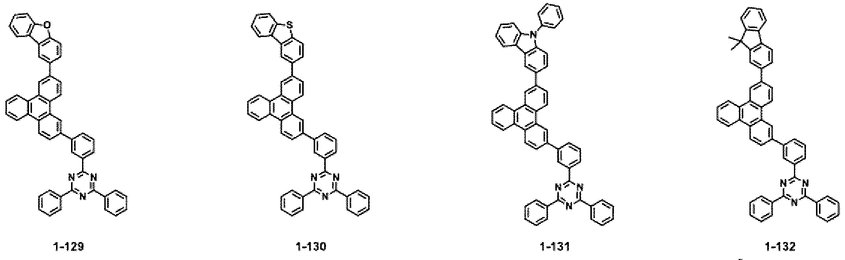

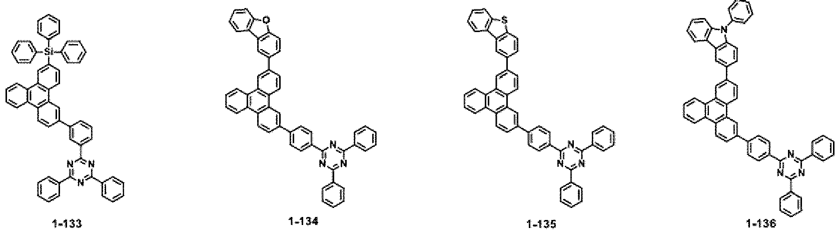

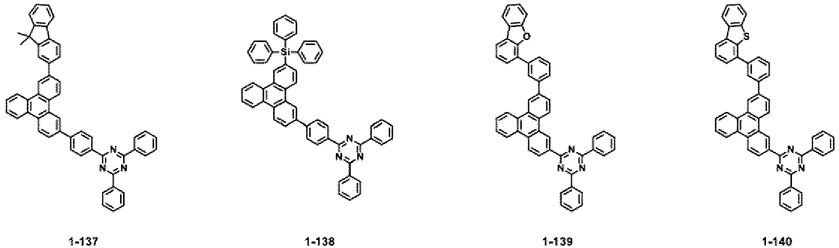

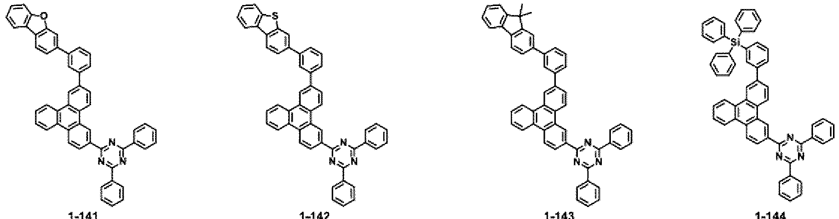

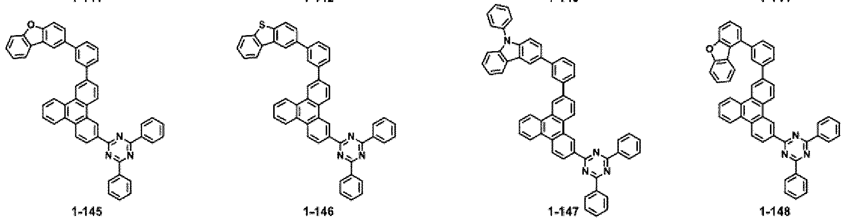

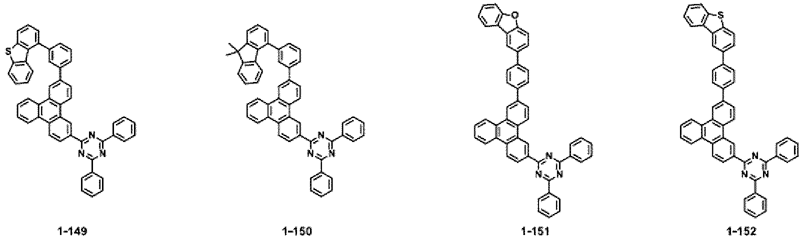

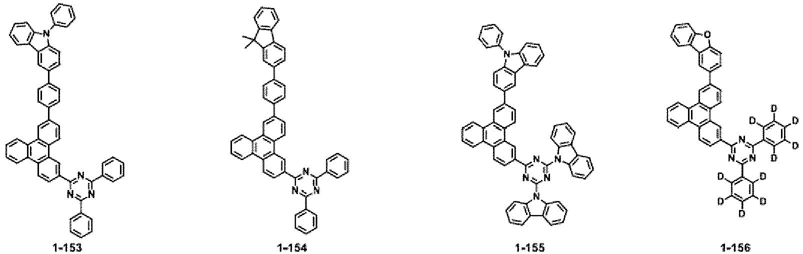

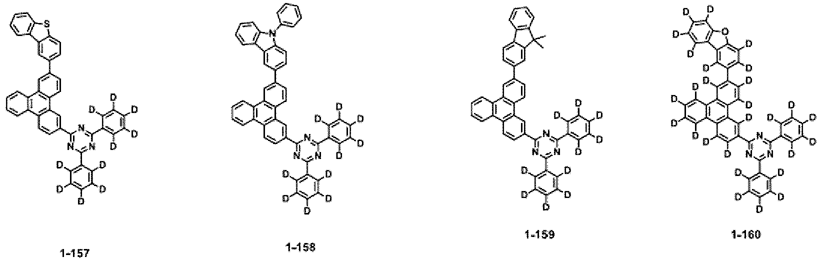

6. Organische lichtemittierende Vorrichtung, umfassend:
eine erste Elektrode;
eine zweite Elektrode; und
eine organische Materialschicht, die zwischen der ersten Elektrode und der zweiten Elektrode bereitgestellt ist,
wobei die organische Materialschicht eine oder mehrere Arten der Verbindung nach einem der Ansprüche 1 bis 5 enthält.

7. Organische lichtemittierende Vorrichtung nach Anspruch 6, wobei die organische Materialschicht eine lichtemittierende Schicht enthält und die lichtemittierende Schicht die Verbindung enthält.

8. Organische lichtemittierende Vorrichtung nach Anspruch 6, wobei die organische Materialschicht eine lichtemittierende Schicht enthält, die lichtemittierende Schicht einen Wirt enthält und der Wirt die Verbindung enthält.

9. Organische lichtemittierende Vorrichtung nach Anspruch 7, wobei die lichtemittierende Schicht ferner eine Verbindung der folgenden Chemischen Formel 2 oder 3 enthält: 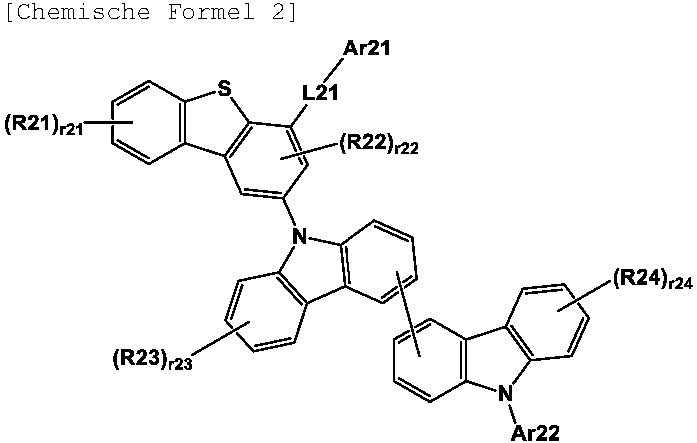 wobei in der Chemischen Formel 2
R21 bis R24 jeweils unabhängig Wasserstoff; Deuterium; eine Halogengruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte C6 - bis C60 -Arylgruppe oder eine substituierte oder unsubstituierte C2 - bis C60 -Heteroarylgruppe sind,
L21 eine direkte Bindung; eine substituierte oder unsubstituierte C6 - bis C60 -Arylengruppe oder eine substituierte oder unsubstituierte C2 - bis C60 - Heteroarylengruppe ist,
Ar21 und Ar22 jeweils unabhängig eine substituierte oder unsubstituierte C6 - bis C60 -Arylgruppe oder eine substituierte oder unsubstituierte C2 - bis C60 -Heteroarylgruppe sind,
r21 eine ganze Zahl von 1 bis 4 ist und, wenn 2 oder größer, R21s gleich oder verschieden voneinander sind,
r22 1 oder 2 ist und, wenn 2, R22s gleich oder verschieden voneinander sind,
r23 eine ganze Zahl von 1 bis 4 ist und, wenn 2 oder größer, R23s gleich oder verschieden voneinander sind, und
r24 eine ganze Zahl von 1 bis 4 ist und, wenn 2 oder größer, R24s gleich oder verschieden voneinander sind, 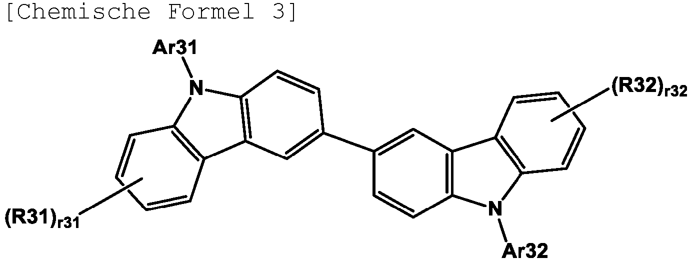
wobei in der Chemischen Formel 3
R31 und R32 jeweils unabhängig Wasserstoff; Deuterium; eine Halogengruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte C6 - bis C60 -Arylgruppe oder eine substituierte oder unsubstituierte C2 - bis C60 -Heteroarylgruppe sind,
Ar31 und Ar32 jeweils unabhängig eine substituierte oder unsubstituierte C6 - bis C60 -Arylgruppe oder eine C2 - bis C60 -Heteroarylgruppe sind, die substituiert oder unsubstituiert ist und O oder N enthält,
r31 eine ganze Zahl von 1 bis 4 ist und, wenn 2 oder größer, R31s gleich oder verschieden voneinander sind, und
r32 eine ganze Zahl von 1 bis 4 ist und, wenn 2 oder größer, R32s gleich oder verschieden voneinander sind.

10. Organische lichtemittierende Vorrichtung nach Anspruch 9, wobei die chemische Formel 2 durch eine der folgenden Verbindungen dargestellt ist:

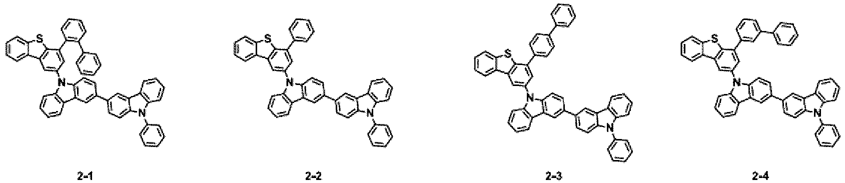

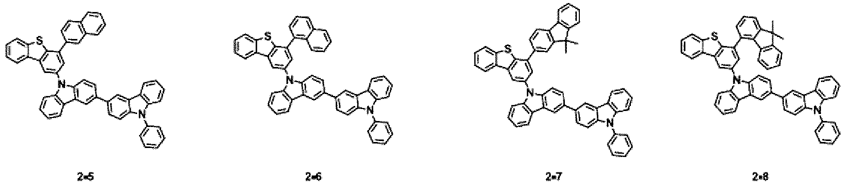

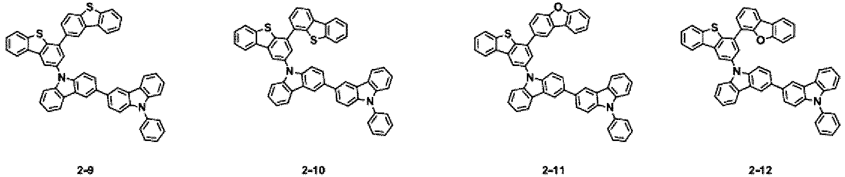

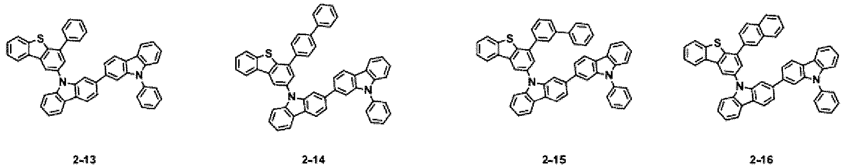

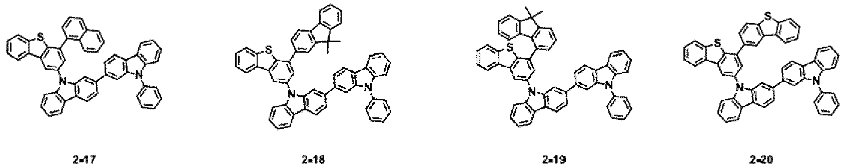

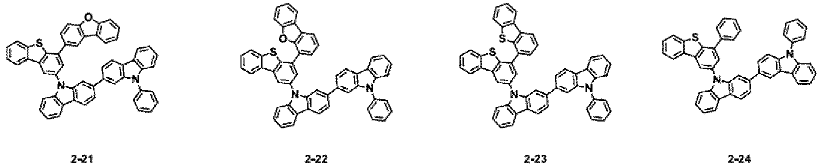

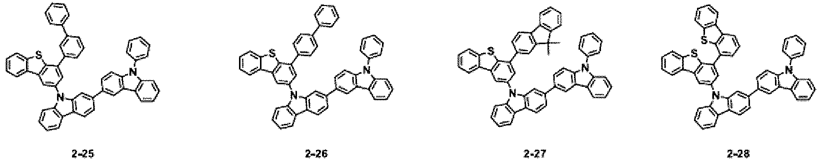

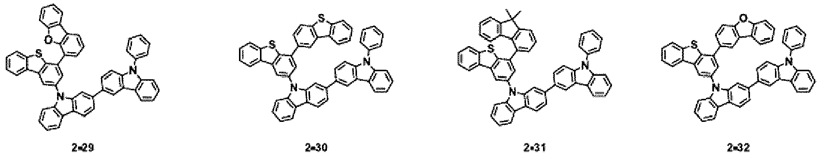

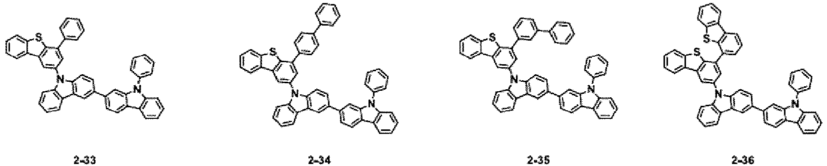

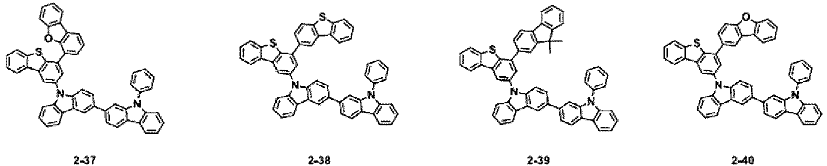

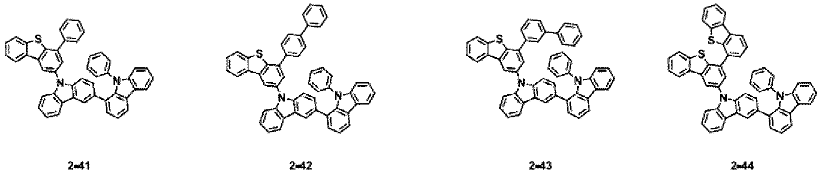

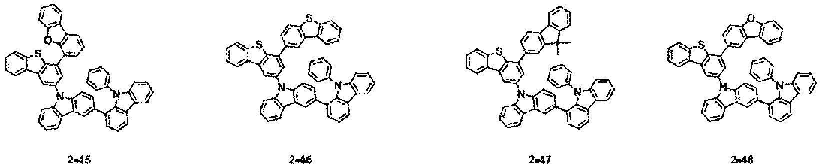

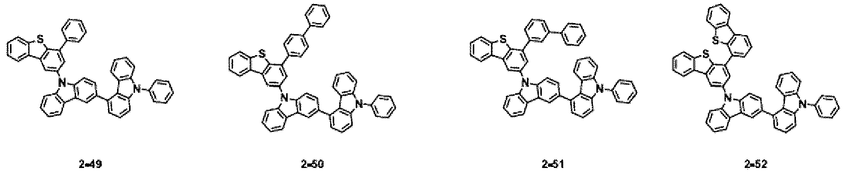

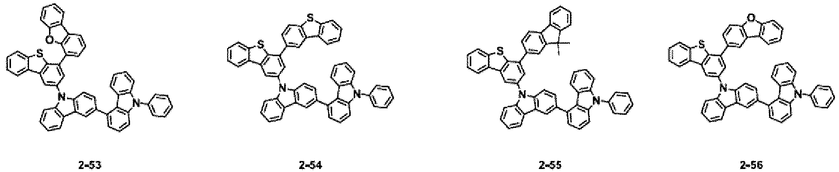

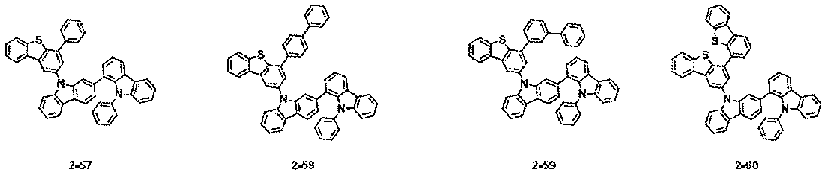

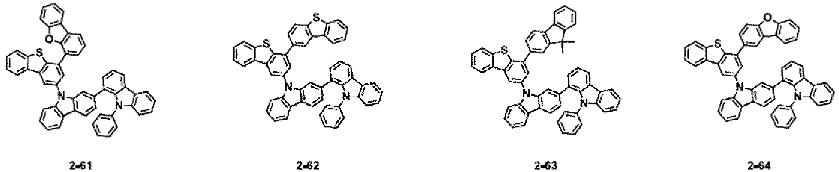

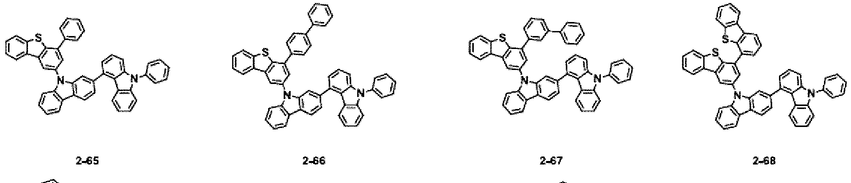

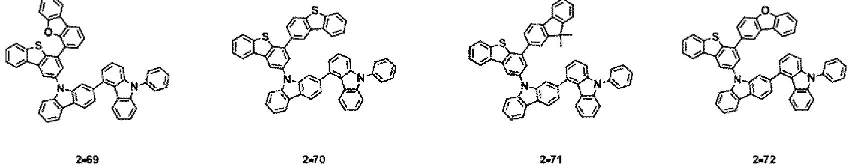

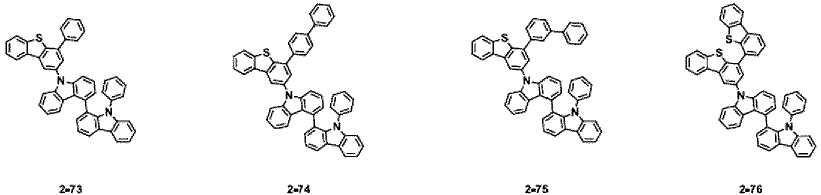

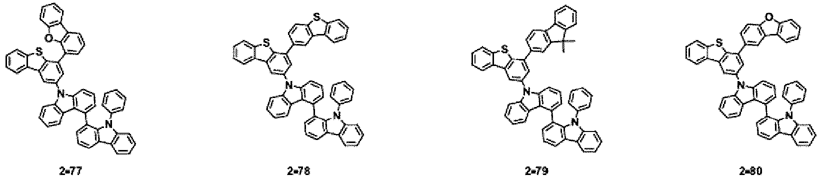

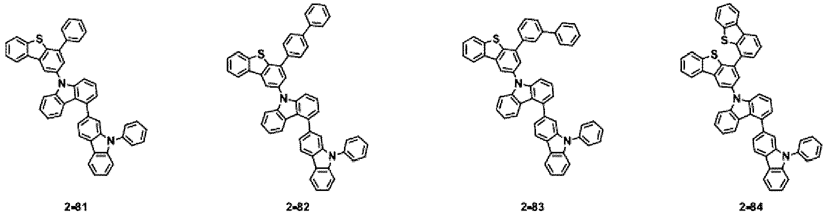

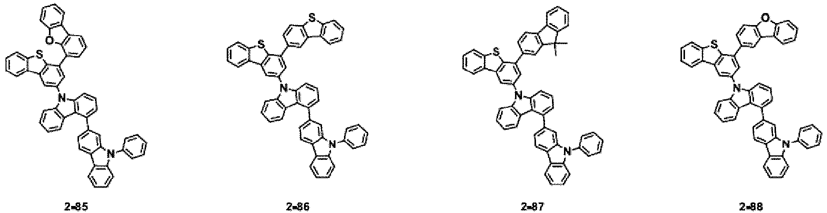

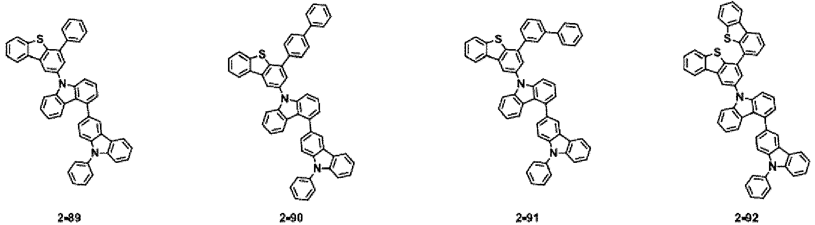

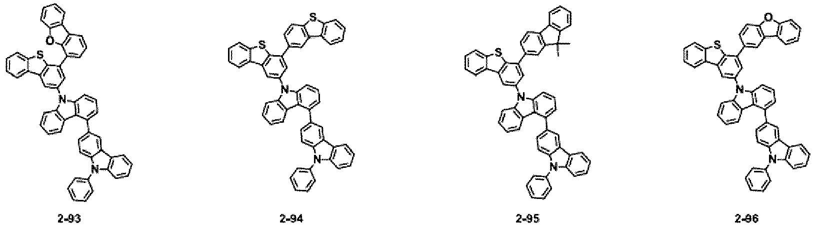

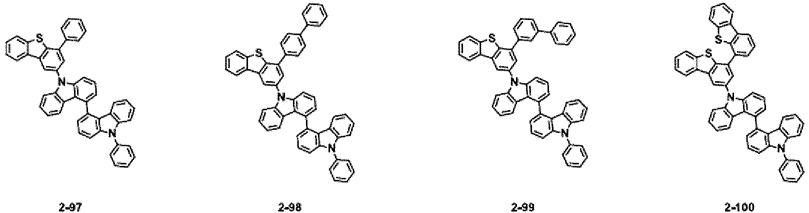

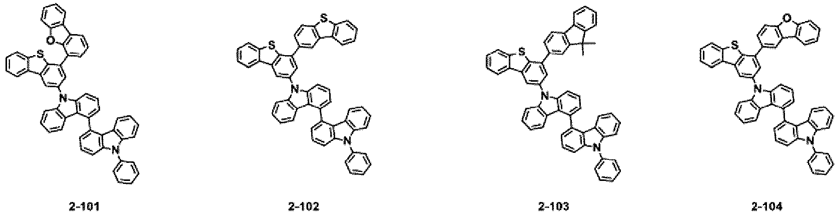

11. Organische lichtemittierende Vorrichtung nach Anspruch 9, wobei die chemische Formel 3 durch eine der folgenden Verbindungen dargestellt ist:

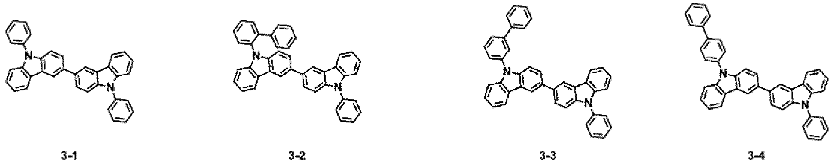

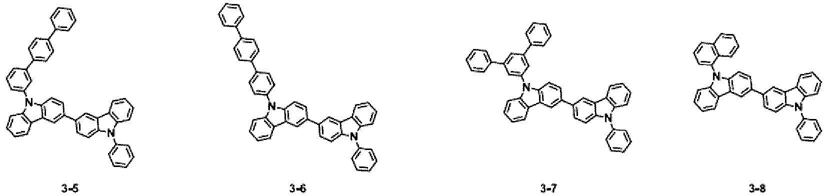

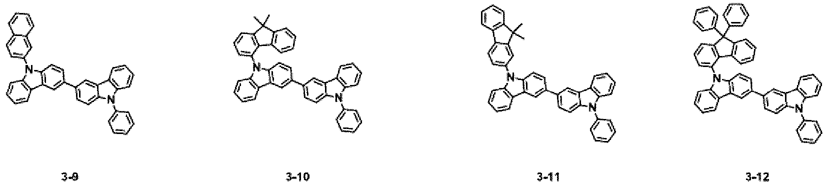

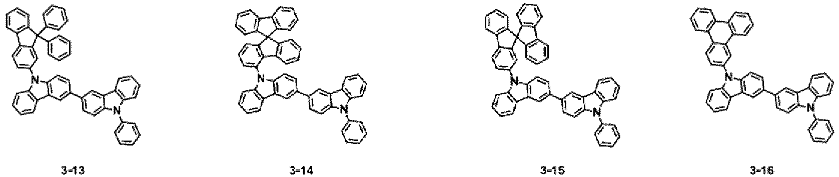

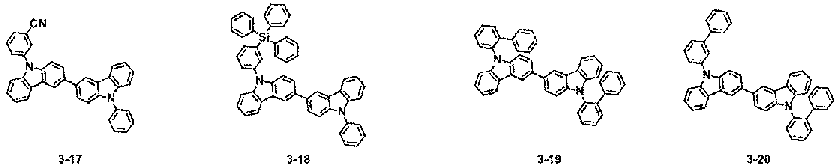

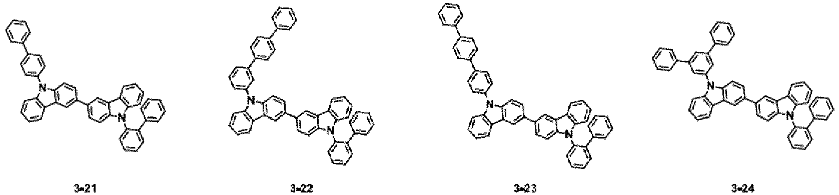

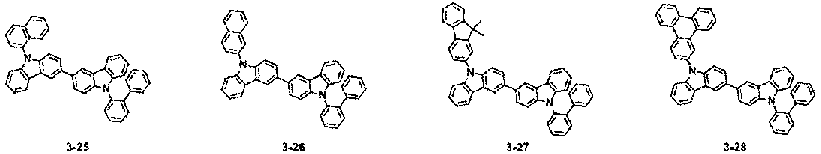

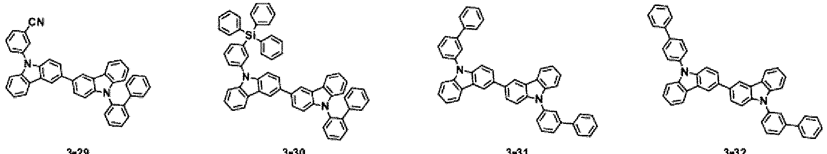

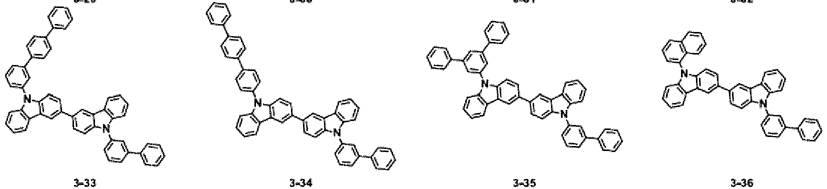

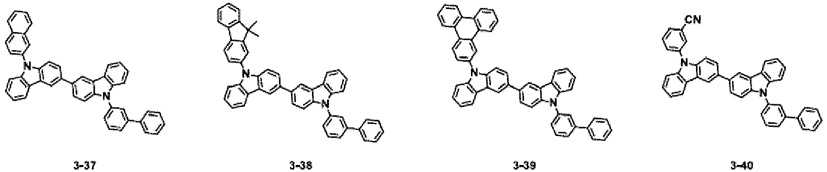

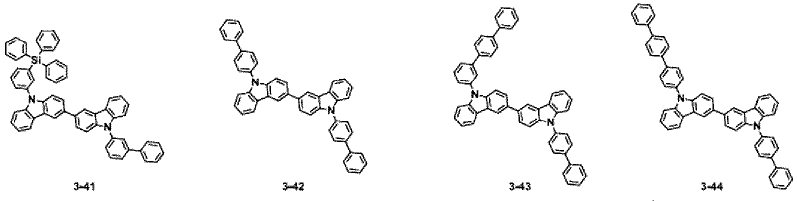

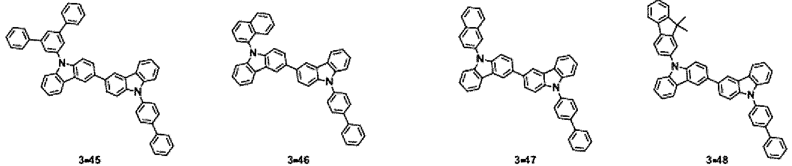

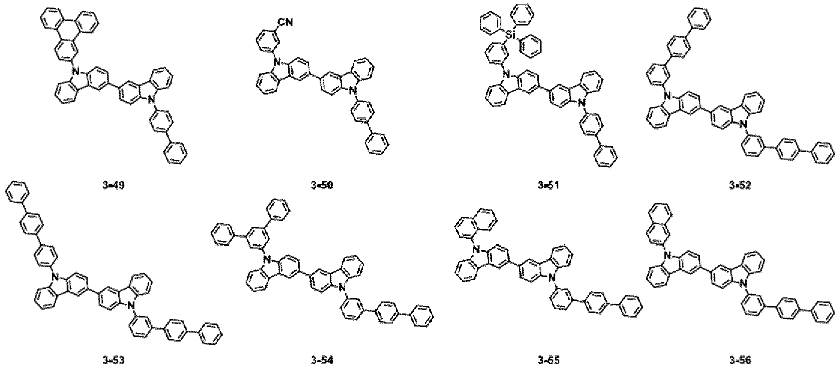

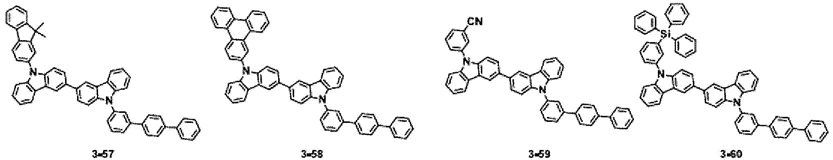

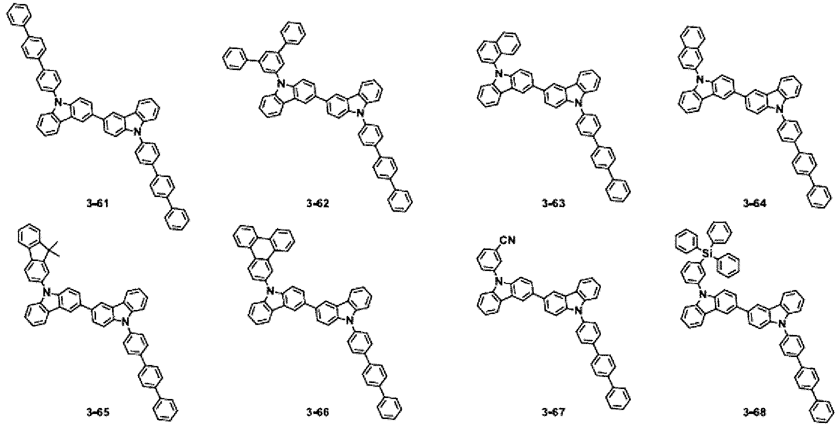

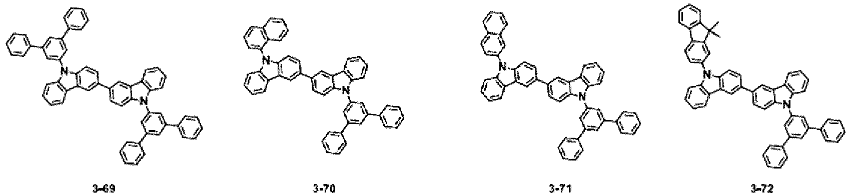

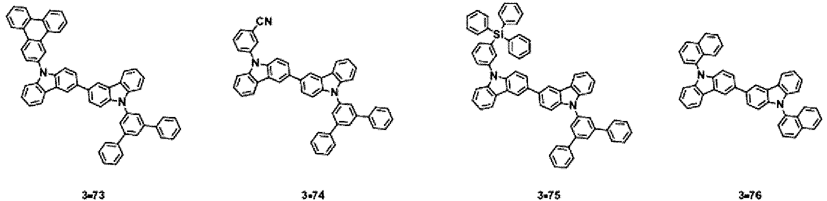

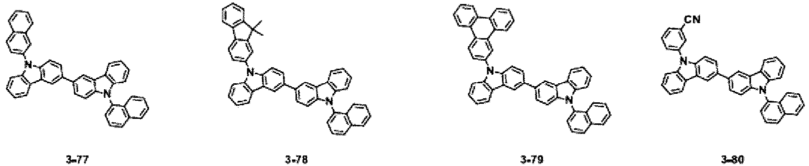

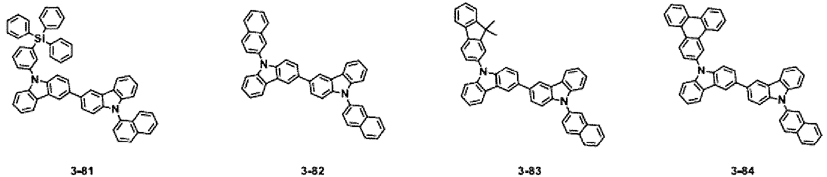

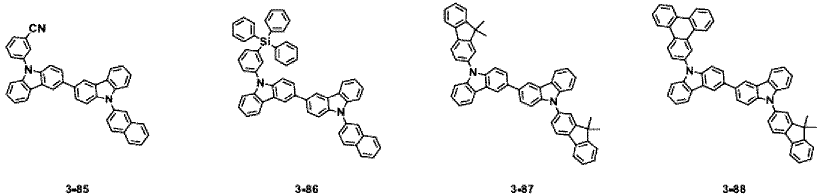

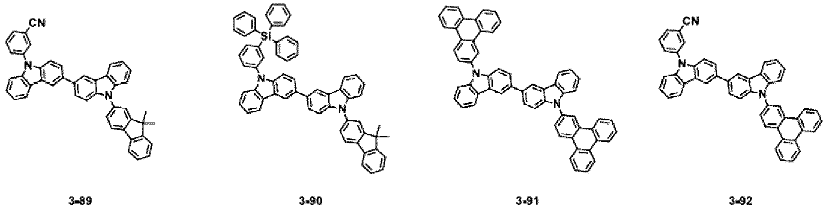

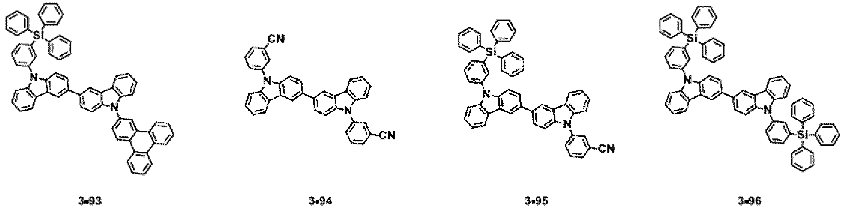

12. Zusammensetzung zum Bilden einer organischen Materialschicht, wobei die Zusammensetzung umfasst:
eine Verbindung der folgenden chemischen Formel 1; und
eine Verbindung der folgenden chemischen Formel 2 oder 3: 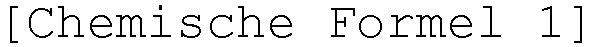 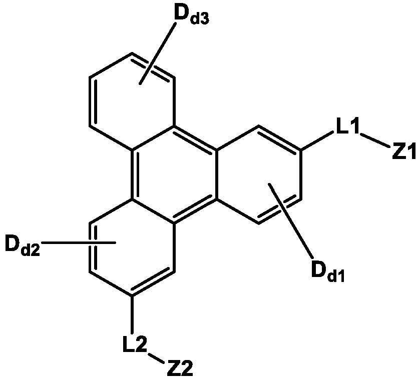
wobei in der chemischen Formel 1
d1 und d2 jeweils eine ganze Zahl von 0 bis 3 sind,
d3 eine ganze Zahl von 0 bis 4 ist,
L1 und L2 gleich oder verschieden voneinander sind und jeweils unabhängig eine direkte Bindung oder eine substituierte oder unsubstituierte C6 - bis C60 -Arylengruppe sind,
Z1 und Z2 gleich oder verschieden voneinander sind und jeweils unabhängig eine substituierte oder unsubstituierte Silylgruppe, eine substituierte oder unsubstituierte Fluorenylgruppe oder eine Heteroarylgruppe sind, die durch eine der folgenden chemischen Formeln 1-1 bis 1-4 dargestellt ist, und
mindestens eines von Z1 und Z2 eine Heteroarylgruppe ist, die durch eine der folgenden chemischen Formeln 1-1 bis 1-3 dargestellt ist, 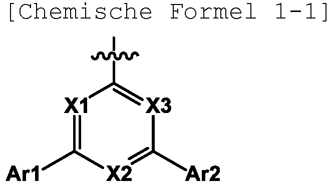 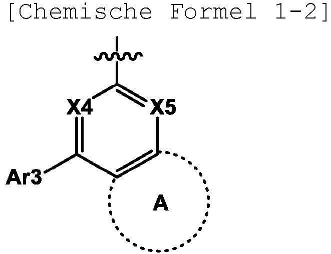 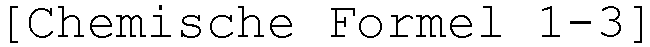 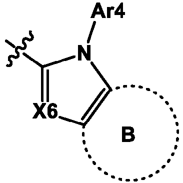 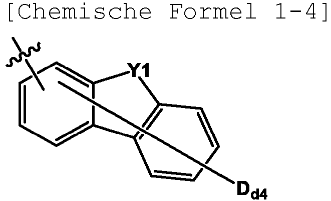
wobei in den chemischen Formeln 1-1 bis 1-4
d4 eine ganze Zahl von 0 bis 7 ist,
X1 bis X6 jeweils N oder CR sind,
mindestens eines von X1 bis X3 N ist,
mindestens eines von X4 und X5 N ist,
Y1 O; S oder NR' ist,
R, R', Ar1 bis Ar4 jeweils unabhängig Wasserstoff; Deuterium; eine Halogengruppe; eine Cyanogruppe; eine substituierte oder unsubstituierte C1 - bis C60 -Alkylgruppe; eine substituierte oder unsubstituierte C6 - bis C60 -Arylgruppe oder eine substituierte oder unsubstituierte C2 - bis C60 - Heteroarylgruppe sind,
A und B jeweils ein substituierter oder unsubstituierter monocyclischer oder polycyclischer Arylring oder ein monocyclischer oder polycyclischer Heteroring sind, der substituiert oder unsubstituiert ist und O oder S enthält, und
wenn L1 eine direkte Bindung ist und Z1 die Heteroarylgruppe ist, die durch die chemische Formel 1-1 dargestellt ist, Z2 eine substituierte oder unsubstituierte Silylgruppe, eine substituierte oder unsubstituierte Fluorenylgruppe oder die tricyclische oder höhere Heteroarylgruppe ist, die durch eine der chemischen Formeln 1-2 bis 1-4 dargestellt ist, 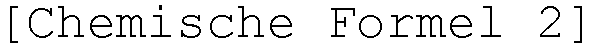 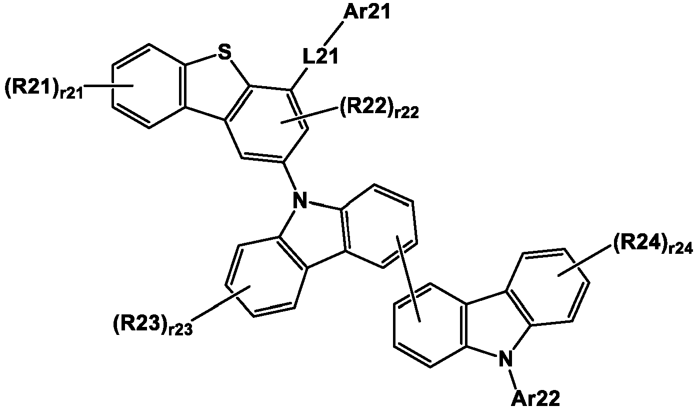
wobei in der chemischen Formel 2
R21 bis R24 jeweils unabhängig Wasserstoff; Deuterium; eine Halogengruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte C6 - bis C60 -Arylgruppe oder eine substituierte oder unsubstituierte C2 - bis C60 -Heteroarylgruppe sind,
L21 eine direkte Bindung; eine substituierte oder unsubstituierte C6 - bis C60 -Arylengruppe oder eine substituierte oder unsubstituierte C2 - bis C60 - Heteroarylengruppe ist,
Ar21 und Ar22 jeweils unabhängig eine substituierte oder unsubstituierte C6 - bis C60 -Arylgruppe oder eine substituierte oder unsubstituierte C2 - bis C60 -Heteroarylgruppe sind,
r21 eine ganze Zahl von 1 bis 4 ist und, wenn 2 oder größer, R21s gleich oder verschieden voneinander sind,
r22 1 oder 2 ist und, wenn **2,** R22s gleich oder verschieden voneinander sind,
r23 eine ganze Zahl von 1 bis 4 ist und, wenn 2 oder größer, R23s gleich oder verschieden voneinander sind, und
r24 eine ganze Zahl von 1 bis 4 ist und, wenn 2 oder größer, R24s gleich oder verschieden voneinander sind, 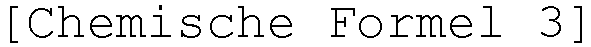 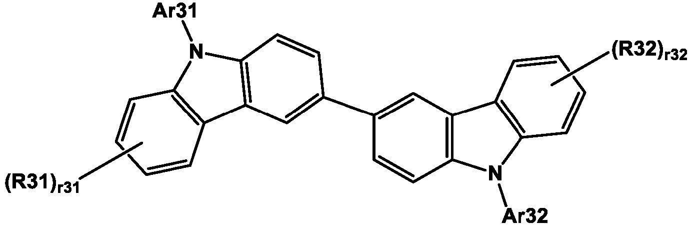
wobei in der Chemischen Formel 3
R31 und R32 jeweils unabhängig Wasserstoff; Deuterium; eine Halogengruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte C6 - bis C60 -Arylgruppe oder eine substituierte oder unsubstituierte C2 - bis C60 -Heteroarylgruppe sind,
Ar31 und Ar32 jeweils unabhängig eine substituierte oder unsubstituierte C6 - bis C60 -Arylgruppe oder eine C2 - bis C60 -Heteroarylgruppe sind, die substituiert oder unsubstituiert ist und O oder N enthält,
r31 eine ganze Zahl von 1 bis 4 ist und, wenn 2 oder größer, R31s gleich oder verschieden voneinander sind, und
r32 eine ganze Zahl von 1 bis 4 ist und, wenn 2 oder größer, R32s gleich oder verschieden voneinander sind.

13. Zusammensetzung zum Bilden einer organischen Materialschicht nach Anspruch 12, wobei die Verbindung der chemischen Formel 1 und die Verbindung der chemischen Formel 2 oder 3 ein Gewichtsverhältnis von 1:10 bis 10:1 aufweisen.

## Revendications

1. Composé de la Formule chimique 1 ci-après : 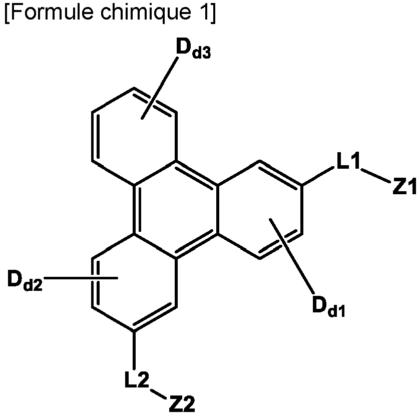 dans lequel, dans la Formule chimique 1,
d1 et d2 sont chacun un nombre entier de 0 à 3,
d3 est un nombre entier de 0 à 4,
L1 et L2 sont identiques ou différents les uns des autres, et chacun indépendamment une liaison directe ; ou un groupe arylène en C6 à C60 non substitué ou substitué,
Z1 et Z2 sont identiques ou différents les uns des autres, et chacun indépendamment un groupe silyle non substitué ou substitué ; un groupe fluorényle non substitué ou substitué ; ou un groupe hétéroaryle représenté par l'une quelconque des Formules chimiques 1-1 à 1-4 ci-après, et
au moins un parmi Z1 et Z2 est un groupe hétéroaryle représenté par l'une quelconque des Formules chimiques 1-1 à 1-3 ci-après, 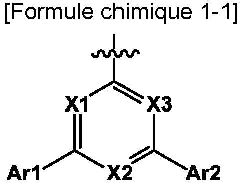 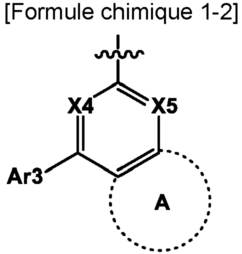 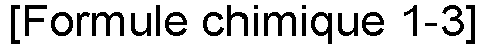 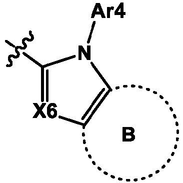 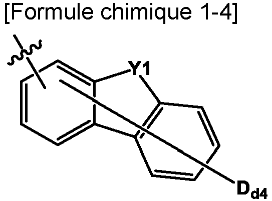
dans les Formules chimiques 1-1 à 1-4,
d4 est un nombre entier de 0 à 7,
X1 à X6 sont chacun N ou CR,
au moins un parmi X1 à X3 est N,
au moins un parmi X4 et X5 est N,
Y1 est O ; S ou NR',
R, R', Ar1 à Ar4 sont chacun indépendamment un hydrogène ; un deutérium ; un groupe halogène ; un groupe cyano ; un groupe alkyle en C1 à C60 non substitué ou substitué ; un groupe aryle en C6 à C60 non substitué ou substitué ; ou un groupe hétéroaryle en C2 à C60 non substitué ou substitué,
A et B sont chacun un groupe cycle aryle monocyclique ou polycyclique non substitué ou substitué ; ou un hétérocycle monocyclique ou polycyclique substitué ou non substitué et incluant O ou S, et
quand L1 est une liaison directe et Z1 est le groupe hétéroaryle représenté par la Formule chimique 1-1, Z2 est un groupe silyle non substitué ou substitué ; un groupe fluorényle non substitué ou substitué ; ou le groupe hétéroaryle tricyclique ou plus représenté par l'une quelconque des Formules chimiques 1-2 à 1-4.

2. Composé selon la revendication 1, dans lequel Ar1 et Ar2 de la Formule chimique 1-1 sont chacun indépendamment un groupe aryle en C6 à C30 non substitué ou substitué ; ou un groupe hétéroaryle en C2 à C30 non substitué ou substitué.

3. Composé selon la revendication 1, dans lequel A de la Formule chimique 1-2 est un cycle aryle monocyclique non substitué ou substitué ; ou un hétérocycle polycyclique substitué ou non substitué et incluant O ou S, et B de la Formule chimique 1-3 est un cycle aryle monocyclique non substitué ou substitué.

4. Composé selon la revendication 1, dans lequel au moins un parmi Z1 et Z2 est le groupe hétéroaryle représenté par la Formule chimique 1-1.

5. Composé selon la revendication 1, dans lequel la Formule chimique 1 est représentée par l'un quelconque des composés suivants :

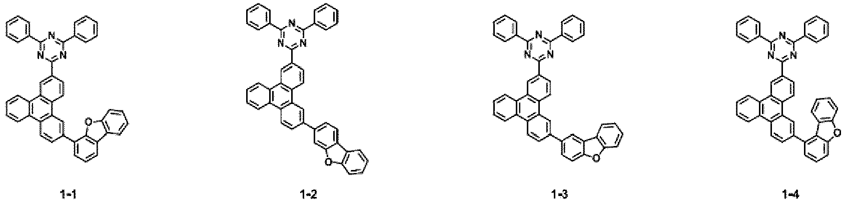

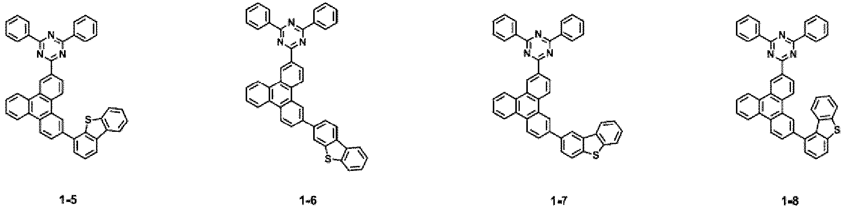

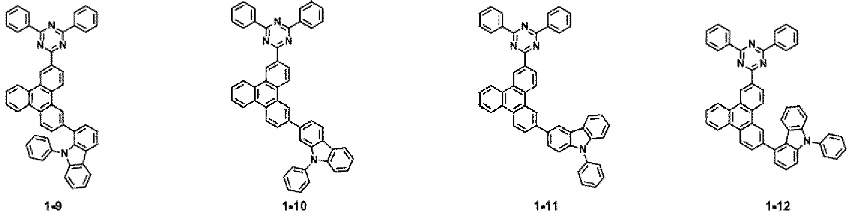

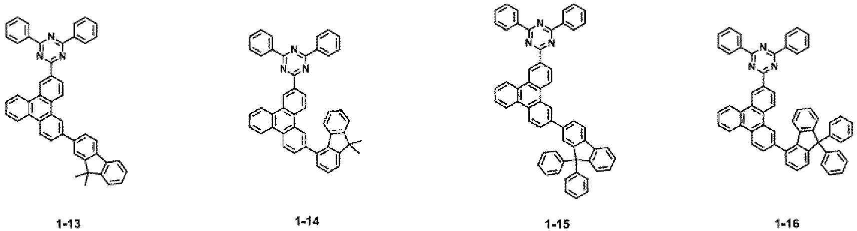

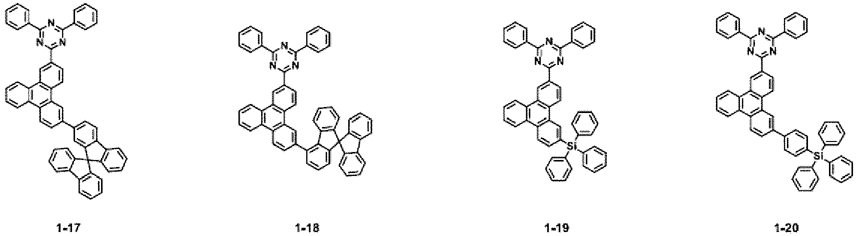

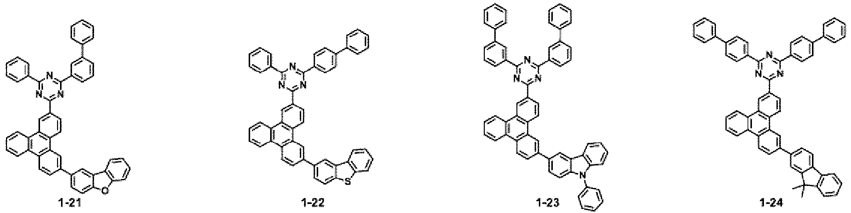

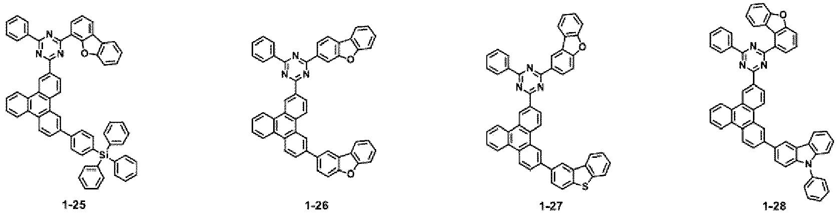

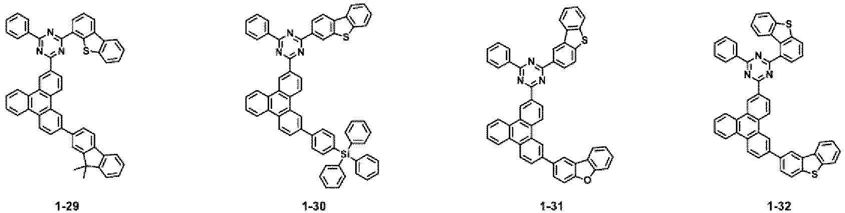

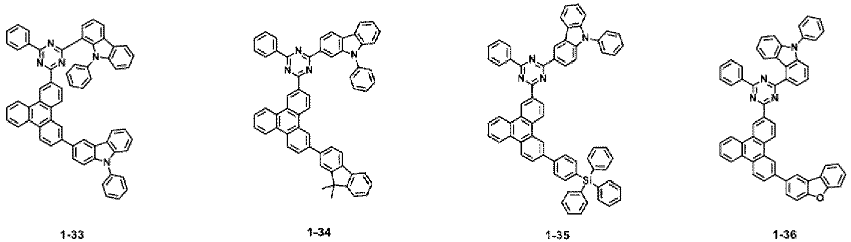

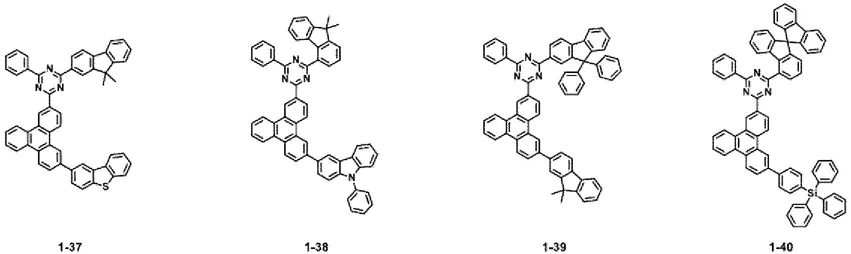

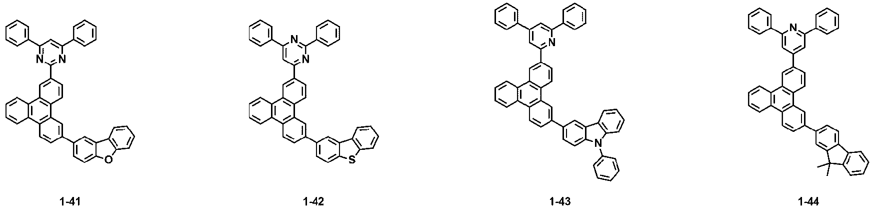

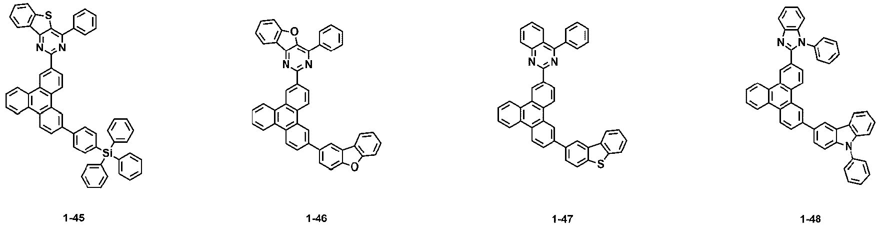

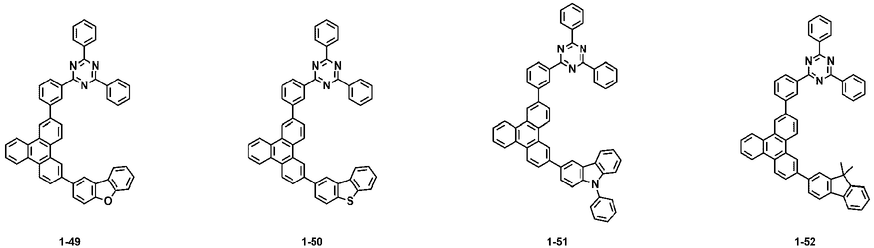

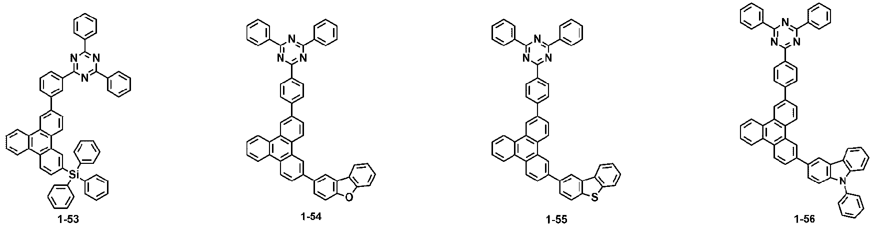

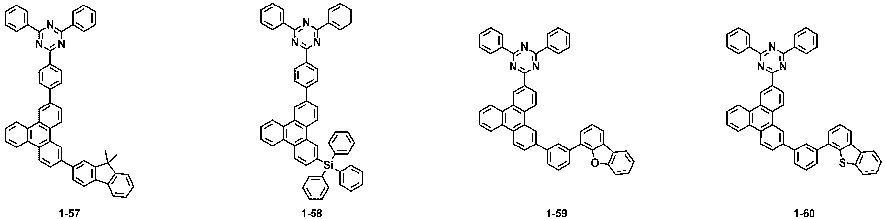

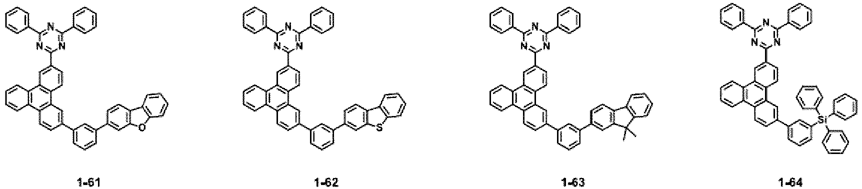

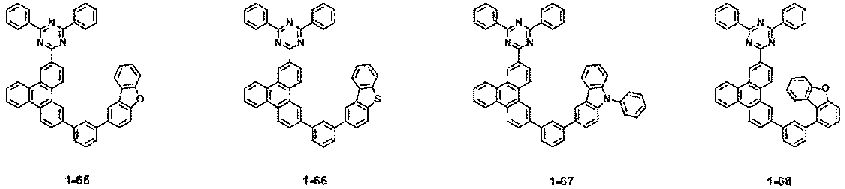

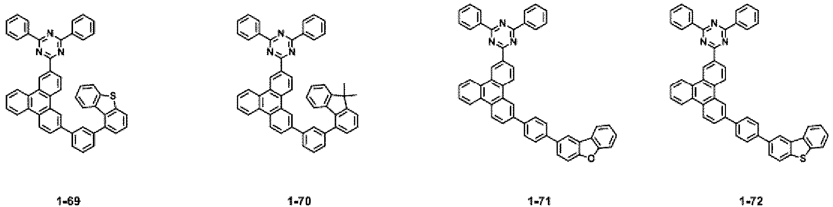

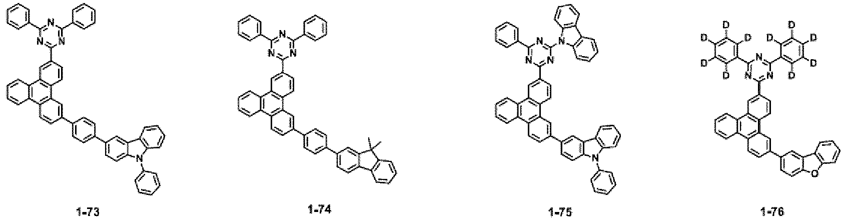

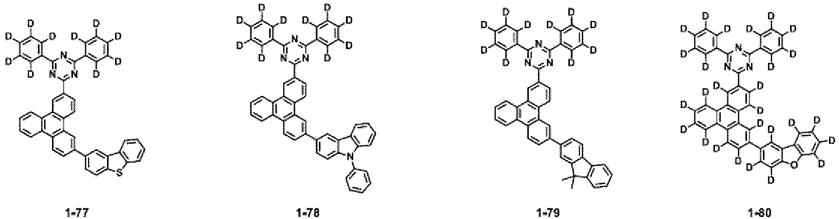

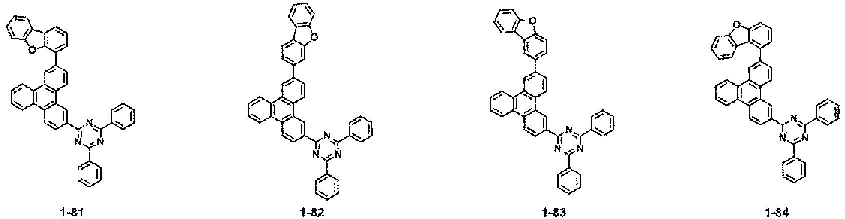

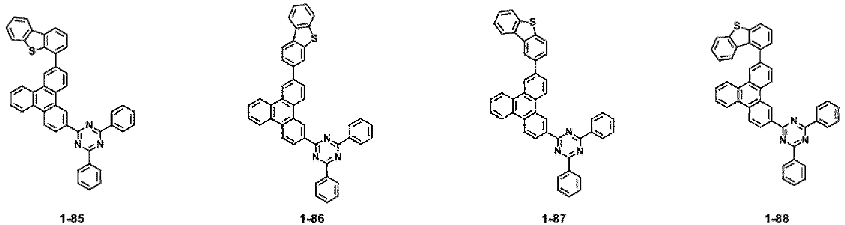

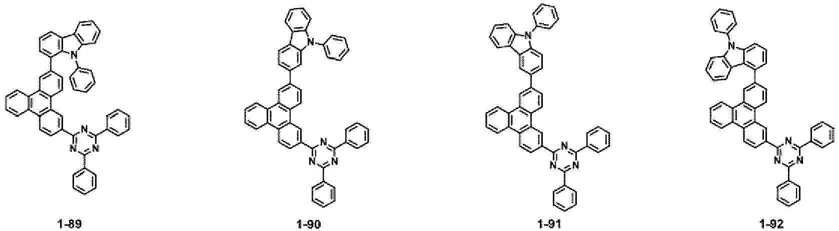

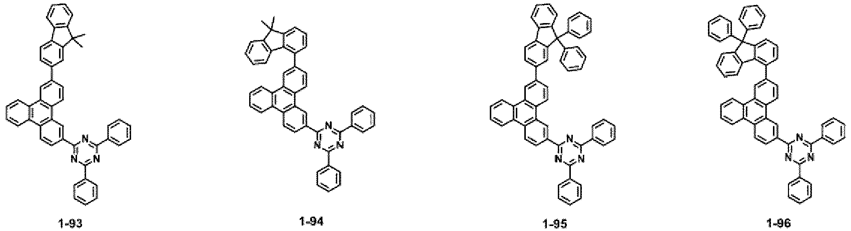

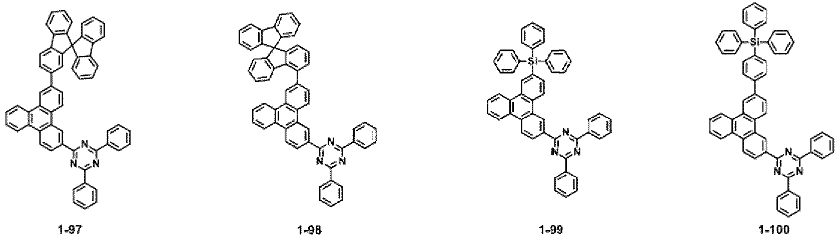

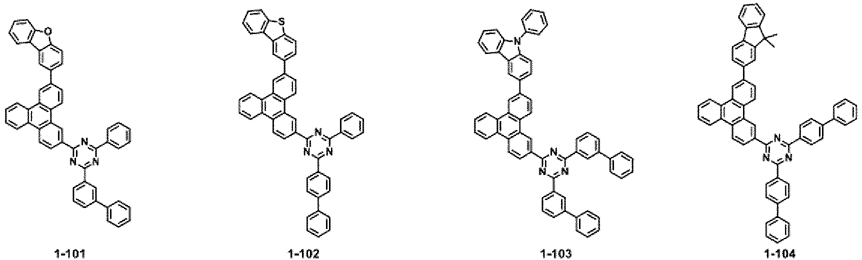

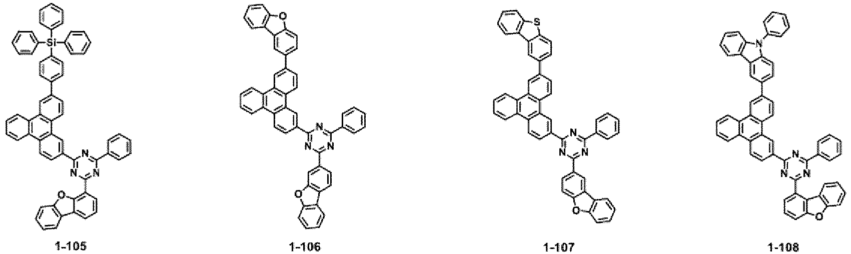

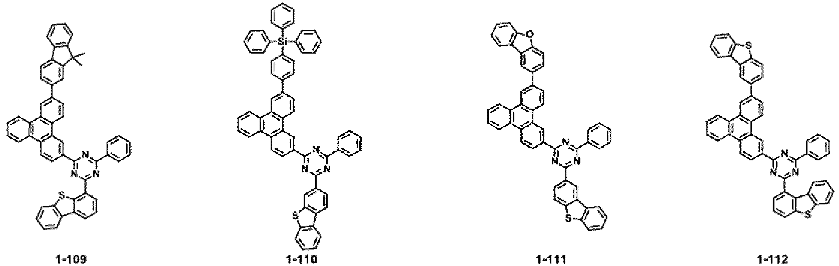

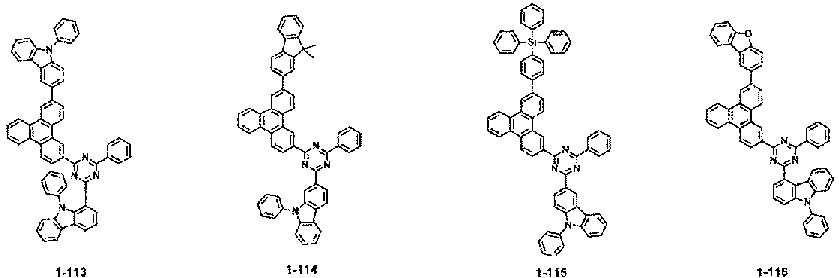

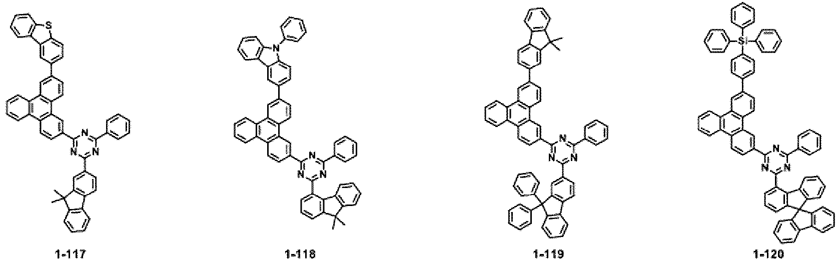

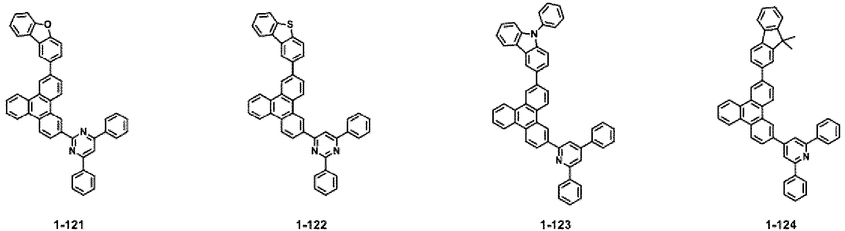

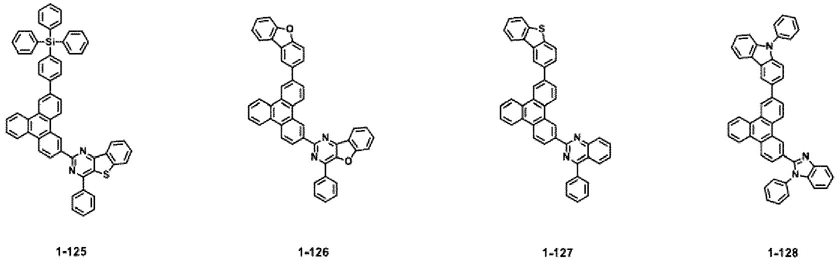

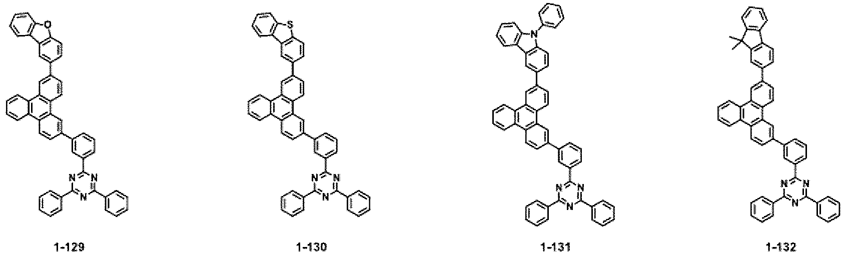

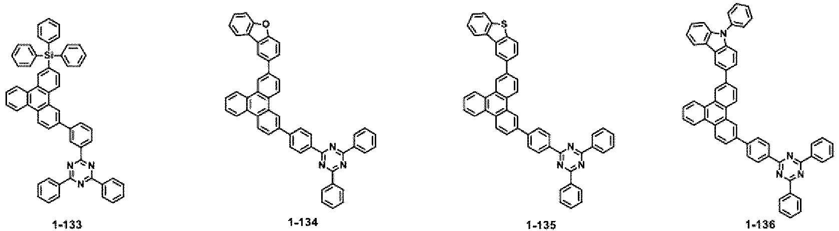

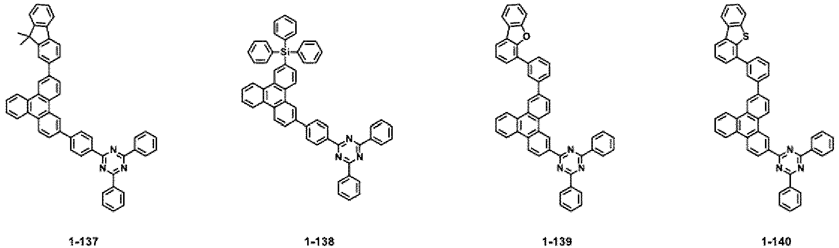

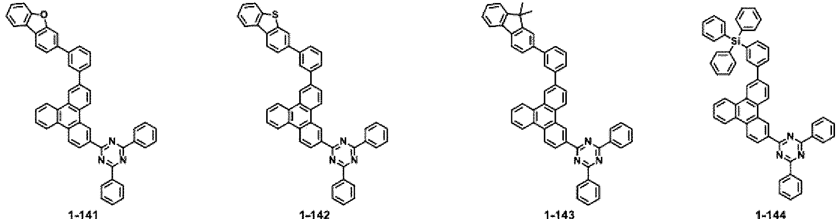

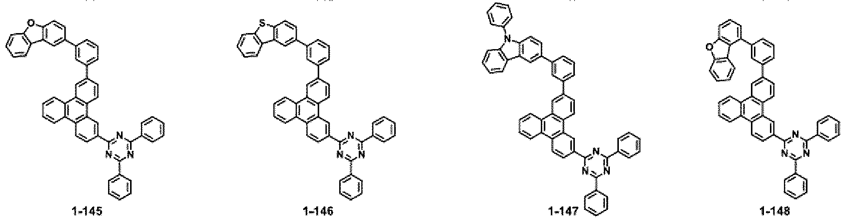

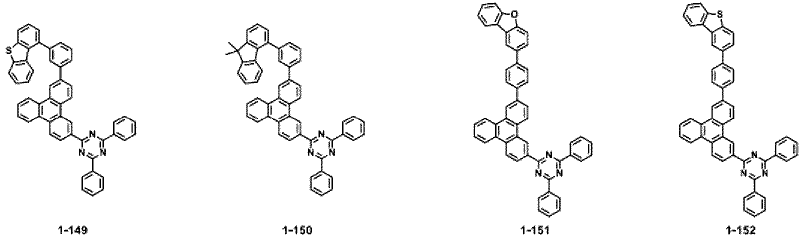

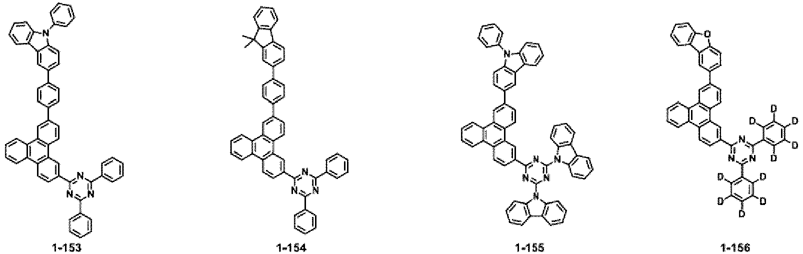

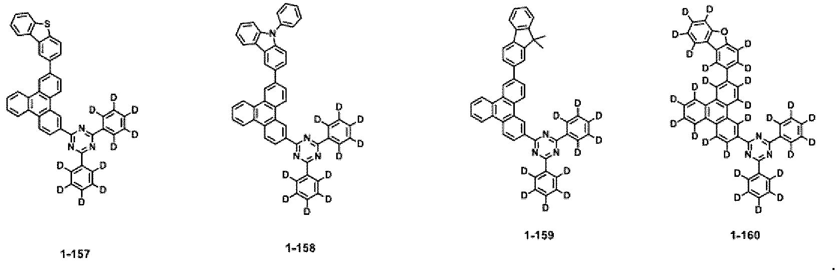

6. Dispositif électroluminescent organique comprenant :
une première électrode ;
une deuxième électrode ; et
une couche de matériau organique fournie entre la première électrode et la deuxième électrode,
dans lequel la couche de matériau organique inclut un ou plusieurs types du composé selon l'une quelconque des revendications 1 à 5.

7. Dispositif électroluminescent organique selon la revendication 6, dans lequel la couche de matériau organique inclut une couche électroluminescente, et la couche électroluminescente inclut le composé.

8. Dispositif électroluminescent organique selon la revendication 6, dans lequel la couche de matériau organique inclut une couche électroluminescente, la couche électroluminescente inclut un hôte, et l'hôte inclut le composé.

9. Dispositif électroluminescent organique selon la revendication 7, dans lequel la couche électroluminescente inclut en outre un composé de la Formule chimique 2 ou 3 ci-après : 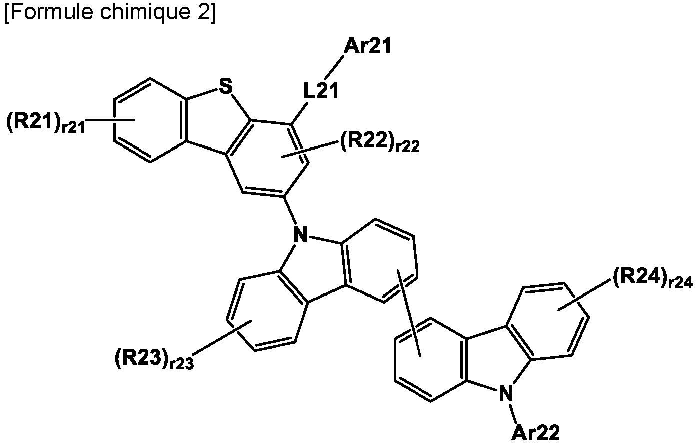 dans la Formule chimique 2,
R21 à R24 sont chacun indépendamment un hydrogène ; un deutérium ; un groupe halogène ; un groupe alkyle en C1 à C60 non substitué ou substitué ; un groupe cycloalkyle en C3 à C60 non substitué ou substitué ; un groupe aryle en C6 à C60 non substitué ou substitué ; ou un groupe hétéroaryle en C2 à C60 non substitué ou substitué,
L21 est une liaison directe ; un groupe arylène en C6 à C60 non substitué ou substitué ; ou un groupe hétéroarylène en C2 à C60 non substitué ou substitué,
Ar21 et Ar22 sont chacun indépendamment un groupe aryle en C6 à C60 non substitué ou substitué ; ou un groupe hétéroaryle en C2 à C60 non substitué ou substitué,
r21 est un nombre entier de 1 à 4, et quand il est 2 ou plus, les R21 sont identiques ou différents les uns des autres,
r22 est 1 ou 2, et quand il est 2, les R22 sont identiques ou différents les uns des autres,
r23 est un nombre entier de 1 à 4, et quand il est 2 ou plus, les R23 sont identiques ou différents les uns des autres, et
r24 est un nombre entier de 1 à 4, et quand il est 2 ou plus, les R24 sont identiques ou différents les uns des autres, 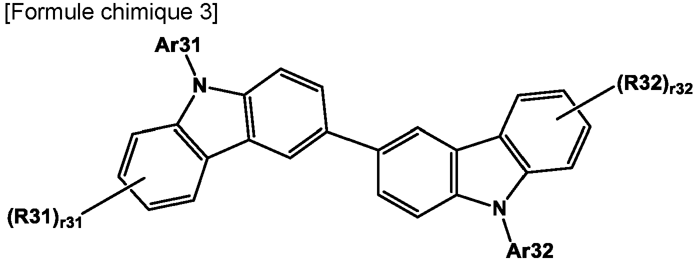
dans la Formule chimique 3,
R31 et R32 sont chacun indépendamment un hydrogène ; un deutérium ; un groupe halogène ; un groupe alkyle en C1 à C60 non substitué ou substitué ; un groupe cycloalkyle en C3 à C60 non substitué ou substitué ; un groupe aryle en C6 à C60 non substitué ou substitué ; ou un groupe hétéroaryle en C2 à C60 non substitué ou substitué,
Ar31 et Ar32 sont chacun indépendamment un groupe aryle en C6 à C60 non substitué ou substitué ; ou un groupe hétéroaryle en C2 à C60 non substitué ou substitué et incluant O ou N,
r31 est un nombre entier de 1 à 4, et quand il est 2 ou plus, les R31 sont identiques ou différents les uns des autres, et
r32 est un nombre entier de 1 à 4, et quand il est 2 ou plus, les R32 sont identiques ou différents les uns des autres.

10. Dispositif électroluminescent organique selon la revendication 9, dans lequel la Formule chimique 2 est représentée par l'un quelconque des composés suivants :

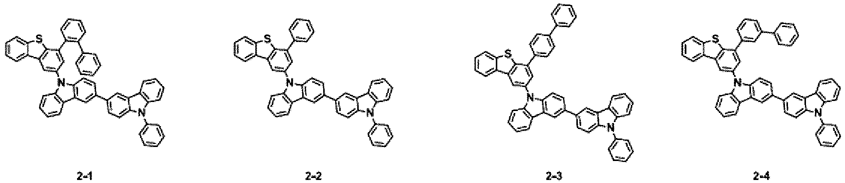

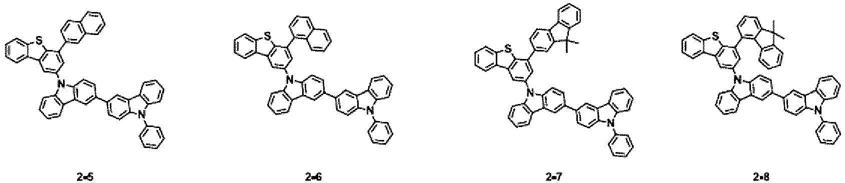

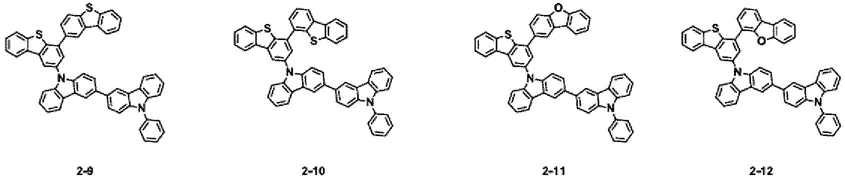

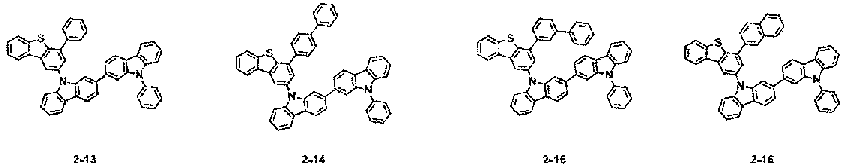

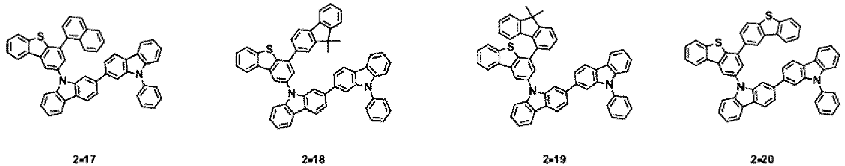

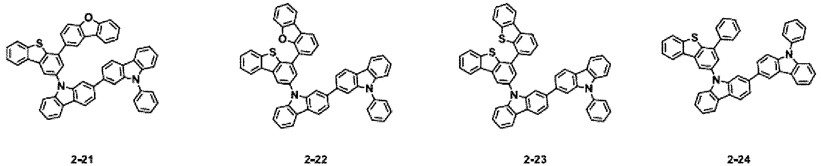

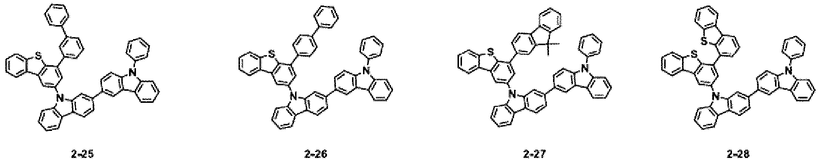

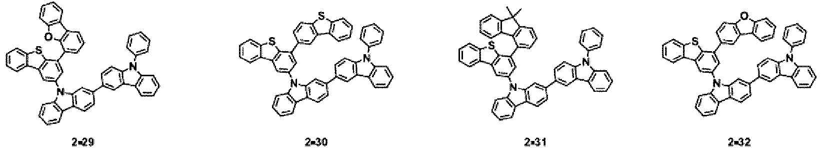

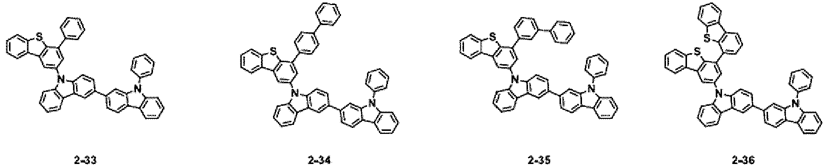

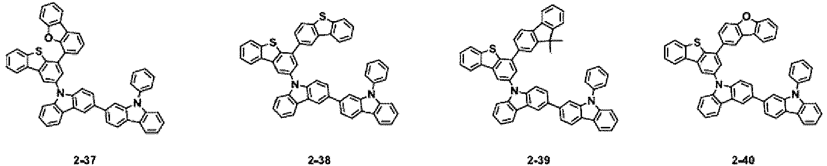

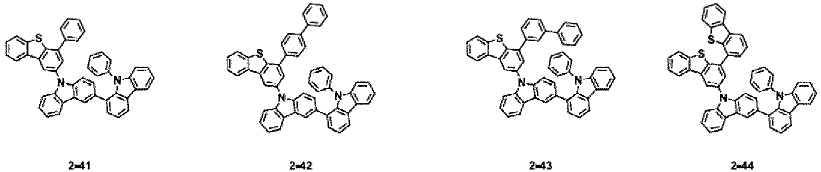

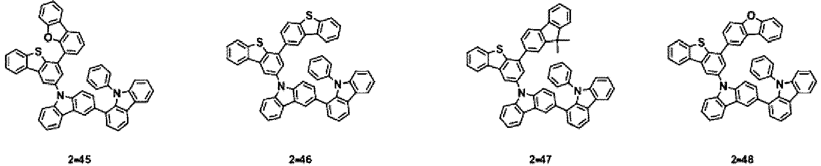

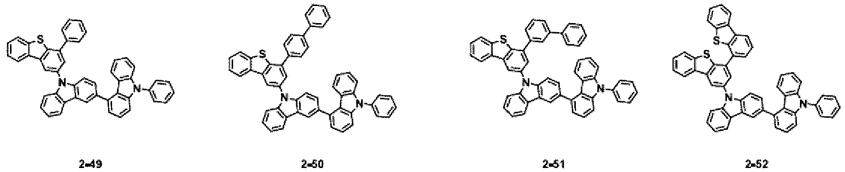

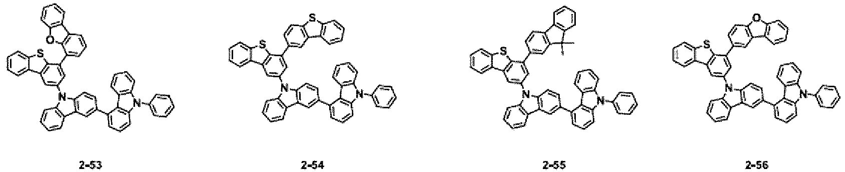

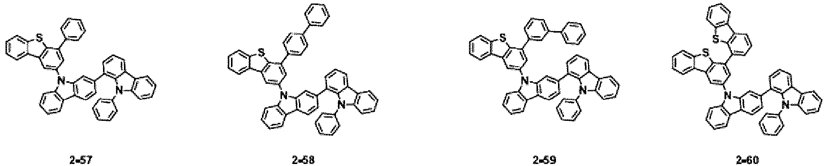

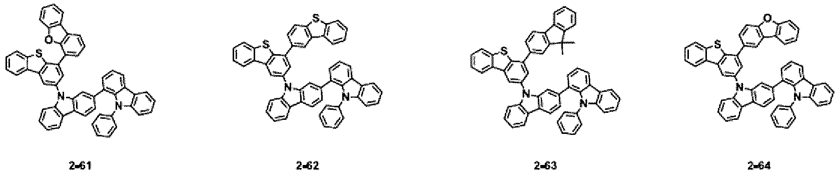

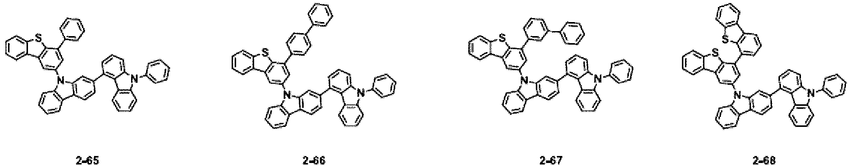

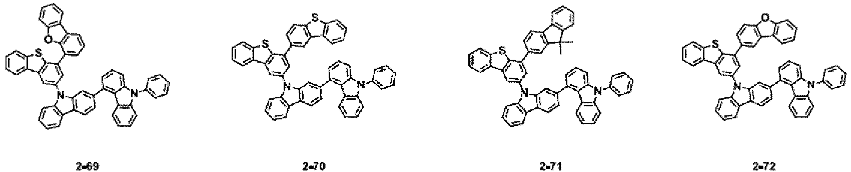

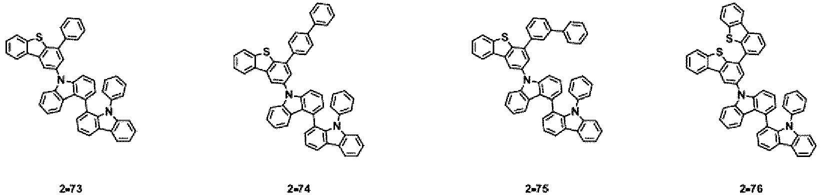

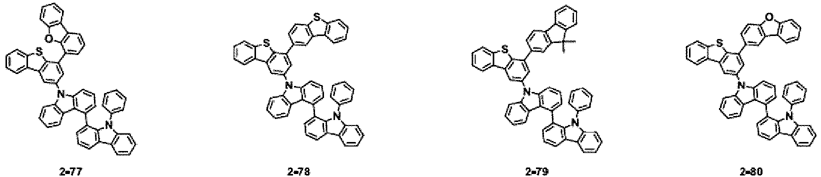

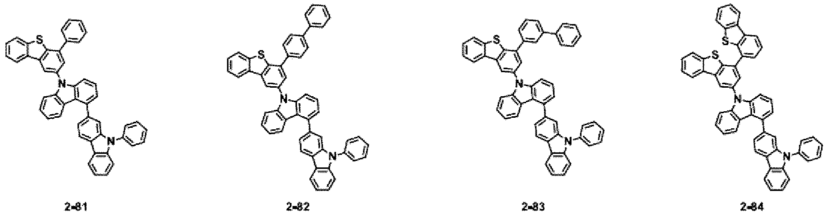

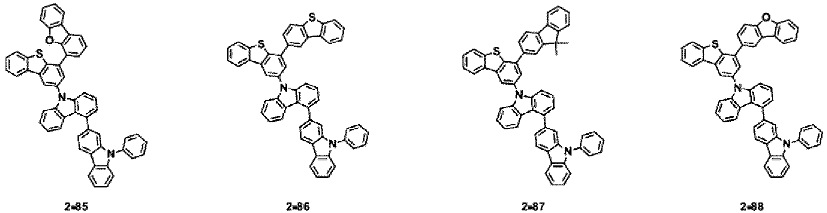

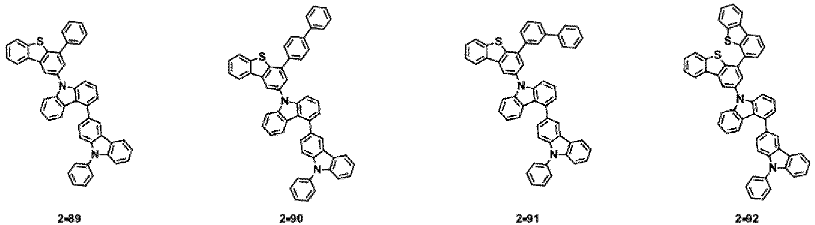

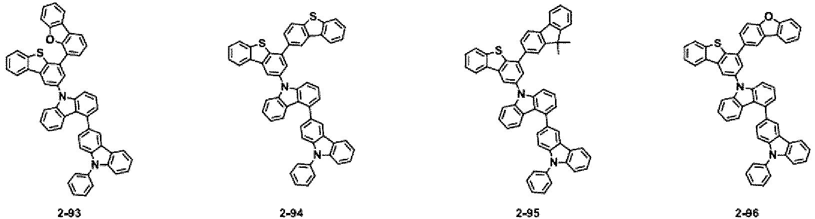

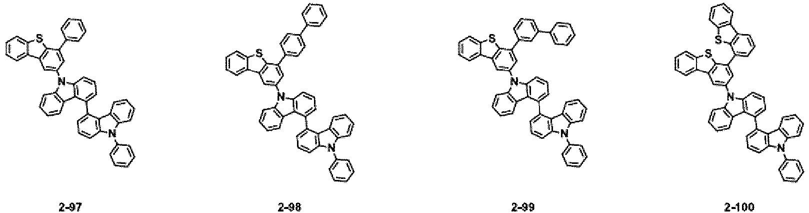

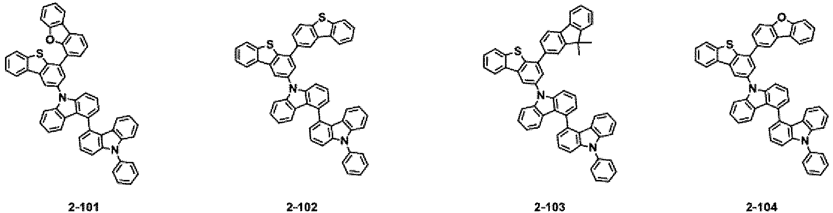

11. Dispositif électroluminescent organique selon la revendication 9, dans lequel la Formule chimique 3 est représentée par l'un quelconque des composés suivants :

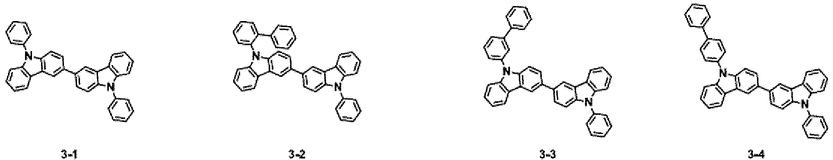

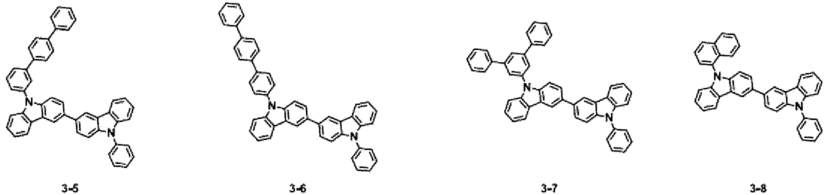

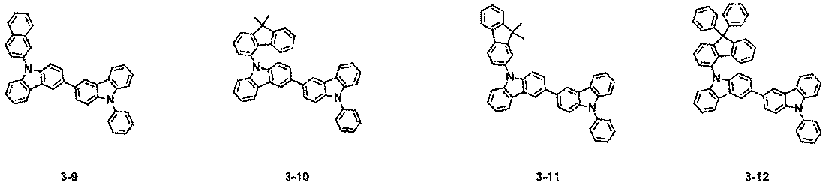

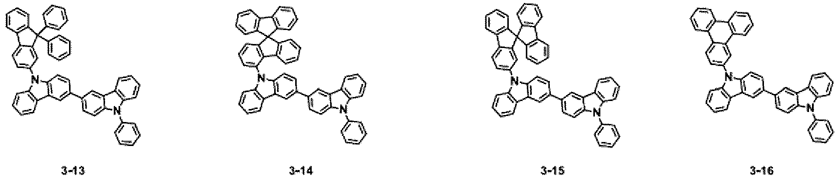

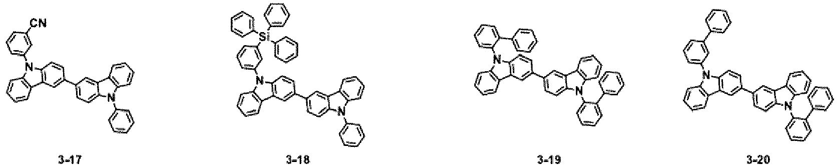

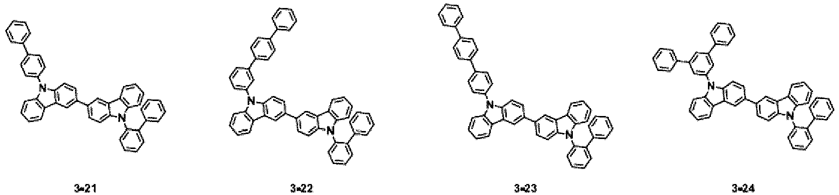

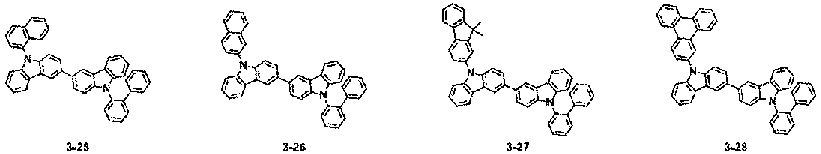

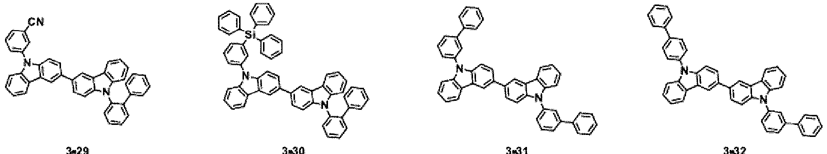

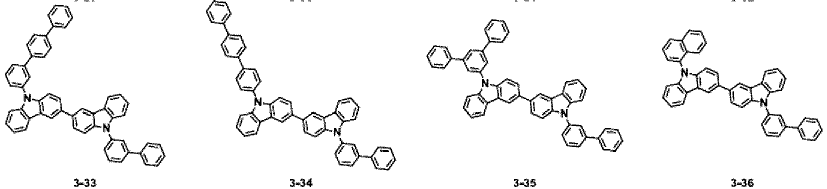

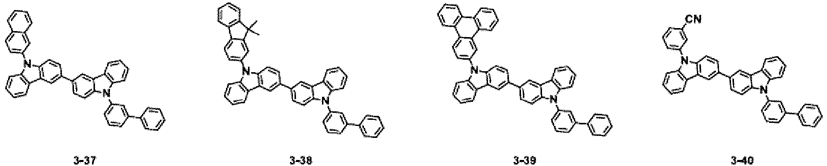

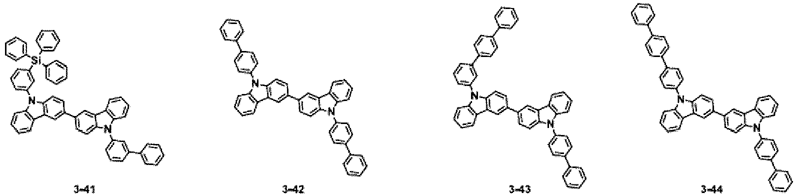

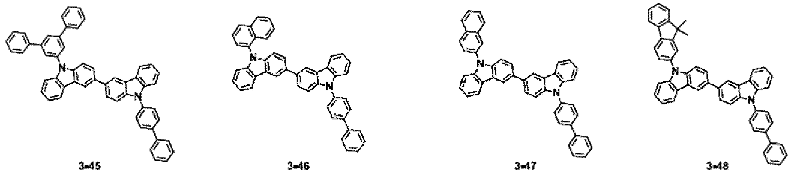

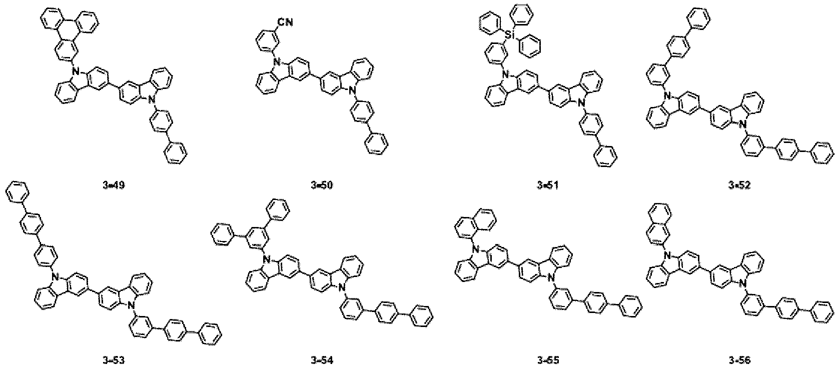

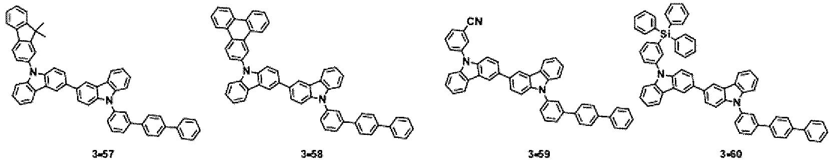

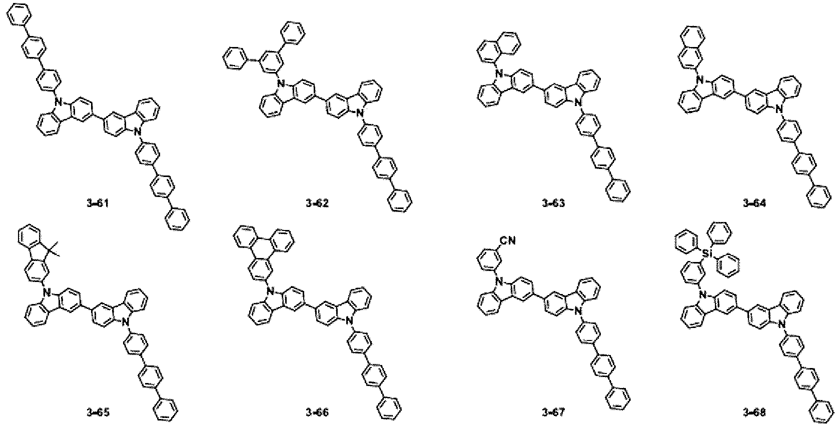

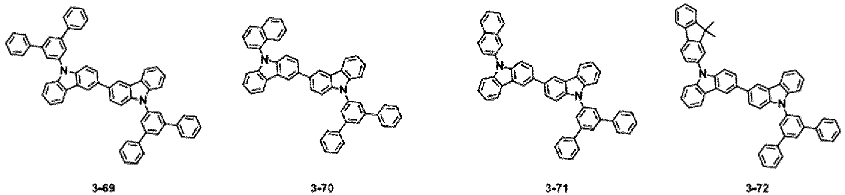

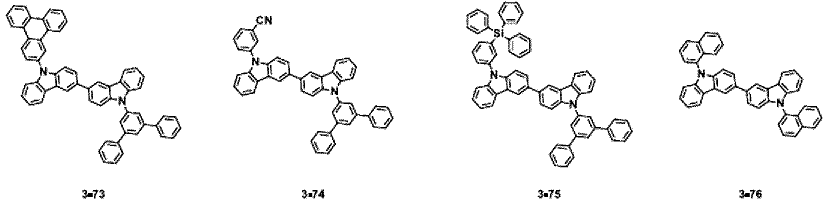

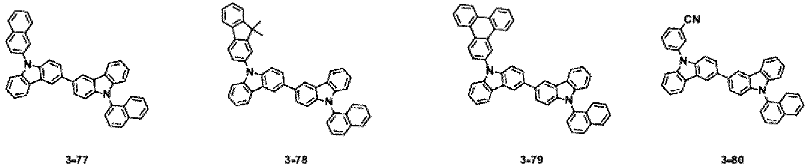

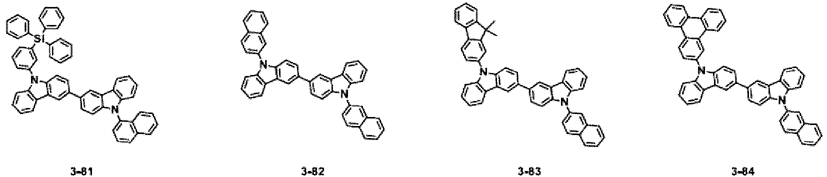

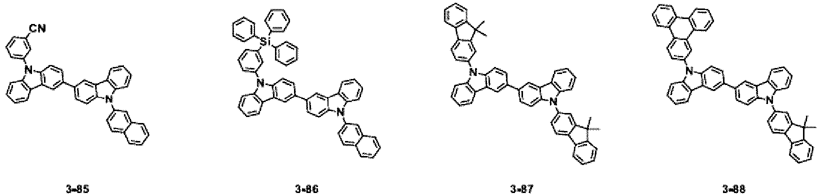

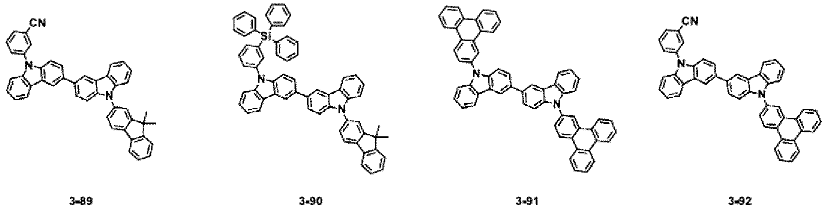

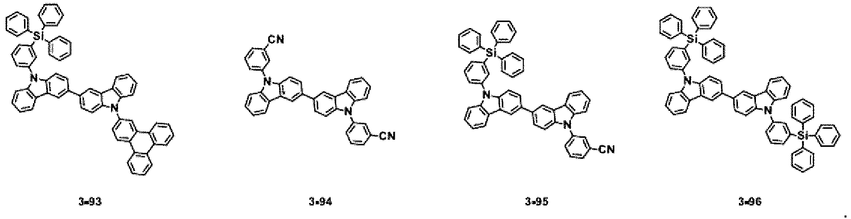

12. Composition pour former une couche de matériau organique, la composition comprenant :
un composé de la Formule chimique 1 ci-après ; et
un composé de la Formule chimique 2 ou 3 ci-après : 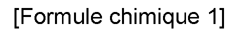 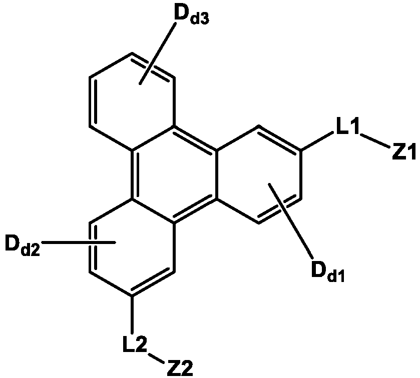
dans lequel, dans la Formule chimique 1,
d1 et d2 sont chacun un nombre entier de 0 à 3,
d3 est un nombre entier de 0 à 4,
L1 et L2 sont identiques ou différents les uns des autres, et chacun indépendamment une liaison directe ; ou un groupe arylène en C6 à C60 non substitué ou substitué,
Z1 et Z2 sont identiques ou différents les uns des autres, et chacun indépendamment un groupe silyle non substitué ou substitué ; un groupe fluorényle non substitué ou substitué ; ou un groupe hétéroaryle représenté par l'une quelconque des Formules chimiques 1-1 à 1-4 ci-après, et
au moins un parmi Z1 et Z2 est un groupe hétéroaryle représenté par l'une quelconque des Formules chimiques 1-1 à 1-3 ci-après, 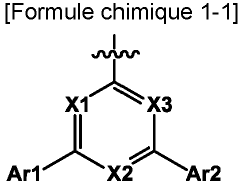 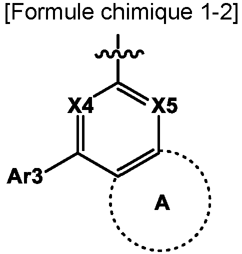 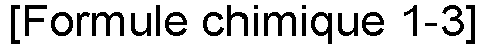 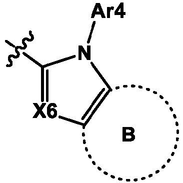 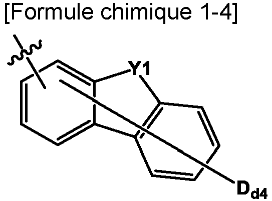
dans les Formules chimiques 1-1 à 1-4,
d4 est un nombre entier de 0 à 7,
X1 à X6 sont chacun N ou CR,
au moins un parmi X1 à X3 est N,
au moins un parmi X4 et X5 est N,
Y1 est O ; S ou NR',
R, R', Ar1 à Ar4 sont chacun indépendamment un hydrogène ; un deutérium ; un groupe halogène ; un groupe cyano ; un groupe hétéroaryle en C1 à C60 non substitué ou substitué ; un groupe aryle en C6 à C60 non substitué ou substitué ; ou un groupe hétéroaryle en C2 à C60 non substitué ou substitué,
A et B sont chacun un cycle aryle monocyclique ou polycyclique non substitué ou substitué ; ou un hétérocycle monocyclique ou polycyclique substitué ou non substitué et incluant O ou S, et
quand L1 est une liaison directe et Z1 est le groupe hétéroaryle représenté par la Formule chimique 1-1, Z2 est un groupe silyle non substitué ou substitué ; un groupe fluorényle non substitué ou substitué ; ou le groupe hétéroaryle tricyclique ou plus représenté par l'une quelconque des Formules chimiques 1-2 à 1-4, 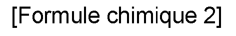 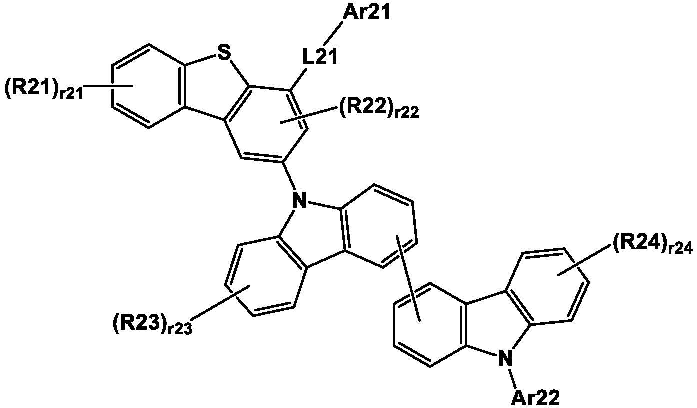
dans la Formule chimique 2,
R21 à R24 sont chacun indépendamment un hydrogène ; un deutérium ; un groupe halogène ; un groupe alkyle en C1 à C60 non substitué ou substitué ; un groupe cycloalkyle en C3 à C60 non substitué ou substitué ; un groupe aryle en C6 à C60 non substitué ou substitué ; ou un groupe hétéroaryle en C2 à C60 non substitué ou substitué,
L21 est une liaison directe ; un groupe arylène en C6 à C60 non substitué ou substitué ; ou un groupe hétéroarylène en C2 à C60 non substitué ou substitué,
Ar21 et Ar22 sont chacun indépendamment un groupe aryle en C6 à C60 non substitué ou substitué ; ou un groupe hétéroaryle en C2 à C60 non substitué ou substitué,
r21 est un nombre entier de 1 à 4, et quand il est 2 ou plus, les R21 sont identiques ou différents les uns des autres,
r22 est 1 ou 2, et quand 2, les R22 sont identiques ou différents les uns des autres,
r23 est un nombre entier de 1 à 4, et quand il est 2 ou plus, les R23 sont identiques ou différents les uns des autres, et
r24 est un nombre entier de 1 à 4, et quand il est 2 ou plus, les R24 sont identiques ou différents les uns des autres, 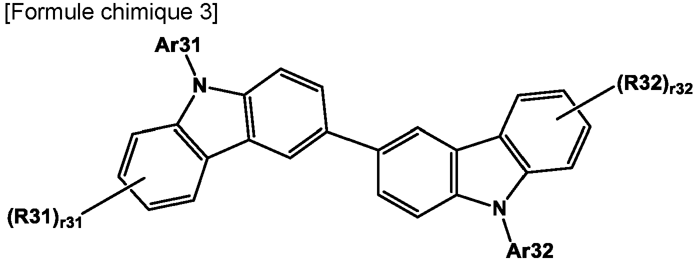
dans la Formule chimique 3,
R31 et R32 sont chacun indépendamment un hydrogène ; un deutérium ; un groupe halogène ; un groupe alkyle en C1 à C60 non substitué ou substitué ; un groupe cycloalkyle en C3 à C60 non substitué ou substitué ; un groupe aryle en C6 à C60 non substitué ou substitué ; ou un groupe hétéroaryle en C2 à C60 non substitué ou substitué,
Ar31 et Ar32 sont chacun indépendamment un groupe aryle en C6 à C60 non substitué ou substitué ; ou un groupe hétéroaryle en C2 à C60 non substitué ou substitué et incluant O ou N,
r31 est un nombre entier de 1 à 4, et quand il est 2 ou plus, les R31 sont identiques ou différents les uns des autres, et
r32 est un nombre entier de 1 à 4, et quand il est 2 ou plus, les R32 sont identiques ou différents les uns des autres.

13. Composition pour former une couche de matériau organique selon la revendication 12, dans lequel le composé de la Formule chimique 1 et le composé de la Formule chimique 2 ou 3 ont un rapport en poids de 1:10 à 10:1.
